(19) Europäisches Patentamt
European Patent Office
Office européen des brevets

(11) **EP 4 055 191 B1**

(12) **EUROPEAN PATENT SPECIFICATION**

(45) Date of publication and mention
of the grant of the patent:
**03.07.2024 Bulletin 2024/27**

(21) Application number: **20800688.2**

(22) Date of filing: **09.11.2020**

(51) International Patent Classification (IPC):
**C12Q 1/6883** (2018.01) **G16B 20/00** (2019.01)
**G16B 40/00** (2019.01)

(52) Cooperative Patent Classification (CPC):
**C12Q 1/6883; G16B 20/00; G16B 40/20;**
C12Q 2600/156

(86) International application number:
**PCT/EP2020/081459**

(87) International publication number:
**WO 2021/089866 (14.05.2021 Gazette 2021/19)**

(54) **DIFFERENTIAL DIAGNOSIS OF BIPOLAR DISORDER AND UNIPOLAR DEPRESSION BY BLOOD RNA EDITING BIOMARKERS**

DIFFERENTIALDIAGNOSE VON BIPOLARER STÖRUNG UND UNIPOLARER DEPRESSION DURCH BLUT-RNA-BEARBEITUNGSBIOMARKER

DIAGNOSTIC DIFFÉRENTIEL DE TROUBLES BIPOLAIRES ET DE LA DÉPRESSION UNIPOLAIRE PAR DES MARQUEURS D'ÉDITION D'ARN SANGUIN

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.11.2019 EP 19208126**

(43) Date of publication of application:
**14.09.2022 Bulletin 2022/37**

(73) Proprietor: **ALCEDIAG**
**13790 Peynier (FR)**

(72) Inventors:
• **WEISSMANN, Dinah**
**34270 Mathieu de Treviers (FR)**
• **SALVETAT, Nicolas**
**34070 Montpellier (FR)**

• **CHIMIENTI, Fabrice**
**34830 Jacou (FR)**

(74) Representative: **Yes My Patent**
**32 Boulevard Richard Lenoir**
**75011 Paris (FR)**

(56) References cited:
**EP-A1- 3 272 881      WO-A1-2021/089865**
**US-A1- 2010 273 153   US-A1- 2017 211 144**
**US-A1- 2019 065 666   US-A1- 2019 185 937**

Remarks:
The complete document including Reference Table(s) and the Sequence Listing(s) can be downloaded from the EPO website

**Description**

The present invention is drawn to a method for in vitro for differential diagnosing bipolar disorder and unipolar depression in a human patient from a biological sample of said patient, using at least two particular A to I editing RNA biomarkers associated to a specific depression score and algorithm. The present invention is also directed to a method for monitoring treatment for bipolar or unipolar disorder, said method implementing the method for differential diagnosing bipolar disorder and unipolar depression of the present invention. Kit for determining whether a patient presents a bipolar disorder versus unipolar depression disorder or healthy control patient is also comprised in the present invention.

Bipolar disorder (BD) is a common, severe and lifelong illness, affecting more than 1% of the population worldwide, regardless of origin, ethnicity or socio-economic status. Bipolar depression is comprised of fluctuating episodes of both depression and mania or hypomania. Symptoms of manic episodes can include unusually and persistently increased activity, or euphoria, decreased need for sleep, elevated self-confidence or impulsivity (for review see [1]). Patients with bipolar disorder are often misdiagnosed, with approximately 70% of them being misdiagnosed with unipolar depression or major depressive disorder. The resulting delay in proper diagnosis and appropriate treatment results in poorer prognosis and has devastating consequences for the patients, and can eventually increase suicide risk. The average interval between onset of BD symptoms and proper diagnosis and treatment is estimated to be 5.8 years. Furthermore, the resistance to treatment commonly observed with classical antidepressant in unipolar depression is often due to misdiagnosis of bipolar depression[2].

The most widely acknowledged diagnostic classifications are the 10th revision of the International Classification of Diseases (ICD-10) and the 5th edition of the Diagnostic and Statistical Manual of Mental Disorders (DSM-5), though the latter remains controversial for accurate diagnosis of bipolar disorder[1]. Both are based on clinical interview and may therefore appear as "subjective", since they are based on psychiatrist's judgment; there is no objective; laboratory-based test or biological markers to set the boundary between the different subtypes of depression. In addition, bipolar patients are much more likely to present to clinicians during a depressive episode. Therefore, a major research goal is to identify reliable and clinically useful biomarkers to differentiate bipolar disorder from unipolar depression. During the last decade, a number of studies have investigated neuroimaging or circulating biomarkers in BD. In a study using fMRI, Grotegerd and colleagues have shown that fMRI and pattern classification could classify depressed subjects as unipolar or bipolar with good confidence, though using small sample size of medicated patients[3]. More recently, an MRI method to measure amygdala activation and connectivity during facial emotion processing distinguished BP patients and MDD patients with 80% accuracy. However the study did not involve MDD patients who were either currently depressed or, for BP patients, who had current manic or hypomanic symptoms. Similarly, there are currently no valid circulating biomarkers for BD. A major step would be to identify circulating biomarkers for early diagnosis in patients. To date, a lot of research has been focused on pathways alterations in BD, including oxidative stress, inflammatory and trophic factor deregulation (for review see [4]). A meta-analysis of 52 studies identified decreased levels of plasma BDNF, a member of the neurotrophin family of growth factors, as consistently replicated. Interestingly, BDNF levels are decreased both during manic and depressive episodes, contrary to euthymia. However, the authors suggested that BDNF levels might only be used as a part of a laboratory blood protein composite measure because of lack of specificity. The existing literature also consistently report the existence of oxidative stress in BD. Lipid peroxidation, DNA/RNA damage, and nitric oxide are significantly increased in BD patients. However, due again to lack of specificity and robustness, no oxidative stress biomarker has been identified as clinically useful *per se*. Alterations of peripheral inflammatory markers have consistently been shown in mood disorders, including unipolar depression and BD, though it is not clear whether they are causative or a consequence of the disease. Circulating cytokines imbalance, including CXCL8, CXCL10 and CXCL11, has been demonstrated in BD when compared to controls. However, no differences were found during euthymia and mania episodes. A pilot study from the same group also identified soluble higher TNFR1 levels in BD patients. Moreover, sTNFR1 levels were higher during mania than in euthymic patients, confirming a previous report where the proinflammatory cytokines, IL-2, IL-4 and IL-6, were increased in comparison with healthy subjects. Transcriptomic analysis recently showed that only few changes in whole blood gene expression were associated with BD, while lithium treatment has a stronger impact, suggesting that monitoring gene expression levels will be of poor usefulness to develop diagnostic tools for BD.

Most promising methods seem to reside in a multi-parameter biomarker panel, for which there is a biological rationale. Those biomarkers should be quantified using a routine blood sample and standard laboratory methodology; a specific diagnostic algorithm is often developed in parallel. As an example, a metabolomics approach based on proton NMR (1H NMR) very recently identified a panel of 10 different compounds which could be used as biomarkers to discriminate between healthy controls, BD and schizophrenias. Accumulating evidence have indicated that RNA editing, which is mostly performed by the ADAR (adenosine deaminase acting on RNA) enzymes may be critically involved in neurological or immune diseases, among other pathologies (for review see[6]). Decreased editing of the R/G sites of AMPA receptors due to down-regulation of ADAR2 has a possible role in the pathophysiology of mental disorders, including schizophrenia and bipolar disorder.

US2017/211144 relates to genes and pathways differentially expressed in bipolar disorder and/or major depressive disorder.

In the current study, we focused on transcriptome-wide RNA editing modification by RNA-Seq on Unipolar and BD patients to identify editing variants that could be used as biomarkers to set up a clinically useful diagnostic assay for differential diagnosis of BD. We identified significant differential editing in a number of genes involved in different pathways relevant for mood disorders, e.g. immune system and CNS function, e.g. regulation of MECP2, which highly expressed in neurons, where it participates in the process synapse formation. The RNA editing variants panel allowed differential diagnosis of bipolar disorder and unipolar depression with high specificity and selectivity. This study is the first to evaluate multiple RNA editing variants in blood samples of healthy controls and patients suffering unipolar or bipolar depression. Our findings will contribute to a better understanding of the molecular pathophysiology of BD, and pave the way for the development of a diagnostic assay for BD with clinical application.

Disclosed, but not part of the claimed invention is a method for selecting a biomarker, preferably a combination of at least two biomarkers, which can be used to differential diagnose bipolar disorder and unipolar depression in a human patient, said method comprising the step of:

a) analyzing the RNA-Seq dataset using Editome analysis pipeline and identifying A-to-I differentially edited positions with a minimum coverage of 30x, which ensures a high degree of confidence at particular base positions;
b) performing a differential analysis to identify sites whose editing could be specifically different between unipolar depression and healthy controls,
c) applying the following pre-specified quality criteria of the group consisting of: coverage >30; AUC>0.6; 0.95>Fold-Change>1.05, p<0.05 and exclusion of intergenic sites;
d) optionally, checking that no difference in global RNA editing was observed between patients and controls, preferably by calculating the global Alu editing index (AEI), which is a measure of the overall rate of RNA editing in Alu repeats; e) by GSEA (enrichment analysis on gene sets) on the biomarkers selecting in step c) or d), preferably by using gene ontology tools, identifying and selecting biomarkers reflecting changes in different biological process including immune and CNS (central nervous system) functions ; and
f) applying the following specified quality criteria of the group consisting of: coverage >30; AUC>0.8; 0.8>Fold-Change>1.20 and p<0.05) to a combination of several biomarkers selected in step e) representing different biological mechanisms, and selected those which clearly discriminated patients exhibiting bipolar disorder and patients exhibiting unipolar depression in two separate groups.

In a preferred embodiment, in step f), the combinations of at least two biomarkers are selected whether said combinations are statistically significant between control and cases with a p value $<10^{-4}$.

The criterion symptoms for bipolar disorders, depression disorders and MDD are well-known from the skilled person and are for example described in the DSM-5™ book from the American Psychiatric Association (2013, pages DSM-V p123-154);

The diagnoses included in this chapter are bipolar I disorder, bipolar II disorder, cyclothymic disorder, substance/medication-induced bipolar and related disorder, bipolar and related disorder due to another medical condition, other specified bipolar and related disorder, and unspecified bipolar and related disorder. However, the vast majority of individuals whose symptoms meet the criteria for a fully syndromal manic episode also experience major depressive episodes during the course of their lives Bipolar II disorder, requiring the lifetime experience of at least one episode of major depression and at least one hypomania episode, is no longer thought to be a "milder" condition than bipolar I disorder, largely because of the amount of time individuals with this condition spend in depression and because the instability of mood experienced by individuals with bipolar II disorder is typically accompanied by serious impairment in work and social functioning. The diagnosis of cyclothymic disorder is given to adults who experience at least 2 years (for children, a full year) of both hypomanie and depressive periods without ever fulfilling the criteria for an episode of mania, hypomania, or major depression.

The criterion symptoms for unipolar depression are also well-known from the skilled person and are for example described in the DSM-5™ book from the American Psychiatric Association (2013, pages 155-188). Depressive disorders include disruptive mood dysregulation disorder, major depressive disorder (including major depressive episode), persistent depressive disorder (dysthymia), premenstrual dysphoric disorder, substance/medication-induced depressive disorder, depressive disorder due to another medical condition, other specified depressive disorder, and unspecified depressive disorder.

The present invention is directed to an in vitro method for for differential diagnosing bipolar disorder and unipolar depression in a human patient from a biological sample of said patient, said method comprising:

a) determining for at least two A to I editing RNA biomarker identified by the method

for selecting a biomarker according to the invention:

- the relative proportion of RNA editing at a given editing site for at least one or a combination of sites which can be edited on the RNA transcript of said t biomarker, and/or
- the percentage of an isoform or of a combination of isoforms of the RNA transcript of said biomarker;

wherein said at least two A to I editing RNA biomarker is selected from the group of consisting of MDM2, PRKCB, PIAS1, IFNAR1, LYN, AHR, RASSF1, GAB2, CAMK1D, IL17RA, PTPRC, KCNJ15, IFNAR2 and PDE8A biomarkers;

b) determining a value resulting from the percentage(s) obtained in step a);
c) determining whether said result value obtained in step b) is greater or not greater than a control value obtained for control unipolar subject, wherein the control value was determined in a manner comparable to that of the result value, obtained in step b); and
d) if said result value obtained for the patient is greater than a threshold (for example, but non-limited to, a cut-off or a probability), classifying said patient as having bipolar disorder or, if said result value is not greater than said threshold, classifying said patient having unipolar depression.

Preferably, the present invention relates to an in vitro method for differential diagnosing bipolar disorder and unipolar depression in a human patient from a biological sample of said patient, said method comprising:

a) determining for a combination of at least two A to I editing RNA biomarkers identified by the method for selecting a combination of at least two biomarkers according to the present invention:

- for each selected biomarker, the relative proportion of RNA editing at a given editing site for at least one or a combination of sites which can be edited on the RNA transcript of said at least two biomarkers, and/or
- the relative percentage of an isoform or of a combination of isoforms of the RNA transcript of each of said two biomarkers;

wherein said at least two A to I editing RNA biomarkers are selected from the group consisting of MDM2, PRKCB, PIAS1, IFNAR1, LYN, AHR, RASSF1, GAB2, CAMK1D, IL17RA, PTPRC, KCNJ15, IFNAR2 and PDE8A biomarkers;
b) determining a result value obtained for each of said at least two biomarkers and a final result value based on an algorithm or equation that includes the result value obtained for each selected biomarker;
c) determining whether said result value obtained in step b) is greater or not greater than a control value obtained for control unipolar subject, wherein the control value was determined in a manner comparable to that of the result value; and
d) if said result value obtained for the patient is greater than a threshold, classifying said patient as having bipolar disorder or, if said result value is not greater than said threshold, classifying said patient having unipolar depression.

For example, but non-limited to, in the method of diagnosing according to the present invention, said threshold, cut-off or probability (named also "depression score") can be but non-limited to:

- the Z value calculated for healthy control patient when using mROC program, or
- the probability for a patient to be considered as having mood disorders, depression disorders or MDD.

In the present description, the term biomarker or target have the same meaning and can be used interchangeably.
In a preferred embodiment, in the method for diagnosing depression according to present invention, the combinations of at least two biomarkers are selected whether said combinations are statistically significant between control healthy control subject and cases (patients having bipolar or unipolar disorders), or between bipolar and unipolar control subject with a p value $<10^{-4}$.
In a preferred embodiment, in the in vitro method for differential diagnosing bipolar disorder and unipolar depression in a human patient according to the invention, said A to I editing RNA biomarker is selected from the group consisting of MDM2, PRKCB, IFNAR1, LYN, GAB2, CAMK1D, KCNJ15 and PDE8A.
In a more preferred embodiment, in the in vitro method for differential diagnosing bipolar disorder and unipolar depression in a human patient according to the invention, said at least two A to I editing RNA biomarkers are selected from the group consisting of MDM2, PRKCB, IFNAR1, LYN, GAB2, CAMK1D, KCNJ15 and PDE8A.
Are preferred, the methods for differential diagnosing bipolar disorder and unipolar depression in a human patient according to the present invention wherein in step b), the result value or final result value is calculated by an algorithm implementing a multivariate method including:

- mROC program, particularly to identify the linear combination, which maximizes the AUC (Area Under the Curve) ROC and wherein the equation for the respective combination is provided and can be used as a new virtual marker Z, as follows:

$$Z = a.(Biomarker\ 1) + b.(Biomarker\ 2) + \ldots i.(Biomarker\ i) + \ldots n.(Biomarker\ n)$$

where i are calculated coefficients and (Biomarker i) are the level of the considered biomarker (i.e. level of RNA editing site or of isoforms for a given target/biomarker); and/or

- a Random Forest (RF) approach applied to assess the RNA editing site(s) and/or isoforms combinations, particularly to rank the importance of the RNA editing site(s) and/or isoform(s), and to combine the best RNA editing site(s) and/or isoform(s), and/or optionally
- a multivariate analysis applied to assess the RNA editing site(s) and/or isoforms combinations for the diagnostic, said multivariate analysis being selecting for example from the group consisting of:

  - Logistic regression model applied for univariate and multivariate analysis to estimate the relative risk of patient at different level of RNA editing site or isoforms values;
  - CART (Classification And Regression Trees) approach applied to assess RNA editing site(s) and/or isoforms combinations; and/or
  - Support Vector Machine (SVM) approach;
  - Artificial Neural Network (ANN) approach;
  - Bayesian network approach;
  - WKNN (weighted k-nearest neighbours) approach;
  - Partial Least Square - Discriminant Analysis (PLS-DA);
  - Linear and Quadratic Discriminant Analysis (LDA / QDA), and
  - Any other mathematical method that combines biomarkers.

In a preferred embodiment, in the in vitro method for differential diagnosing bipolar disorder and unipolar depression in a human patient according to the invention, said unipolar depression disorder is mainly major depressive disorder (MDD).

In a preferred embodiment, in the in vitro method for differential diagnosing bipolar disorder and unipolar depression in a human patient according to the invention said biological sample is whole blood, serum, plasma, urine, or cerebrospinal fluid, whole blood being the most preferred biological sample

Are preferred selected biomarkers wherein the result value or depression score obtained in step c) is statistically/specifically different from said control result value at p<0.05.

Are also preferred the methods of the present invention wherein the following criteria has to be satisfied for the biomarker or for the combination of biomarkers selected tin step a):

- the coverage >30;
- AUC>0.6, more preferred AUC>0.8;
- 0.95>FoldChange>1.05, and
- p<0.05.

Are also preferred the methods of the present invention wherein said selected A to I editing RNA biomarker(s) comprised in step a) is selected from the group consisting of a combination comprising at least 3, 4, 5, 6, 7 or 8 of the following biomarkers: MDM2, PRKCB, IFNAR1, LYN, GAB2, CAMK1D, KCNJ15 and PDE8A, preferably the combination of the cited 8 biomarkers.

Are also preferred the methods of the present invention wherein said selected A to I editing RNA biomarker(s) comprised in step a) the combination of the following 5 biomarkers: GAB2, IFNAR1, KCNJ15, MDM2 and PRKCB.

Are also preferred the methods of the present invention wherein said selected A to I editing RNA biomarker(s) comprised in step a) the combination of the following 5 biomarkers: GAB2, IFNAR1, KCNJ15, MDM2 and PRKCB and at least another biomarker selected from the group consisting the following biomarkers; CAMK1D and LYN, preferably the combination of the following 6 biomarkers: GAB2, IFNAR1, KCNJ15, MDM2, PRKCB and LYN when the model is adjusted by age, sex, psychiatric treatment and addiction, or the following 6 biomarkers: GAB2, IFNAR1, KCNJ15, MDM2, PRKCB and CAMK1D when the model is adjusted by age and sex, preferably when using a Random Forest (RF) algorithm applied to assess the RNA editing site(s) and/or isoforms combinations, particularly to rank the importance of the RNA editing site(s) and/or isoform(s), and to combine the best RNA editing site(s) and/or isoform(s).

Are also preferred the methods of the present invention wherein said selected A to I editing RNA biomarker(s) comprised in step a) the combination of the following 5 biomarkers: GAB2, IFNAR1, KCNJ15, MDM2 and PRKCB and at least another biomarker selected from the group consisting the following biomarkers; CAMK1D and LYN, and wherein the calculation of the relative percentage of at least one of the RNA edition site or isoform is based on the RNA edition site or isoform listed in Tables 5.1 and 5.2, preferably when using a Random Forest (RF) algorithm applied to assess the RNA editing site(s) and/or isoforms combinations, particularly to rank the importance of the RNA editing site(s) and/or isoform(s), and to combine the best RNA editing site(s) and/or isoform(s)

Are also preferred the methods of the present invention wherein said selected A to I editing RNA biomarker comprised in step a) one or combination of at least 2, 3, 4, 5, 6, 7 or 8 selected biomarkers and wherein the calculation of the relative percentage of at least one of the RNA edition site or isoform is based on the RNA edition site or isoform listed in Tables 2A (edition sites), 2B (isoforms) and 3 (edition sites)

Are also preferred the methods of the present invention wherein said combination of at least 2, 3, 4, 5, 6, 7 or 8 selected biomarkers is a combination selected from the biomarkers combinations given Tables 4, 5 and 6, preferably the combinations comprising the following 8 biomarkers MDM2, PRKCB, IFNAR1, LYN, GAB2, CAMK1D, KCNJ15 and PDE8A.

Are also preferred the methods of the present invention wherein the algorithm or equation allowing the calculation of the result value or depression score Z are selected from the Z equations listed in the examples, preferably the equation implemented a combination of the following 8 biomarkers: MDM2, PRKCB, IFNAR1, LYN, GAB2, CAMK1D, KCNJ15 and PDE8A.

In another aspect, the present invention is directed to an in vitro method for differential diagnosing bipolar disorder and unipolar depression in a human patient according to the preceding claims, wherein if the patient to be tested is a female, in step a) said at least one or combination of at least two A to I editing RNA biomarker(s) is selected from the group of biomarkers consisting of MDM2, PRKCB, PIAS1, IFNAR1, LYN, AHR, RASSF1, GAB2, CAMK1D, IL17RA, PTPRC, KCNJ15, IFNAR2 and PDE8A biomarker, preferably the biomarkers combinations or Z equation given in the Examples or figures with patients of the female population, more preferably the combinations comprising the 8 biomarkers MDM2, PRKCB, IFNAR1, LYN, GAB2, CAMK1D, KCNJ15 and PDE8A, more preferably the combinations listed in Table 7.

In another aspect, the present invention is directed to an in vitro method for differential diagnosing bipolar disorder and unipolar depression in a human patient according to the preceding claims, wherein if the patient to be tested is a male, in step a) said at least one or combination of at least two A to I editing RNA biomarker(s) is selected from the group of biomarkers consisting of MDM2, PRKCB, PIAS1, IFNAR1, LYN, AHR, RASSF1, GAB2, CAMK1D, IL17RA, PTPRC, KCNJ15, IFNAR2 and PDE8A biomarker, preferably the biomarkers combinations or Z equation given in the Examples or figures with patients of the male population, more preferably the combinations comprising the 8 biomarkers MDM2, PRKCB, IFNAR1, LYN, GAB2, CAMK1D, KCNJ15 and PDE8A, more preferably the combinations listed in Table 8.

In a more preferred embodiment, the present invention is directed to a method in vitro for differential diagnosing bipolar disorder and unipolar depression in a human patient, according to the present invention, wherein the combination of biomarkers and/or the Z equation or algorithm associated with said combination is different whether the patient to be tested is a female or a male.

Preferably, the combination of biomarkers and/or the Z equation whether the patient to be tested is a female or a male is selected from the combination and/or the Z equation depicted in the examples or in the figures for this particular case.

Disclosed, but not part of the claimed invention is a method in vitro for differential diagnosing bipolar 1 and bipolar 2 disorder in a human patient from a biological sample, preferably a blood sample, of the patient, wherein said method comprising the step of:

a) determining for a combination of at least two A to I editing RNA biomarkers selected from the group consisting of MDM2, PRKCB, PIAS1, IFNAR1, LYN, AHR, RASSF1, GAB2, CAMK1D, IL17RA, PTPRC, KCNJ15, IFNAR2 and PDE8A biomarkers;

- for each selected biomarker, the relative proportion of RNA editing at a given editing site for at least one or a combination of sites which can be edited on the RNA transcript of said at least two biomarkers, and/or
- the relative percentage of an isoform or of a combination of isoforms of the RNA transcript of each of said two biomarkers;

b) determining a result value obtained for each of said at least two biomarkers and a final result value based on an algorithm or equation that includes the result value obtained for each selected biomarker;

c) determining whether said result value obtained in step b) is greater or not greater than a control value obtained for control bipolar 1 and/or bipolar 2 subject, wherein the control value was determined in a manner comparable to that of the result value; and

d) if said result value obtained for the patient is greater or not than a threshold, classifying said patient as having bipolar 1 or bipolar 2 disorder.

Are also preferred the methods in vitro for differential diagnosing bipolar 1 and bipolar 2 disorder in a human patient of the present invention wherein said combination of at least 2, 3, 4, 5, 6, 7 or 8 selected biomarkers is a combination selected from the biomarkers selected from the group of MDM2, PRKCB, IFNAR1, LYN, GAB2, CAMK1D, KCNJ15 and PDE8A biomarkers, preferably the combinations comprising the following 8 biomarkers MDM2, PRKCB, IFNAR1, LYN, GAB2, CAMK1D, KCNJ15 and PDE8A.

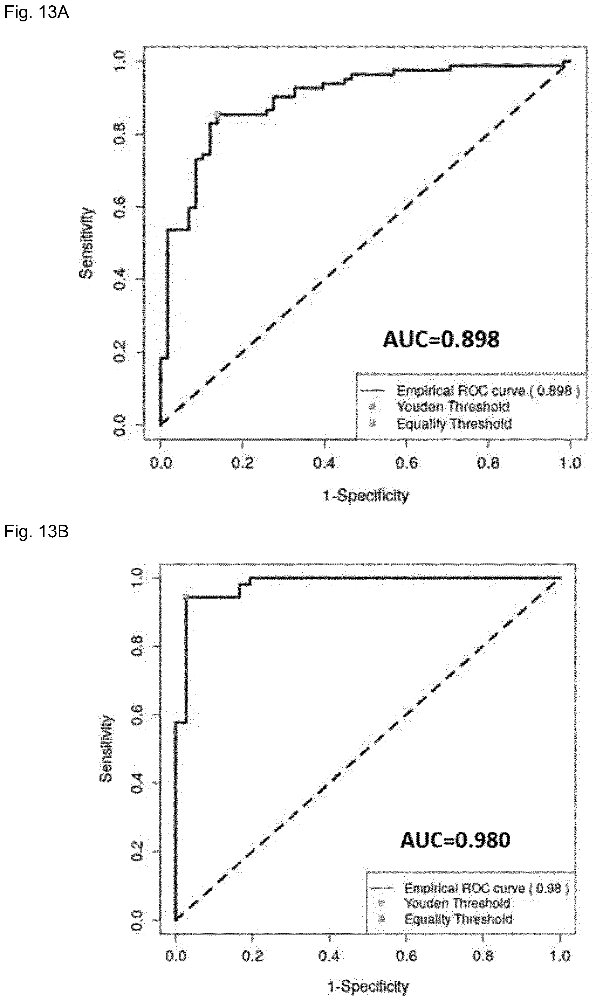

Are also preferred the methods wherein the algorithm or equation allowing the calculation of the result value or depression score Z are selected from the Z equations listed in the examples, particularly in Table 13 and Figure 13 B, preferably the equation implemented a combination of the following 8 biomarkers: MDM2, PRKCB, IFNAR1, LYN, GAB2, CAMK1D, KCNJ15 and PDE8A.

Disclosed, but not part of the claimed invention is a method in vitro for differential diagnosing patient having bipolar disorder and healthy patient from a biological sample, preferably a blood sample, of the patient, wherein said method comprises the steps of:

a) determining for a combination of at least two A to I editing RNA biomarkers selected from the group consisting of MDM2, PRKCB, PIAS1, IFNAR1, LYN, AHR, RASSF1, GAB2, CAMK1D, IL17RA, PTPRC, KCNJ15, IFNAR2 and PDE8A biomarkers;

- for each selected biomarker, the relative proportion of RNA editing at a given editing site for at least one or a combination of sites which can be edited on the RNA transcript of said at least two biomarkers, and/or
- the relative percentage of an isoform or of a combination of isoforms of the RNA transcript of each of said two biomarkers;

b) determining a result value obtained for each of said at least two biomarkers and a final result value based on an algorithm or equation that includes the result value obtained for each selected biomarker;
c) determining whether said result value obtained in step b) is greater or not greater than a control value obtained for control healthy and bipolar subject, wherein the control value was determined in a manner comparable to that of the result value; and
d) if said result value obtained for the patient is greater or not than a threshold, classifying said patient as being heathy patient or having bipolar disorder.

Are also preferred the methods in vitro for differential diagnosing patient having bipolar disorder and healthy patient in a human patient of the present invention wherein said combination of at least 2, 3, 4, 5, 6, 7 or 8 selected biomarkers is a combination selected from the biomarkers selected from the group of MDM2, PRKCB, IFNAR1, LYN, GAB2, CAMK1D, KCNJ15 and PDE8A biomarkers, preferably the combinations comprising the following 8 biomarkers MDM2, PRKCB, IFNAR1, LYN, GAB2, CAMK1D, KCNJ15 and PDE8A.

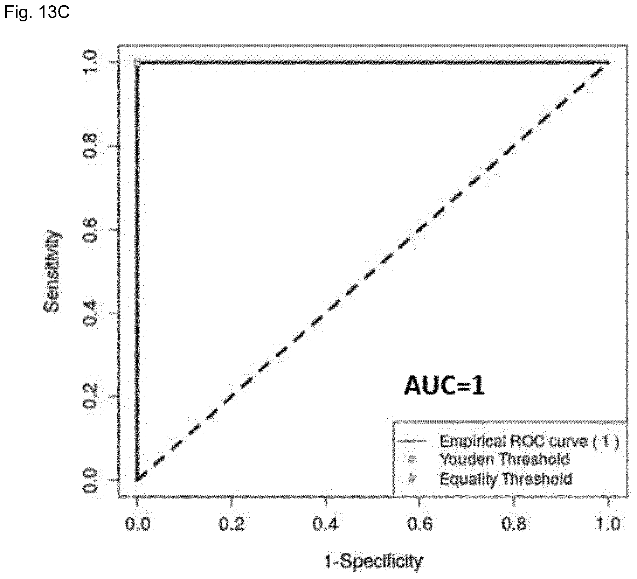

Are also preferred the methods of the present invention wherein the algorithm or equation allowing the calculation of the result value or depression score Z are selected from the Z equations listed in the examples, particularly in Table 12 and Figure 13 C, preferably the equation implemented a combination of the following 8 biomarkers: MDM2, PRKCB, IFNAR1, LYN, GAB2, CAMK1D, KCNJ15 and PDE8A.

Are also preferred the methods of the present invention, wherein in step a) the relative proportion of RNA editing at a given editing and/or the percentage of an isoform are measuring by NGS in said biological sample.

Are also preferred the methods of the invention, wherein in step a), the amplicon/nucleic sequence used for the detection of the RNA editing sites and/or the isoforms of the RNA transcript of said biomarker is obtained or obtainable with the set of primers listed in Table 1 for each of the selected biomarkers (SEQ ID NO. 1 to 36). Are also preferred the methods of the present invention, wherein in step a), the amplicon/nucleic sequence used for the detection of the RNA editing sites and/or the isoforms of the RNA transcript of said biomarker is obtained or obtainable with a set or a combination of sets of primers allowing to obtain amplicon(s) or nucleic acid sequence(s) including, or identical to, the amplicon(s) or nucleic acid sequence(s) obtainable by the set of primers listed in Table 1 (SEQ ID No.1 to SEQ ID No.36).

Said amplicon or nucleic acid sequence used for the detection of the RNA editing sites and/or the isoforms of the RNA transcript of said biomarker could be the RNA transcript complete sequence itself.

In another aspect, the present invention is directed to a method for monitoring treatment for bipolar disorder or unipolar depression in a human patient, from a biological sample of said patient, said method comprising:

A) differential diagnosing bipolar disorder or unipolar depression in said human by the method according to the method of the invention before the beginning of the treatment which is desired to be monitored.
B) repeating steps (a) to c) of the method of the method of the invention, after a period of time during which said patient receives treatment for said bipolar disorder or unipolar depression, to obtain a post-treatment result value;
C) comparing the post-treatment result value from step (c) to:

- the result value obtained before the period of time during which said patient receives treatment for said bipolar disorder or unipolar depression, and
- to the result value (control final value) for control bipolar disorder or unipolar depression patients, and

classifying said treatment as being effective if the post-treatment result value obtained in step B) is closer than the result value obtained before the period of time during which said patient receives treatment for control bipolar disorder or unipolar depression patients.

In another aspect, the present invention is directed to a method for determining whether a patient will be a responder to a bipolar disorder treatment allowing the patient to be in a euthymic state (state of normal mood) from a biological sample, preferably a blood sample of said patient, said method comprising the step of:

A) differential diagnosing bipolar disorder for said human by the method according to the present invention before the beginning of the treatment which is desired to be monitored.
B) repeating steps (a) to c) of the method of the present invention, after a period of time during which said patient receives treatment for said bipolar disorder, to obtain a post-treatment result value;
C) comparing the post-treatment result value from step (c) to:

- the result value obtained before the period of time during which said patient receives treatment for said bipolar disorder, and
- to the result value (control final value) for control bipolar disorder patients being in an euthymic state, and

classifying said patient as being responder to the treatment if the post-treatment result value obtained in step B) is closer than the result value obtained before the period of time during which said patient receives treatment for control bipolar disorder patients being in an euthymic state.

In another aspect, the present invention is directed to a kit for differential diagnosing bipolar disorder and unipolar, preferably a MDD, in a human patient said kit comprising:

1) - optionally, instructions to apply the method for differential diagnosing bipolar disorder and unipolar depression in a human patient, in order to obtain the result value the analysis of which determining whether said patient presents a bipolar disorder or unipolar depression; and
2) - one pair of primers or a combination of at least two pairs of primers selected from the group of primers consisting of SEQ ID No.1 to SEQ ID No.36 and, the selection of which being dependent of the biomarker(s) used for differential diagnosing bipolar disorder and unipolar depression in human patient;
or

- a pair or a combination of pairs of primers allowing to obtain amplicon(s) identical to, the amplicon(s) obtainable by the pairs of primers listed in Table 1 (SEQ ID No.1 to SEQ ID No.36).

The following examples and the figures and the legends hereinafter have been chosen to provide those skilled in the art with a complete description in order to be able to implement and use the present invention These examples are not intended to limit the scope of what the inventor considers to be its invention, nor are they intended to show that only the experiments hereinafter were carried out.

Other characteristics and advantages of the invention will emerge in the remainder of the description with the Examples, Figures and Tables, for which the legends are given herein below.

## BRIEF DESCRIPTION OF THE DRAWINGS AND THE TABLES

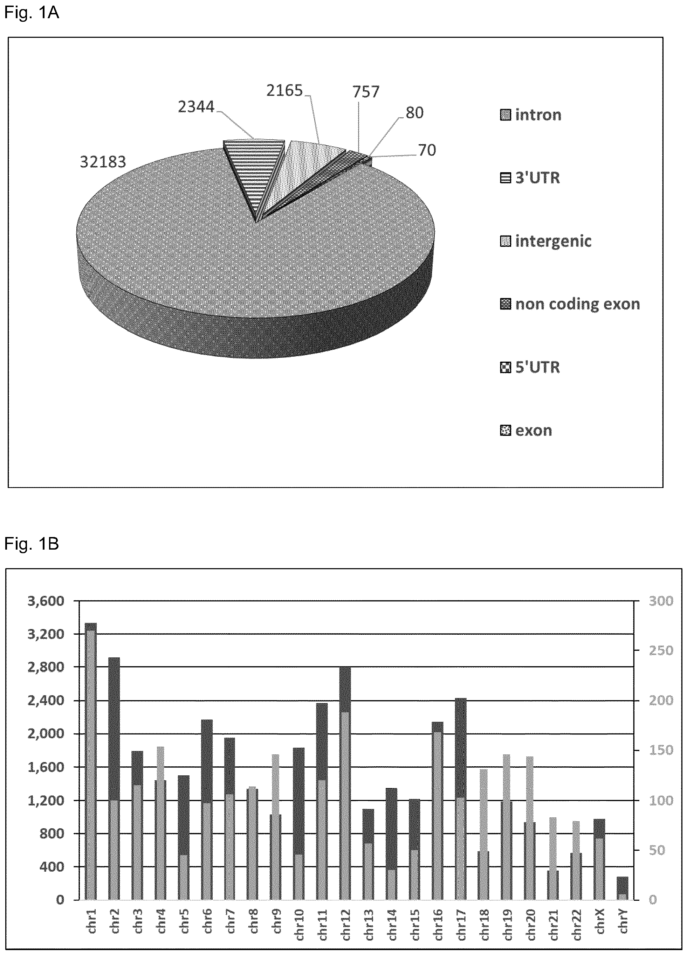

**Figures 1A-1D:** Identification and validation of differentially A-to-I RNA editing sites between healthy controls and DEP patients using RNA-seq data from human blood.

A: Genomic localization of detected editing sites
B: Repartition of A-to-I editing events or edited genes by chromosome.

Dark grey (left y-axis): Repartition of A-to-I editing events identified with a minimum coverage of 30 in the RNA-Seq study.
Light grey (right y-axis): Repartition of edited genes identified with a minimum coverage of 30 in the RNA-Seq study

C: Volcano plot of differentially edited sites between controls and DEP patients. The volcano plot shows the upregulated and downregulated differentially edited genes sites between depressed patients (DEP) group and the control group. For each plot, the x-axis represents the log (2)(fold change) (FC), and the y-axis represents -log 10(p values). Editing sites with an adjusted p value of less than 0.05 AND FC>5% were assigned as differentially edited and are indicated in green.

D: Alu Editing index between controls and depressed patients. Distribution of Alu editing index (AEI) values in controls and DEP patients. Data are the mean $\pm$ SEM. p-value of editing index was calculated using the Wilcoxon rank-sum test.

**Figure 2:** Principal component analysis of the 646 variants in 366 genes differentially edited between depressed patients and healthy controls identified in the RNA-Seq study.
The scatter plot visualizes the first, second and third principal components and respective variance percentages on the x-, y- and z-axis of ratio (fold change) of biomarkers. grey circles: DEP patients; black circle: Healthy Controls.

**Figure 3:** Protein-protein associations in the 14 differentially edited genes used in the prioritization cohorts.
Diagram shows the protein-protein interactions in the cluster of 14 genes selected in the prioritization cohort. Genes were annotated and colored regarding to their involvement in Biological processes.

: regulation of immune system process (GO:0002682) ;

: receptor signaling pathway via JAK-STAT (GO:0007259) ;

: regulation of immune response (GO:0050776)

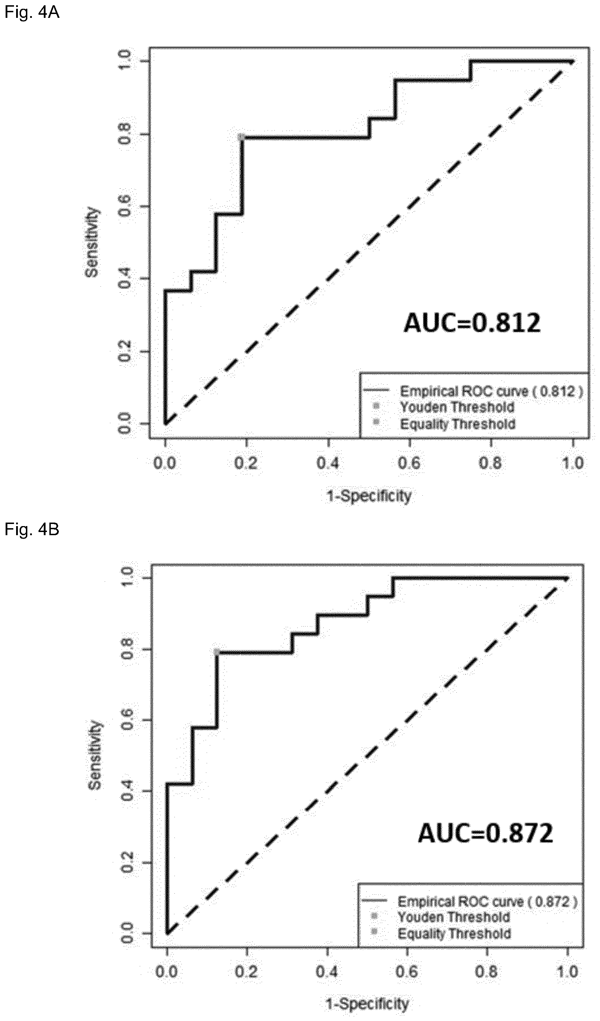

**Figure 4A-4D:** Examples of diagnostic performance of mRNA editing of the first prioritization cohort (n=35) analyzed by single-plex.

A: Example of diagnostic performances obtained by using 2 RNA editing sites in 2 different targets.
B: Example of diagnostic performances obtained by using 3 RNA editing sites in 2 different targets.
C: Example of diagnostic performances obtained by using 4RNA editing sites in 3 different targets.
D: Example of diagnostic performances obtained by using 24 RNA editing sites present in 3 different targets.

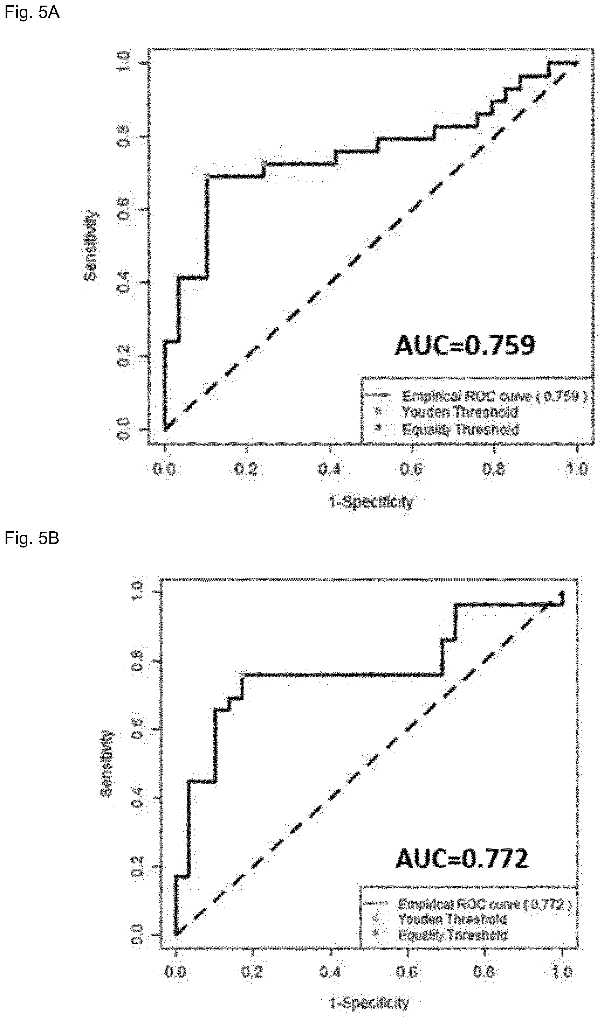

**Figures 5A-5E:** Diagnostic performance of mRNA editing of the second prioritization cohort (n=58) analyzed by multiplex sequencing.

A: Example of diagnostic performances obtained by using 3 RNA editing sites in 2 different targets.
B: Example of diagnostic performances obtained by using 4 RNA editing sites in 2 different targets.
C: Example of diagnostic performances obtained by using 5 RNA editing sites in 3 different targets.
D: Example of diagnostic performances obtained by using 6 RNA editing sites present in 3 different targets.
E: Example of diagnostic performances obtained by using 14 RNA editing sites in 4 different targets.

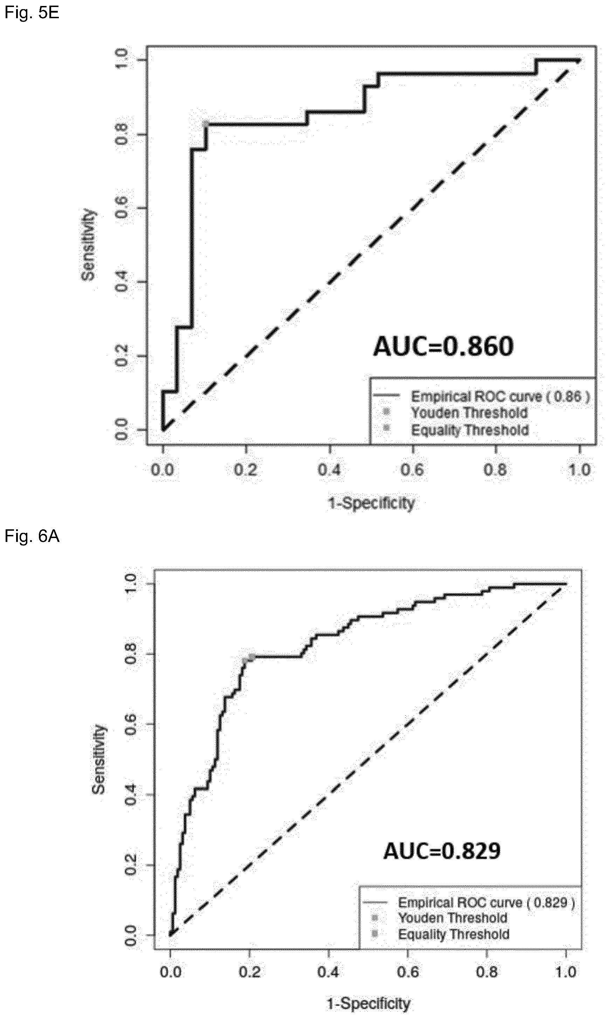

**Figures 6A-6C** Diagnostic performance of mRNA editing of the validation cohort (n=256) analyzed by multiplex sequencing of 8 targets.

A: Example of diagnostic performances obtained by using 29 RNA editing sites in 6 different targets.
B: Example of diagnostic performances obtained by using 32 RNA editing sites in 6 different targets.
C: Example of diagnostic performances obtained by using 44 RNA editing sites in 7 different targets.

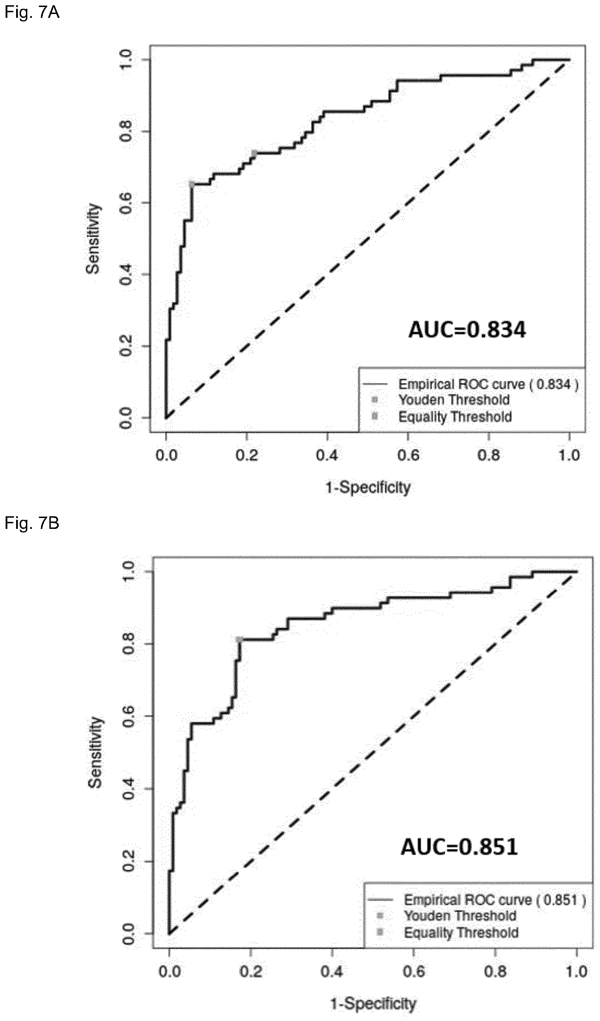

**Figures 7A-7E:** Diagnostic performance of mRNA editing of the female population of the validation cohort (n=179) analyzed by multiplex sequencing of 8 targets.

A: Example of diagnostic performances obtained by using 17 RNA editing sites in 7 different targets.
B: Example of diagnostic performances obtained by using 23 RNA editing sites in 7 different targets.
C: Example of diagnostic performances obtained by using 44 RNA editing sites in 8 different targets.
D: ROC curve obtained after machine-learning with the mROC method.
E: ROC curve obtained after machine-learning with the RandomForest method

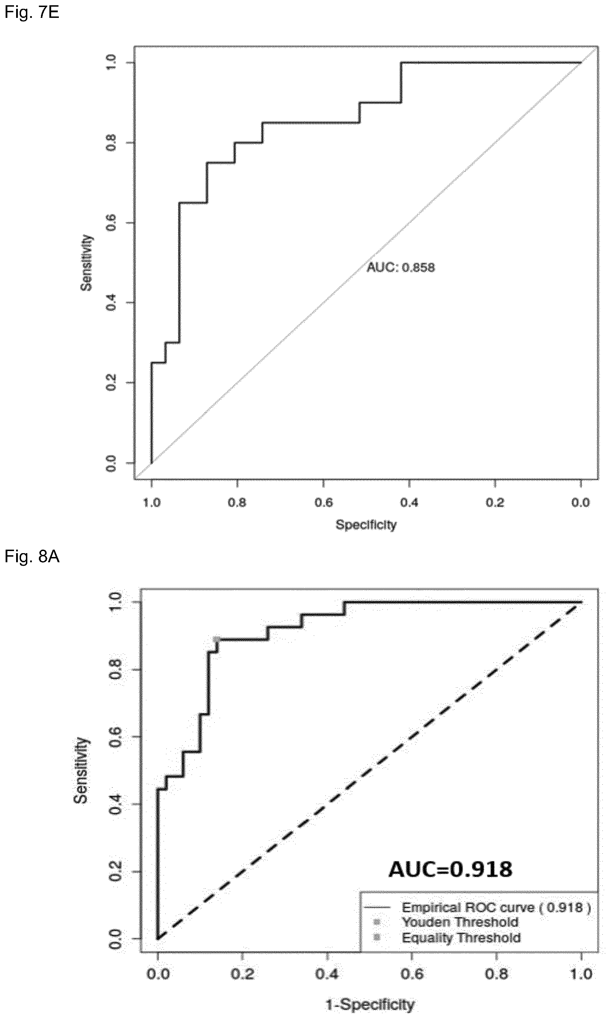

**Figures 8A-8E:** Diagnostic performance of mRNA editing of the male population of the validation cohort (n=77) analyzed by multiplex sequencing of 8 targets.

A: Example of diagnostic performances obtained by using 18 RNA editing sites in 6 different targets.
B: Example of diagnostic performances obtained by using 39 RNA editing sites in 6 different targets.
C: Example of diagnostic performances obtained by using 47 RNA editing sites in 6 different targets.
D: ROC curve obtained after machine-learning with the mROC method.
E: ROC curve obtained after machine-learning with the RandomForest method

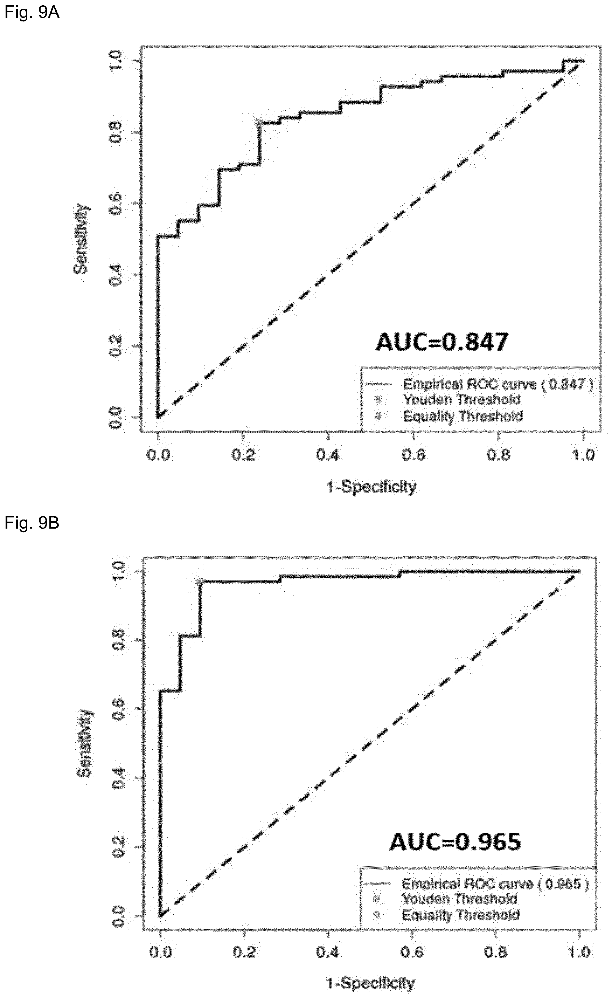

**Figures 9A-9C:** Diagnostic performance of mRNA editing between Bipolar patients and euthymic patients of the female population of the validation cohort (n=90) analyzed by multiplex sequencing of 8 targets.

A: Example of diagnostic performances obtained by using 9 RNA editing sites in 4 different targets.
B: Example of diagnostic performances obtained by using 18 RNA editing sites in 4 different targets.
C: Example of diagnostic performances obtained by using 24 RNA editing sites in 5 different targets.

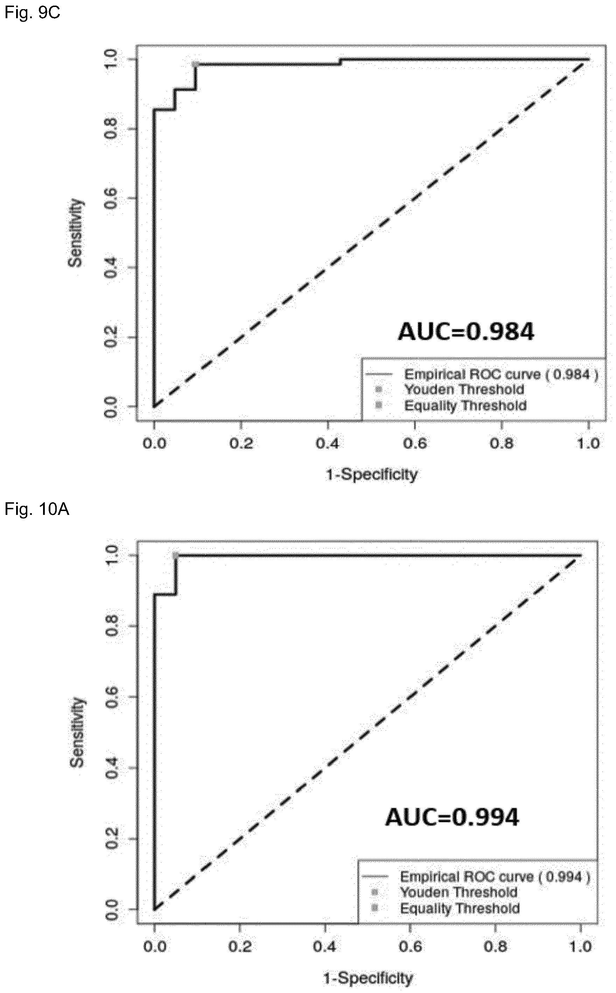

**Figures 10A-10C:** Diagnostic performance of mRNA editing between Bipolar patients and euthymic patients of the male population of the validation cohort (n=47) analyzed by multiplex sequencing of 8 targets.

A: Example of diagnostic performances obtained by using 12 RNA editing sites in 6 different targets.
B: Example of diagnostic performances obtained by using 23 RNA editing sites in 6 different targets.
C: Example of diagnostic performances obtained by using 28 RNA editing sites in 6 different targets.

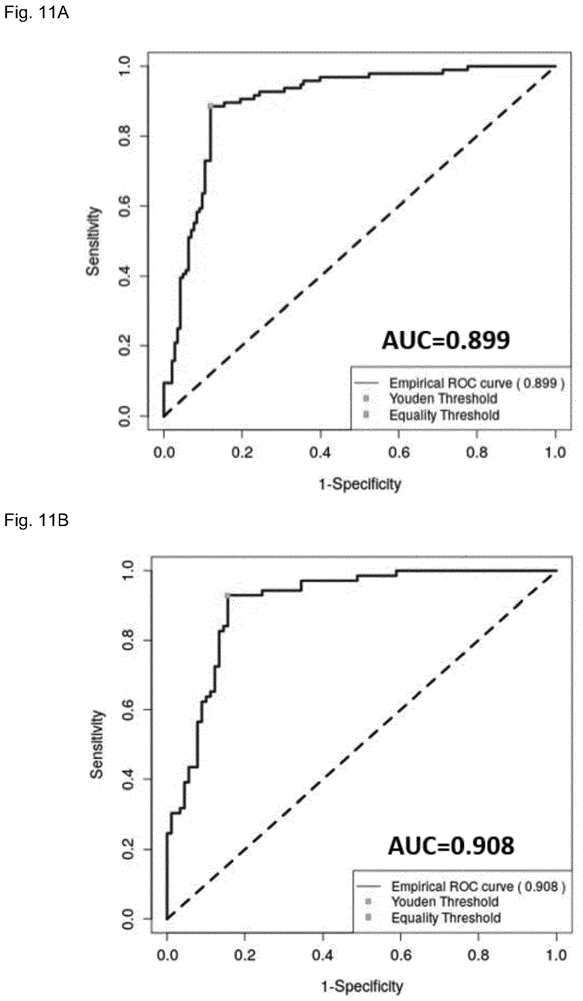

**Figures 11A-11C:** Diagnostic performance of mRNA editing between Bipolar depressive patients and healthy controls (n=239) analyzed by multiplex sequencing of 8 targets.

A: Example of diagnostic performances obtained in the whole population by using 60 RNA editing sites in 7 different targets.
B: Example of diagnostic performances obtained in women by using 32 RNA editing sites in 7 different targets.
C: Example of diagnostic performances obtained in men by using 50 RNA editing sites in 7 different targets.

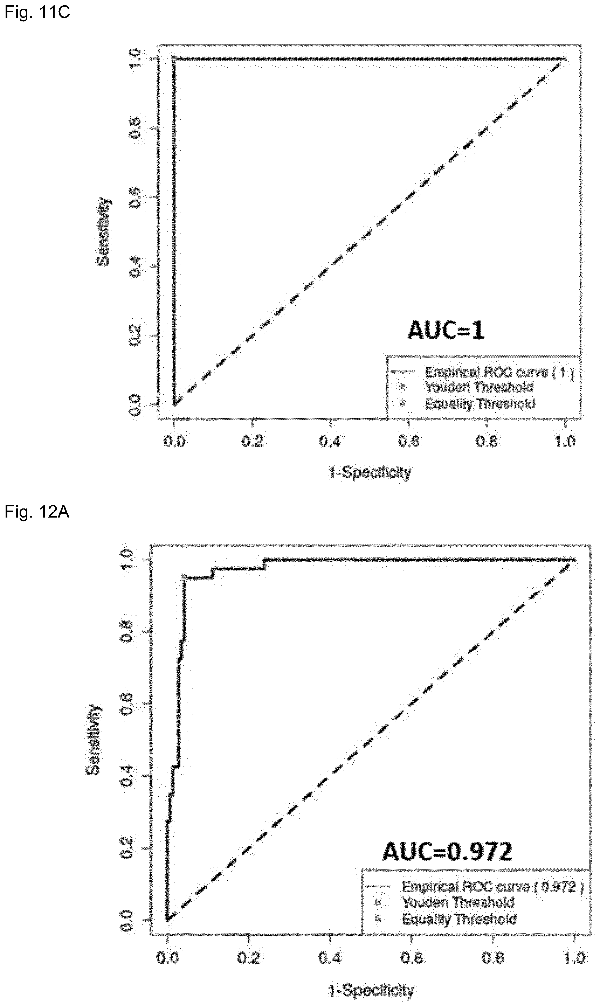

**Figures 12A-12C:** Diagnostic performance of mRNA editing between Bipolar euthymic patients and healthy controls (n=183) analyzed by multiplex sequencing of 8 targets.

A: Example of diagnostic performances obtained in the whole population by using 53 RNA editing sites in 6 different targets.
B: Example of diagnostic performances obtained in women by using 41 RNA editing sites in 8 different targets.
C: Example of diagnostic performances obtained in men by using 35 RNA editing sites in 7 different targets.

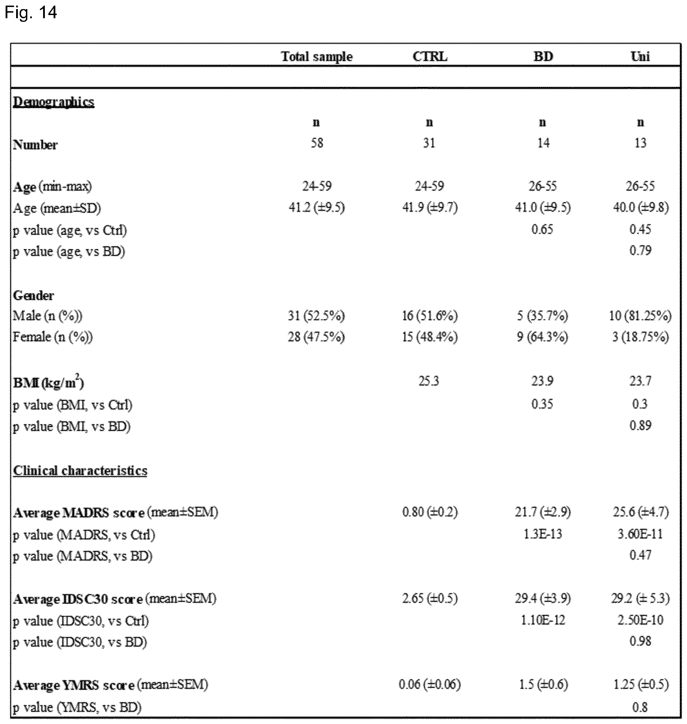

**Figure 14:** Demographic characteristics and psychiatric diagnosis of the study population included in the RNA-Seq study. Data are the mean ± SEM. p-values of main characteristics are displayed with the Student's t-test.; BMI: Body Mass Index

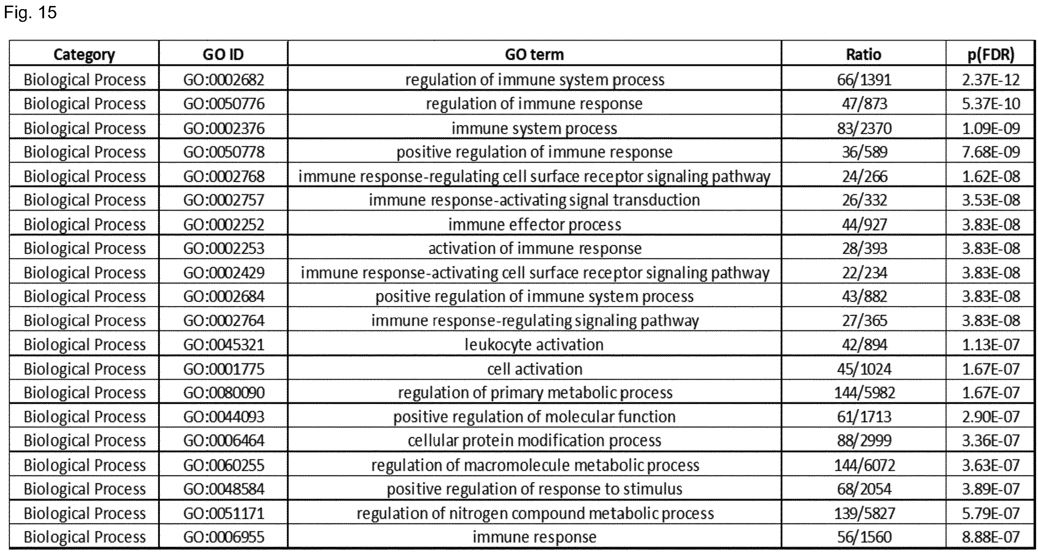

**Figure 15:** Functional categorization of the 366 genes differentially edited between DEP and controls based on gene ontology (GO) annotations.
Shown are the results of top 20 GO over-representation tests for enrichment of Biological Process GO terms with

EP 4 055 191 B1

differentially edited genes. The input into the GO enrichment analysis tools was the list of 366 genes differentially edited between DEP and controls. The ratio of the number of genes/total number of genes included in the GO term and p-values corrected using the Benjamini-Hochberg method for multiple testing are presented. FDR, false discovery rate; GO, gene ontology.

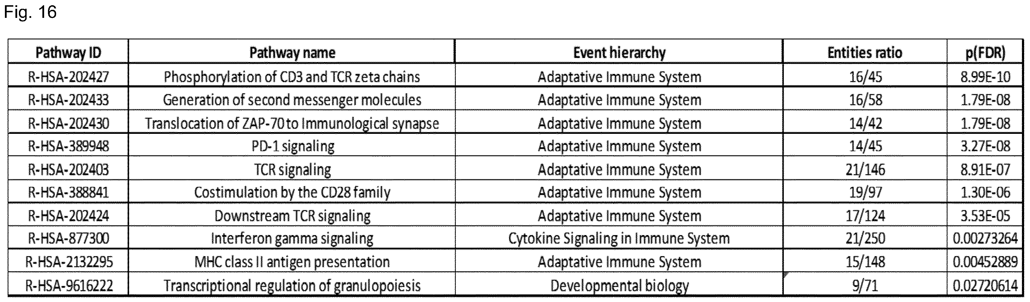

**Figure 16:** Signaling pathway enrichment analysis of the 366 genes differentially edited between DEP and controls. The figure shows the significant pathways obtained performing over-representation analysis in Reactome. The ratio of the number of genes/total number of genes included in the pathway and p-value corrected using the Benjamini-Hochberg method for multiple testing are shown.

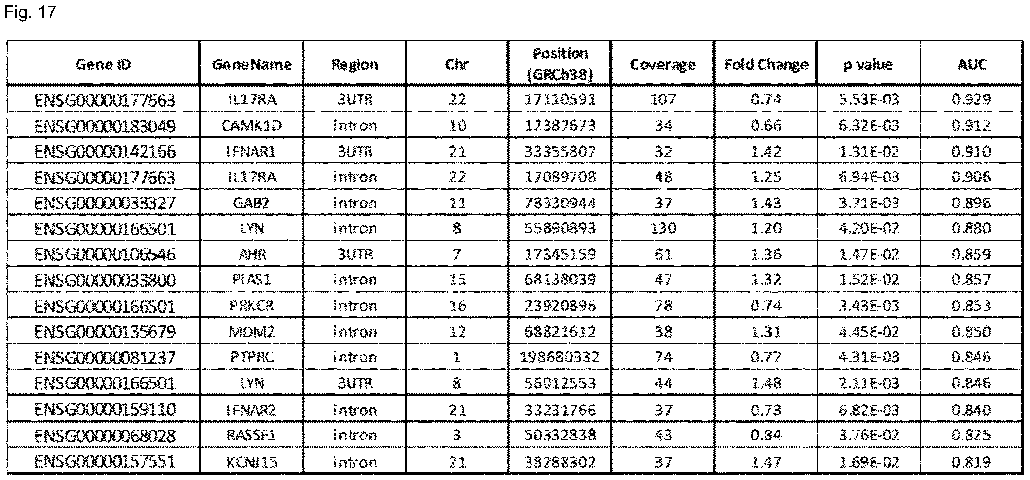

**Figure 17:** Gene and RNA editing variants analyzed in the prioritization cohort.

Shown are the gene and position (GRCh38) of the editing variant, coverage following RNA-Seq, Fold Change between controls and DEP patients, p value and Area under the Curve calculated from RNA-Seq data. p-values were calculated using the Wilcoxon rank-sum test.

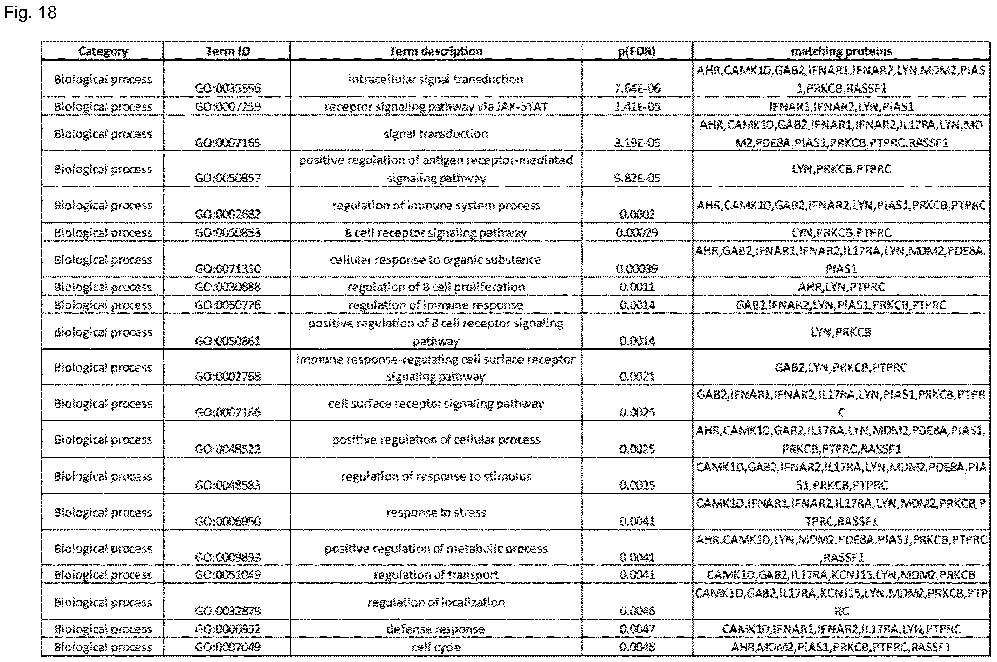

**Figure 18:** Functional categorization of the 14 differentially edited genes used in the prioritization cohorts. based on gene ontology (GO) annotations.

Shown are the results of top 20 GO over-representation tests for enrichment of Biological Process GO terms with differentially edited genes. The input into the GO enrichment analysis tools was the list of 14 genes differentially edited between DEP and controls analyzed in the prioritization cohorts. p-values corrected using the Benjamini-Hochberg method for multiple testing are presented. FDR, false discovery rate; GO, gene ontology.

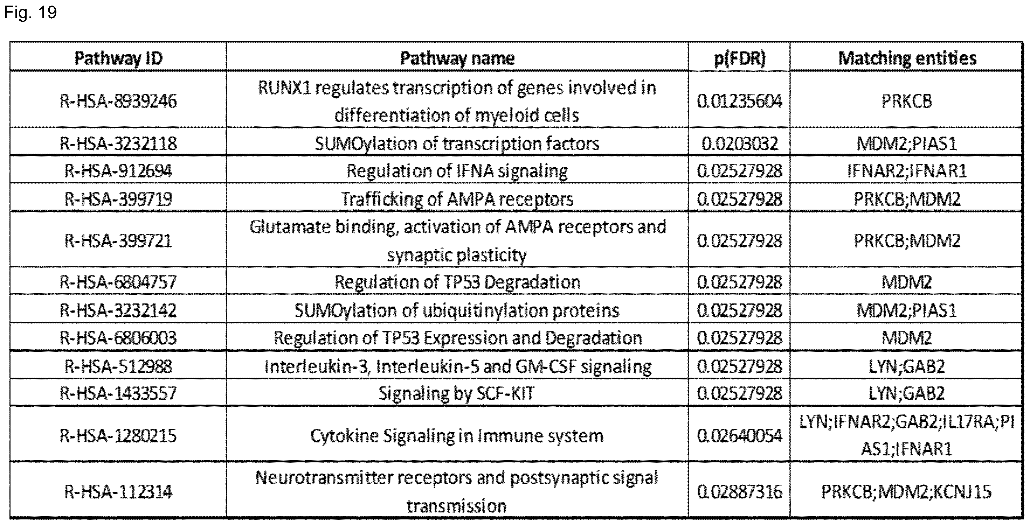

**Figure 19:** Signaling pathway enrichment analysis of the 14 genes analyzed in the prioritization cohorts.

The figure shows the significant pathways obtained performing over-representation analysis in Reactome. p-value corrected using the Benjamini-Hochberg method for multiple testing are shown.

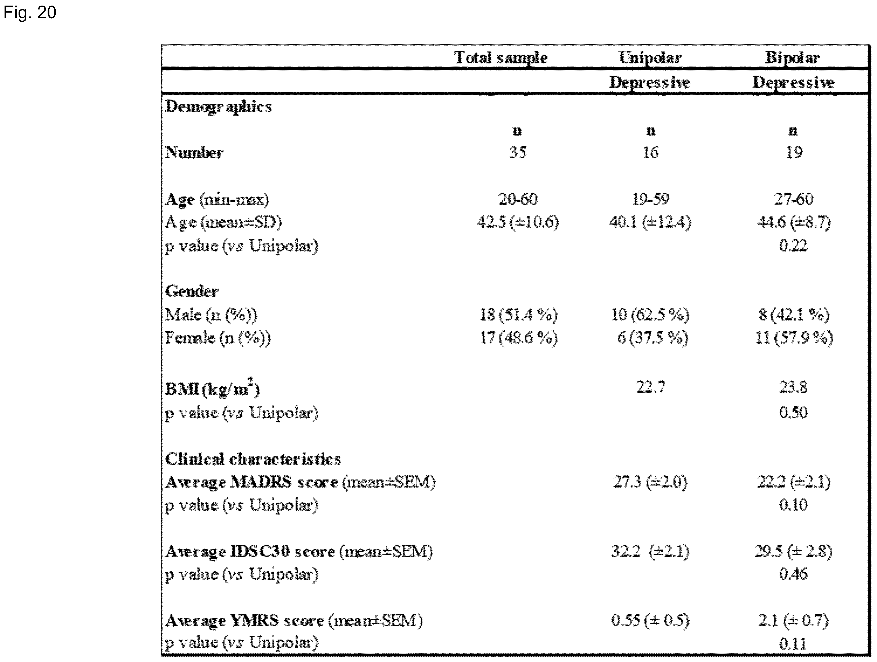

**Figure 20:** Demographic characteristics and psychiatric diagnosis of the study population included the first prioritization study analyzed in single-plex.

Data are the mean ± SEM. p-values of main characteristics are displayed with the Student's t-test. BMI: Body Mass Index. Testing are shown.

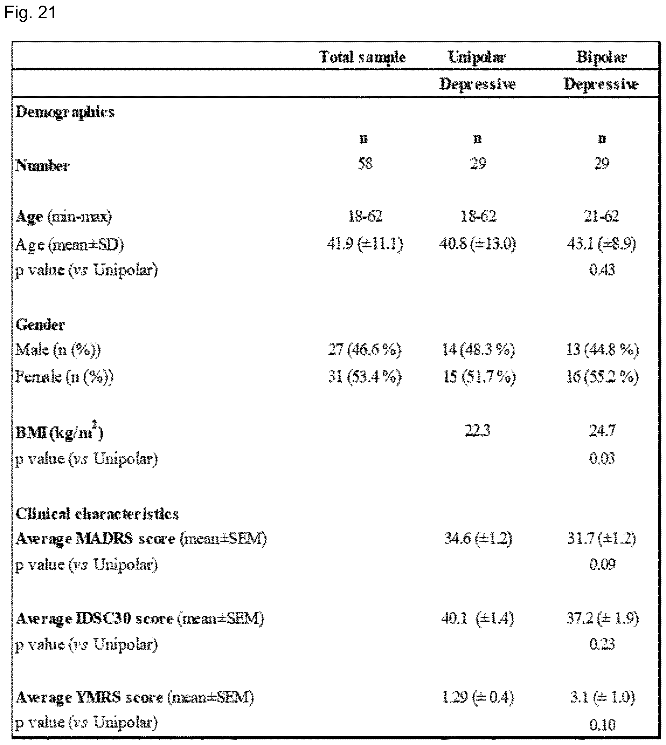

**Figure 21:** Demographic characteristics and psychiatric diagnosis of the study population included the second prioritization study analyzed by with multiplex sequencing.

Data are the mean ± SEM. p-values of main characteristics are displayed with the Student's t-test. BMI: Body Mass Index.

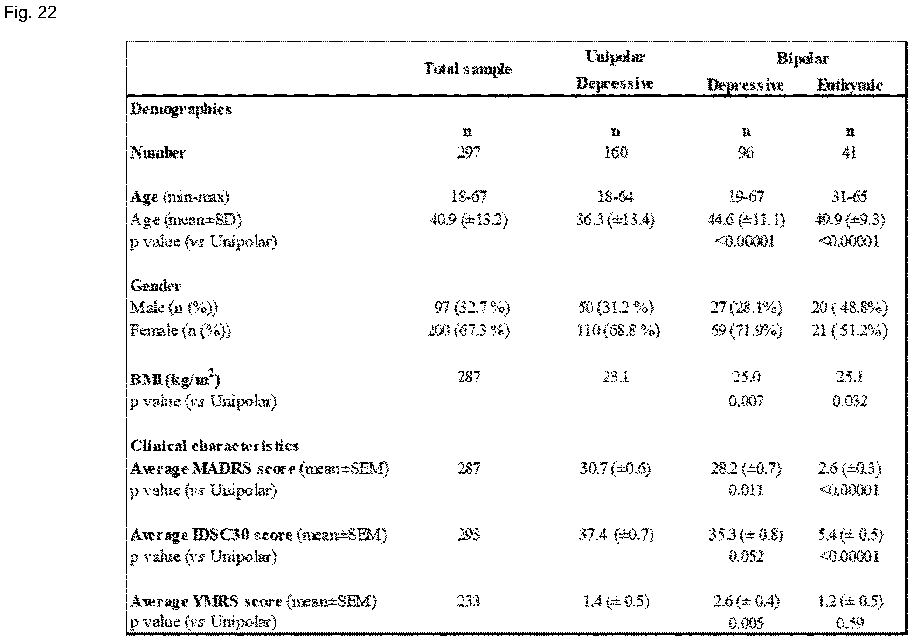

**Figure 22:** Demographic characteristics and psychiatric diagnosis of the study population included in the validation cohort.

Data are the mean ± SEM. p-values of main characteristics are displayed with the Student's t-test. BMI: Body Mass Index

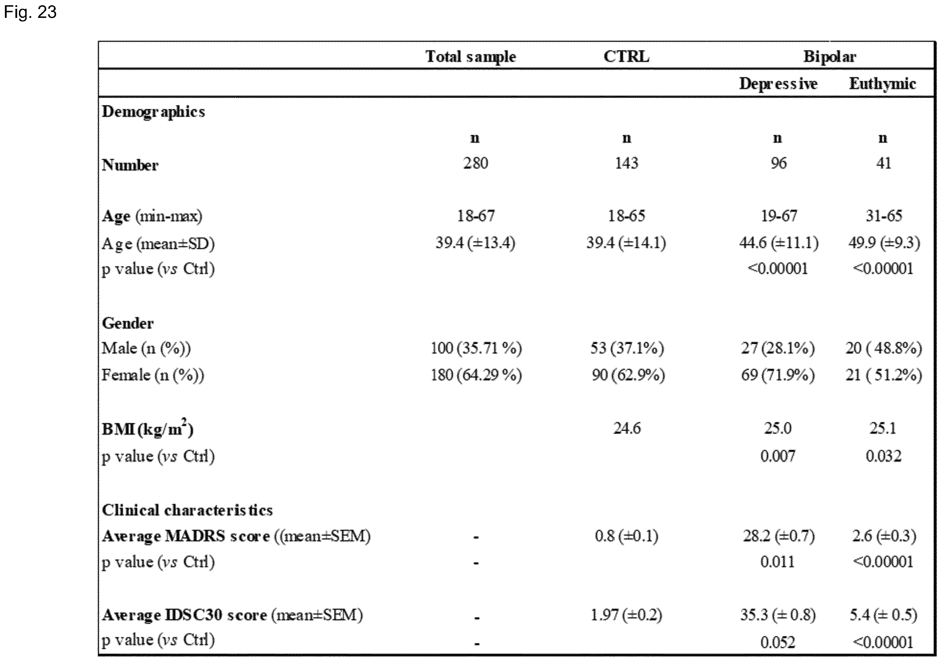

**Figure 23:** Demographic characteristics and psychiatric diagnosis of the healthy controls and Bipolar patients.

Data are the mean ± SEM. p-values of main characteristics are displayed with the Student's t-test. CTRL: Healthy controls; BMI: Body Mass Index

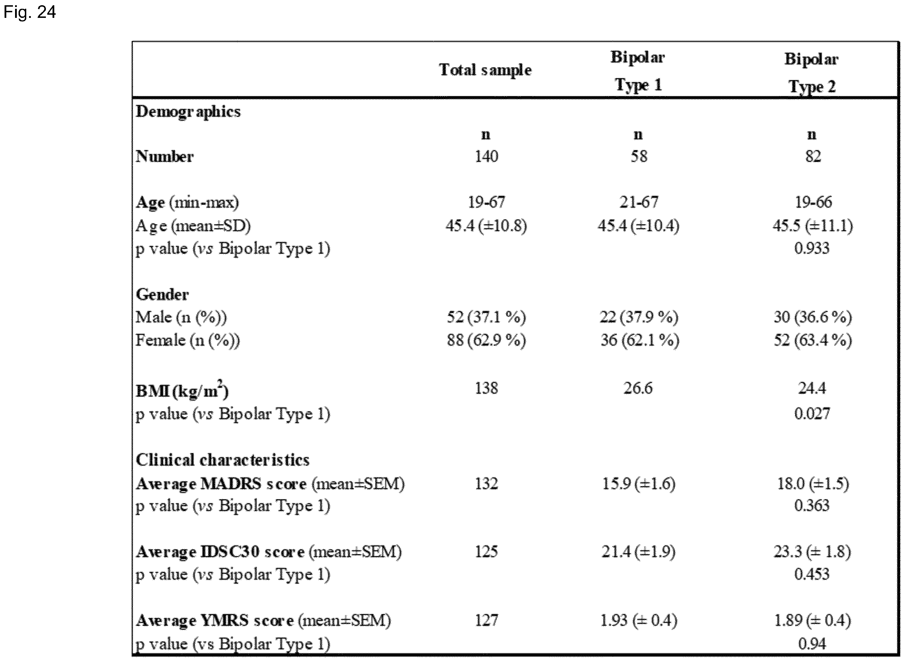

**Figure 24:** Demographic characteristics and psychiatric diagnosis of type I and type II Bipolar patients.

Data are the mean ± SEM. p-values of main characteristics are displayed with the Student's t-test. CTRL: Healthy controls; BMI: Body Mass Index

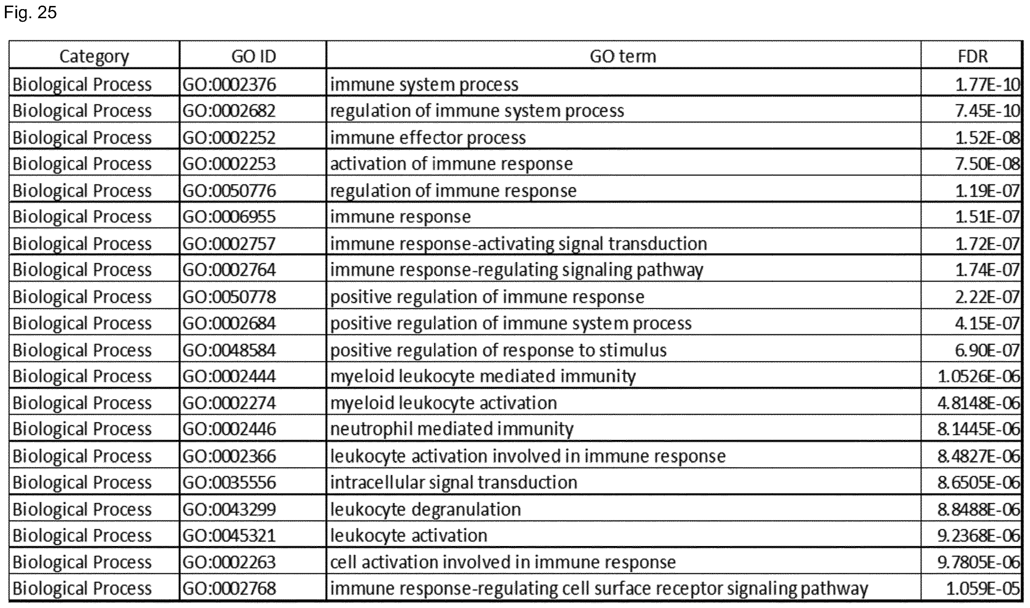

**Figure 25:** Functional categorization of the 366 genes differentially edited between DEP and controls based on g:Profiler annotations.

Shown are the results of top 20 GO over-representation tests for enrichment of Biological Process GO terms with differentially edited genes. The input into the g:Profiler was the list of 366 genes differentially edited between DEP and controls. The ratio of the number of genes/total number of genes included in the GO term and p-values corrected using the Benjamini-Hochberg method for multiple testing are presented. FDR, false discovery rate; GO, gene ontology.

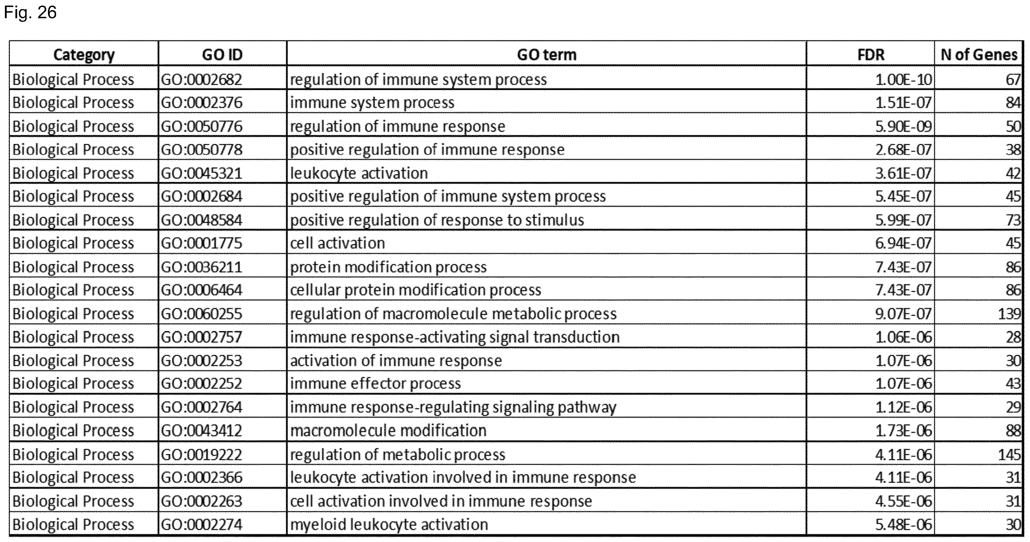

**Figure 26:** Functional categorization of the 366 genes differentially edited between DEP and controls based on GOnet annotations.

Shown are the results of top 20 GO over-representation tests for enrichment of Biological Process GO terms with differentially edited genes. The input into the GOnet was the list of 366 genes differentially edited between DEP and controls. The ratio of the number of genes/total number of genes included in the GO term and p-values corrected using the Benjamini-Hochberg method for multiple testing are presented. FDR, false discovery rate; GO, gene ontology.

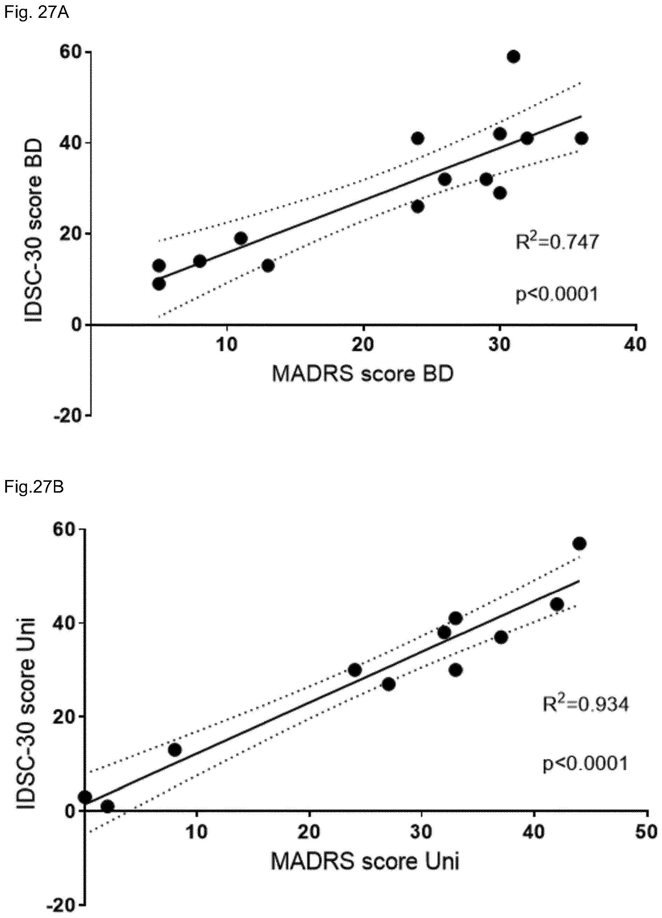

**Figures 27A-27B:** Correlation between clinical MADRS and IDS-C30 scores for the subjects included in the RNA-Seq study.

  A: Correlation between clinical MADRS and IDS-C30 scores for the BD patients. Graph shows the 95% Intervals confidence. The Pearson correlation coefficient and p value are indicated.
  B: Correlation between clinical MADRS and IDS-C30 scores for the Uni patients. Graph shows the 95% Intervals confidence. The Pearson correlation coefficient and p value are indicated.

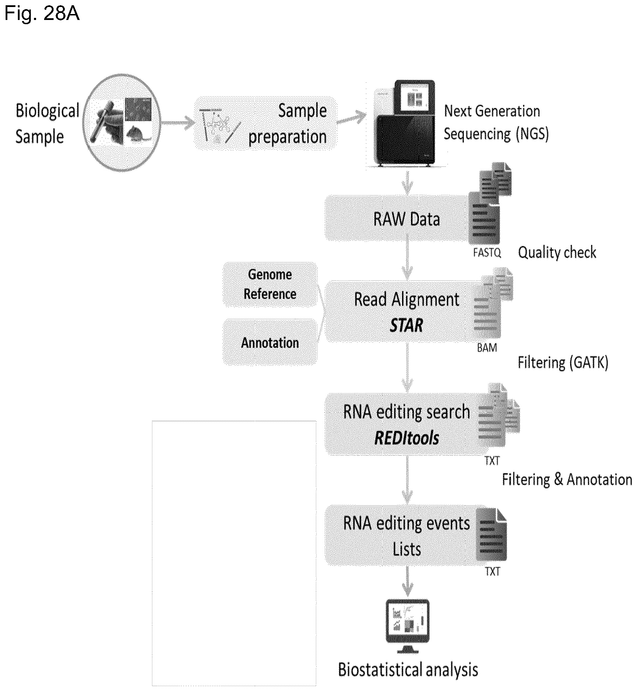

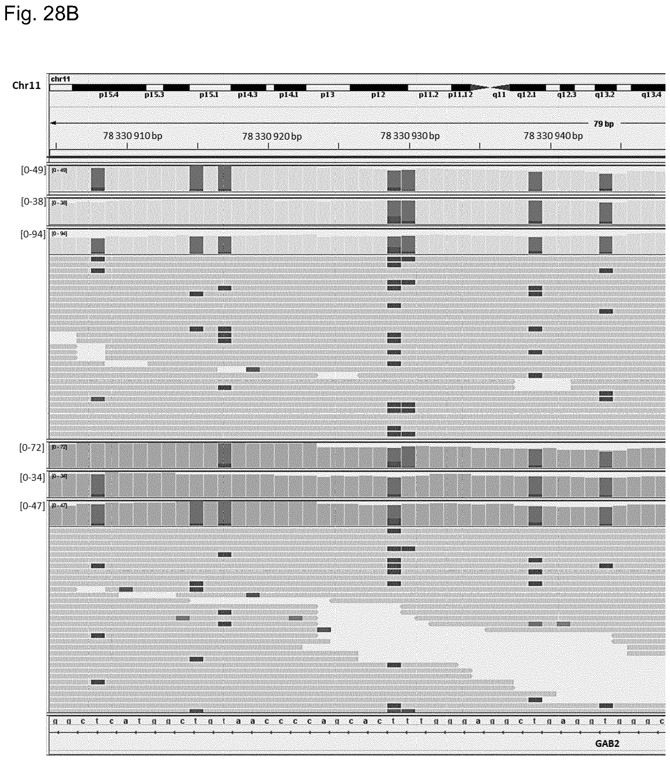

**Figure 28:** Overview of the Editome analysis pipeline

RNA editing search pipeline from editome analysis

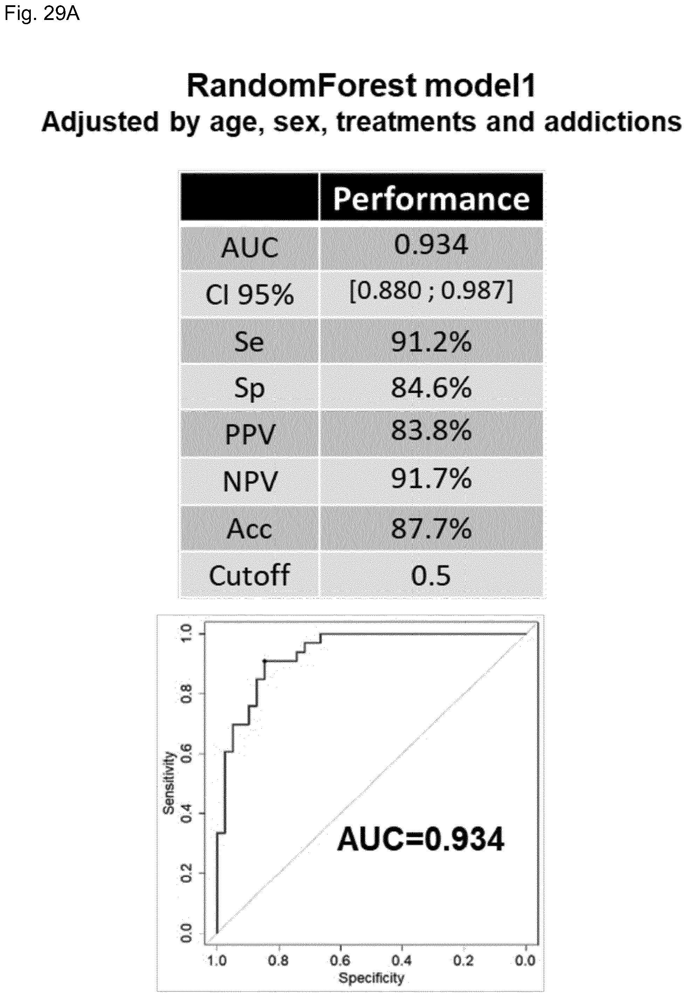

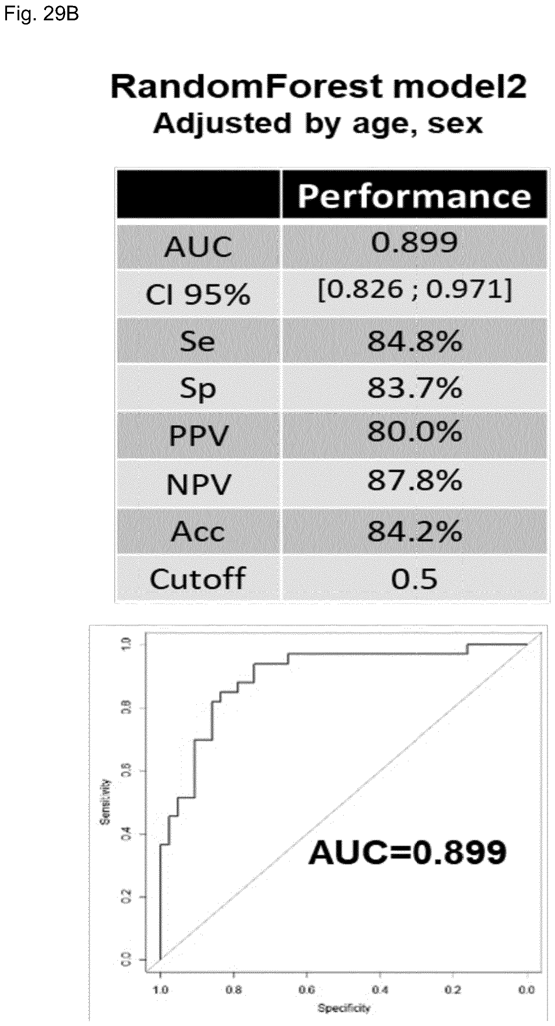

**Figures 29A-29B:** Diagnostic performance of mRNA editing of the validation cohort (n=256) analyzed by multiplex sequencing of 6 targets using RandomForest model1 (FIG.29A) and model2 (FIG. 29B).

A. Receiver operating characteristic curve and diagnostic performances for bipolar depression with the first model adjusted by age, sex, psychiatric treatment and addiction. Plotted are the probabilities for the correct response of the tested sets

B. Receiver operating characteristic curve and diagnostic performances for bipolar depression with the second model adjusted by age and sex. Plotted are the probabilities for the correct response of the tested sets

## EXAMPLE 1: Materials and Methods

### Subjects and clinical assessment

Depressed patients (n=297; DEP) were recruited from the outpatients of the Department of Emergency Psychiatry and Post-Acute Care (CHRU of Montpellier) according to the principles of the Helsinki Declaration of 1975 and its successive updates. This study was approved by the French local Ethical Committee (CPP Sud-Méditerranée IV in Montpellier, CPP No.A01978-41). All participants, aged between 18 and 67 years, understood and signed a written informed consent before entering the study. The study was approved by the local Institutional Review Board, according to the approval requirements and good clinical practice. All patients met the Unipolar depression and Bipolar Disorder criteria in Diagnostic and Statistical Manual of Mental disorders IV (DSM-IV) using the Mini-International Neuropsychiatric Interview. The presence or absence of psychiatric diagnoses and mood states at time entering the study were confirmed by trained psychiatrists. During the standardized interview, psychiatrists managed the French version of the Montgomery-Åsberg Depression Rating Scale (MADRS) and the 30-item Inventory of Depressive Symptomatology, Clinician Rated (IDS-C30). Severity of manic symptoms in Bipolar Disorder was assessed by The Young Mania Rating Scale (YMRS). Importantly, we chose to include only patients whose depression severity levels were moderate and severe (MADRS>20 and/or IDSC-30>24), to avoid avoiding "borderline" patients, for example, patients with low depression scores or patients for whom one depression scale defined them as depressed while the other defined them as healthy. Age-, race- and sex-matched control subjects (n=143) were recruited from a list of volunteers from the Clinical Investigation Center (CHRU of Montpellier).

### RNA extraction and qualification from whole blood

A volume of 4 ml of whole blood from each patient was retrieved in PAXgene™ blood RNA tubes, which are optimized for stabilization of RNA, and stored at -20°C before being transferred to -80°C. Samples were distributed randomly in the different sets of extractions. Blood samples were thawed at 4°C overnight and centrifuged 10 min at 3000g. The pellet was washed with 4ml of DPBS 1X, resuspended in 400μl of DPBS 1X. Samples total RNAs were isolated using the MagNA Pure 96 Cellular RNA Large Volume Kit (LifeScience), according to the manufacturer's automated protocol. RNAs were eluted in 100μl MagNA Pure 96 Elution Buffer. During sample preparation and RNA extraction, standard precautions were taken to avoid RNA degradation by RNAses. Total RNA concentrations and quality levels were determined with Qubit Fluorometer (Invitrogen) and LabChip (Perkin-Elmer, HT RNA Reagent Kit) instruments, respectively.

### Library preparation for RNA sequencing

For RNA library preparation from blood derived RNAs, we chose a specific strategy aiming at depleting in a single step both ribosomal RNA and globin RNAs, which are two forms of abundant RNA, in order to enrich the library with total RNAs (mRNAs). Total RNAs were preferred to messenger RNAs, as it has been shown that RNA editing in blood cells was enriched in intronic region of repetitive elements, which are removed during splicing to produce messenger RNAs. We thus used a TruSeq Stranded Total RNA library kit (Illumina) with Ribo-Zero Globin, which is specifically tailored for blood samples, according to the manufacturer's instructions. Briefly, 300ng total RNAs were depleted in ribosomal RNA and globin RNAs using rRNA and globin depletion probes in combination with magnetic beads. Following purification, the RNAs are fragmented into 250bp fragments in average using divalent cations under elevated temperature. First strand cDNA synthesis was then achieved using Superscript II Reverse Transcriptase (Thermo Fisher Scientific) and random primers. Next, second strand cDNA synthesis is performed using DNA Polymerase I and RNase H. During second strand cDNA synthesis dUTP is incorporated in place of dTTP to generate a strand-specific library. Following second strand cDNA synthesis, the 3' ends of the blunt fragments are 3' adenylated before adapter ligation. Importantly, each index has been attributed to all groups to prevent putative bias due to unspecific effect of index on the depth of sequencing. DNA fragments that have adapter molecules on both ends were then amplified by PCR to increase the amount of DNA in the library. Quality controls were then performed using both Qubit Fluorometer (Invitrogen) and LabChip (Perkin-Elmer, HT DNA 5K Reagent Kit) instruments for quantification of the DNA library templates and quality control analysis, respectively. Samples were then pooled into a library and purified using Magbio PCR cleanup system. The library was denatured using 0.1M NaOH and sequenced on an Illumina NextSeq 500/550 High-Output run using paired-end chemistry with 75-bp read length.

**Processing of RNA-Seq data, differential expression analysis and editome analysis**

Paired-end reads were generated using an Illumina NextSeq 500 and demultiplexed using bcl2fastq (version 2.17.1.14, Illumina). Sequencing quality was performed using FastQC software (version 0.11.7, https://github.com/s-andrews/FastQC). Read alignments were done using the human genome version hg38 with STAR aligner[7], followed by realignment with Genome Analysis Tool Kit (GATK version 4.1.4.0)[8], removal of PCR duplicates removal and final single nucleotide variants (SNV) calling. Identification and quantification of A-to-I editing events was performed using RNAEditor (Version 1.0)[9]. Further filtering and functional annotation of edited events was then performed with ANNOVAR[10], RepeatMasker[11] and BiomaRt[12].

**Targeted Next Generation Sequencing Library preparation and sequencing**

For Next Generation Sequencing (NGS) library preparation, we chose a multiplexed targeted approach to selectively sequence the region of interest within each relevant target. Validated PCR primers were used to amplify the region of interest by PCR (Table 1). For PCR amplification, the Q5 Hot Start High Fidelity enzyme (New England Biolabs) was used according to manufacturer guidelines. The PCR reaction was performed on a Peqstar 96x thermocycler using optimized PCR protocol. Both quantity and quality of the PCR product were assessed. Purity of the amplicon was determined with Nucleic Acid Analyzer (LabChipGx, Perkin Elmer) and quantification was performed using the fluorescence-based Qubit method. After quality control, the PCR reactions were purified using magnetic beads (High Prep PCR MAGbio system, Mokascience). DNA was then quantified using Qubit system and purification yield was calculated. Next, samples were individually indexed by PCR amplification using Q5 Hot start High fidelity PCR enzyme (New England Biolabs) and the Illumina 96 Indexes kit (Nextera XT index kit; Illumina). Samples were then pooled into a library and purified using Magbio PCR cleanup system. The library was denatured using 0.1M NaOH and loaded onto a sequencing cartridge (Illumina MiSeq Reagent Kit V3 or Illumina NextSeq 500/550 Mid-Output) according to Illumina's guidelines. A commercial total RNA pool from human blood peripheral leukocytes (Clontech, #636592) was incorporated into the libraries to determine variability between different sequencing flow cells during the course of the experiment. NGS libraries were spiked in to introduce library diversity using PhiX Control V3 (Illumina) and sequenced (single-read sequencing, read length 150bp) at standard concentrations using deep sequencing (>50K sequences per sample).

**Bioinformatics analysis of targeted sequencing data**

The sequencing data were downloaded from the NextSeq or MiSeq sequencers (Illumina). Sequencing quality was performed using FastQC software (version 0.11.7, https://github.com/s-andrews/FastQC). A minimal sequencing depth of 20 000 reads for each sample was considered for futher analysis. A pretreatment step was performed consisting of removing adapter sequences and filtering of the sequences according to their size and quality score. Short reads (<100nts) and reads with an average QC<20 were removed. To improve sequence alignment quality of the sequences, flexible read trimming and filtering tools for Illumina NGS data were used (fastx_toolkit v0.0.14 and prinseq version 0.20.4). After performing pre-processing steps, an additional quality control of each cleaned fastq file was carried out prior further analysis.

Alignment of the processed reads was performed using bowtie2[13] (version 2.2.9) with end-to-end sensitive mode. The alignment was done to the latest reference human genome sequence[14] (GRCh38). Non-unique alignments, unaligned reads or reads containing insertion/deletion (INDEL) were removed from downstream analysis by SAMtools software[15] (version 1.7). SAMtools mpileup[16] was used for SVN calling. Finding edited position in the alignment was done by using in-house script's in order to count the number of different nucleotides in each genomic location. For each position, the script computes the percentage of reads that have a 'G' [Number of 'G' reads/ (Number of 'G' reads + Number of 'A' reads)*100]. The genomic location 'A' reference with percentage in 'G' reads > 0.1 are automatically detected by the script and are considered as 'A-to-I edition site'. The last stage was to compute the percentage of all possible isoforms of each transcript. By definition the relative proportion of RNA editing at a given editing 'site' represents the sum of editing modifications measured at this unique genomic coordinate. Conversely, an mRNA isoform is a unique molecule that may or may not contain multiple editing modifications on the same transcript. For example for a given transcript, the mRNA isoform BC contains an A-to-I modification on both site B and site C within the same transcript. A relative proportion of at least 0.1% was set as the threshold in order to be included in the analysis.

**Statistical analysis of data**

All statistics and figures were computed with the "R/Bioconductor" statistical open source software (R version 3.5.3)[17] or GraphPad Prism software (version 7.0). Biomarkers (i.e RNA editing sites and isoforms of target genes and mRNA expression of ADARs) values are usually presented as mean $\pm$ standard error of the mean (SEM). In order to guarantee normally distributed data, all biomarker could be transformed using bestNormalize R package (version 1.4.2). In order to guarantee normally distributed data, all biomarker could be transformed using bestNormalize R package (version 1.4.2). A differential analysis was carried out using the most appropriate test between the Mann-Whitney rank-sum test, Student's t-test or Welch's t-test according to normality and sample variance distribution. A p-value below 0.05 was considered as statistically significant.

The relative proportion of RNA editing, on both sites and isoforms, were analyzed by adjusting to the relative transcript level of the reference gene. The adjusted RNA editing values were calculated as following:

$$\text{Adjusted editing value*} = (\text{RNA editing value X relative transcript level})/100$$

The biomarker diagnostic performance could be characterized by: sensitivity, which represents its ability to detect the 'bipolar' group and specificity which represents its ability to detect the 'unipolar' group. The results of the evaluation of a diagnostic test can be summarized in a 2x2 contingency table comparing these two well-defined groups. By fixing a cut-off, the two groups could be classified into categories according to the results of the test, categorized as either positive or negative. Given a particular biomarker, one can identify a number of A patients with a positive test result among the bipolar group (the "True Positive": TP) and B patients with a negative test result among the 'unipolar' group (the "True Negative": TN). In the same fashion, C patients with a negative test result among the 'bipolar' group (the "False Negative": FN) and D patients with a positive test result among the 'unipolar' group (the "False Positive": FP) are observed. Sensitivity is defined as TP/(TP+FN); which is herein referred to as the "true positive rate". Specificity is defined as TN/(TN+FP); which is herein referred to as the "true negative rate".

The accuracy of each biomarker and its discriminatory power was evaluated using a Receiving Operating Characteristics (ROC) analysis[18]. ROC curves are the graphical visualization of the reciprocal relation between the sensitivity (Se) and the specificity (Sp) of a test for various values. In addition, all biomarkers were combined with each other to evaluate the potential increase in sensibility and specificity using multiple approaches as for example mROC program[19] or RandomForest[20].

mROC is a dedicated program to identify the linear combination[21,22], which maximizes the AUC (Area Under the Curve) ROC[23]. The equation for the respective combination is provided and can be used as a new virtual marker Z, as follows:

$$Z = a \text{ X biomarker } 1 + b \text{ X biomarker } 2 + c \text{ X biomarker } 3$$

where a, b, c are calculated coefficients and biomarkers 1,2,3 are the level of the considered biomarker.

Random Forest (RF) was applied as previously to assess the RNA editing isoforms/sites combinations. This method combines Breiman's "bagging" idea[20] and the random selection of features in order to construct a collection of decision trees with controlled variance. So, random forests can be used to rank the importance of editing isoform and to combine the best isoforms. Multiple down-sizing (MultDS) asymmetric bagging with embedded variable classifier was implemented[24]. This method tries to take advantage of the whole information of majority calls by multiple down sampling but keeping the minority class fix. Our algorithm generated 100 random forest from the training set, each containing the same fix number of the minority class and equal or adjusted number of the randomly sampled, generating nearly balanced trees. For classification of new data, the results afterwards were combined by majority voting of the trained 100 random forest. To estimate RF parameters for each trained 100 randomForest, a 10-fold cross-validation were applied. The implementation was done using the R randomForest package (version 4.6-14) and R caret package (version 6.0-84).

**EXAMPLE 2: Results**

**Characteristics of the Discovery Study Samples**

Patients included in the RNA-Seq samples were matched with respect to all variables, e.g. sex, age, or BMI, which were not statistically different between groups (Figure 14). We chose two different clinical evaluations to confirm the presence of psychiatric diagnoses, namely MADRS and IDSC-30, both designed by the American Psychiatry Association Diagnostic and Statistical Manual of Mental Disorders. Unipolar and Bipolar patients were both classified as depressed according to clinical scores in MADRS and IDSC-30 depression scales, and were statistically significant from healthy controls (Figure 14). Moreover, we observed a significant association between the two clinical evaluations (for BD ($r^2$=0.747, p<0.0001 for BD, $r^2$=0.934, p<0.0001 for Uni Figures 27A and 27B), suggesting a correct assignment of patients and a relative homogeneity in the scoring of depression. There was no statistical difference in the severity of manic symptoms assessed by the YMRS between patients suffering from Unipolar depression or those suffering Bipolar Disorder (Figure 14), suggesting that the BD patients included in the study were in an actual depressive episode.

**Editome analysis**

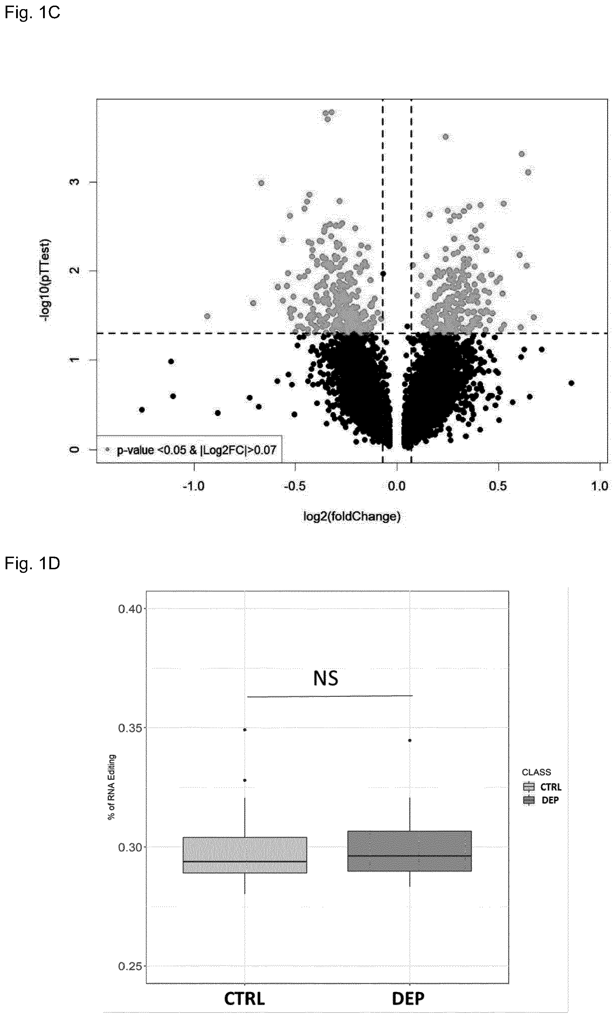

To obtain a global landscape of the modification of RNA editing events in depressed patients, we analyzed the RNA-Seq dataset using an in-house Editome analysis pipeline (see methods Figure 28). We identified 37, 599 A-to-I differentially edited positions with a minimum coverage of 30x, which ensures a high degree of confidence at particular base positions. The coverage describes the average number of reads that align to, or "cover," known reference bases. Most of the edited position (32, 183 sites, 85.6% of total) were present in introns (Figure 1A), consistent with other studies in human tissues[25]. The percentage of edited position in 3' untranslated regions (3'UTR) of mRNAs was 6.2% (2344 sites), in line with previous reports using RNA-Seq. The number of editing sites had a homogeneous repartition all over the genome, though edited genes on chromosomes 4, 9, 18, 19, 20, 21 and 22 were slightly overrepresented (Figure 1B). We then performed a differential analysis to identify sites whose editing could be specifically different between DEP and healthy controls. After applying pre-specified quality criteria (exclusion of intergenic sites, coverage >30; AUC>0.6; 0.95>FoldChange>1.05 and p<.05,), we identified 646 variants differentially edited between depressed patients and healthy controls (Figure 1C), representing 366 genes. The quality criteria were set in order to provide potential diagnostic biomarkers (BMKs) for depressive disorders with clinical usefulness. Importantly, no difference in global RNA editing was observed between patients and controls. Indeed, we calculated the global *Alu* editing index (AEI), which is a measure of the overall rate of RNA editing in *Alu* repeats. *Alu* repeats are by far the most common edited region in the human genome, and thus are indicative of the global editing rate[26]. We found no significant differences in AEI between controls and depressed patients (AEI control mean=0.300$\pm$0.002, AEI DEP mean=0.299$\pm$0.003, p value=0.57, Figure 1D). As the Alu Editing index characterizes the weighted average editing level across all expressed *Alu* sequences, these data suggest that differential RNA editing was target-specific rather than dependent on the disease status.

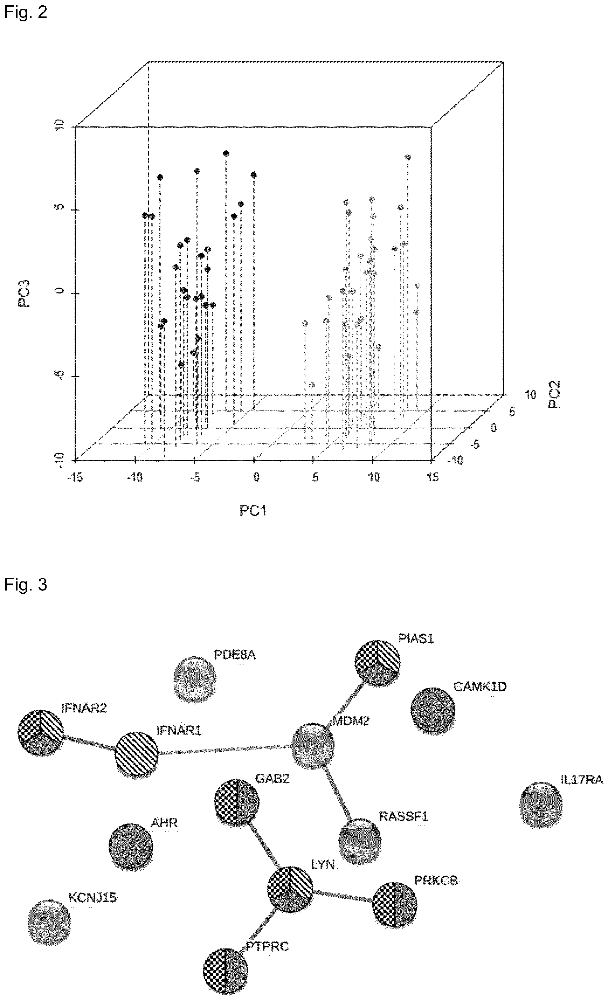

GSEA (enrichment analysis on gene sets) on these 366 genes using gene ontology tools[27] displayed a strong term enrichment (false discovery rate <0.05) for biological processes, including multiple immune categories (response, activation and regulation), cell activation and metabolic processes (Figure 15). This analysis was replicated with other tools, such as G:profiler[28] (Figure 25) or GOnet[29] (Figure 26), and consistently indicated a strong enrichment in biological processes related to immune system. Moreover the reactome pathways analysis of those 366 genes revealed a strong enrichment in pathways related to the immune system, i.e. TCR signaling, Translocation of ZAP-70 to Immunological synapse or Interferon gamma signaling (Figure 16). Noteworthy, Principal Component Analysis (PCA) showed that this subset of the 646 editing variants representing 366 genes clearly discriminated controls and depressed patients in 2 separate groups (Figure 2), suggesting that editing variants in those genes could be used in combination to selectively discriminate between depressed patients and healthy controls.

To further narrow down the list of genes, much more stringent quality criteria were used (coverage >30; AUC>0.8; 0.8>FoldChange>1.20 and p<0.05). The database of these markers was then manually curated to reach a combination of several biomarkers representing different biological mechanisms. Using these secondary criteria, a list of 15 variants, representing 13 genes (Figure 17), was selected for both diagnostic performance and for their potential role in the immune system or psychiatric disease biology and to be studied in a subsequent prioritization cohort. Additionally, we nominated one other target, PDE8A, for which previous data indicated altered editing in suicide brain and in blood of drug-induced depression in HCV patients[30,31]. Protein-protein association networks analysis with STRING database[32] revealed a network with strong enrichment in protein-protein interaction (PPI pvalue <4.5.10$^{-6}$). The interactions inside STRING were based on evidence and consist of direct (physical) and indirect (functional) interactions; we focused here on a minimum required interaction score of 0.7, indicative of high confidence in actual protein-protein interactions. Functional protein association network analysis of the 14 selected genes using gene ontology tools[27] also showed a strong enrichment in genes related to: regulation of immune system process (8 genes, GO:0002682; p(FDR=4.10$^{-4}$), regulation of immune response, (6 genes, (GO:0050776, p(FDR=2.2.10$^{-3}$)) or receptor signaling pathway via JAK-STAT (4 genes, GO:0007259), which is typically triggered following interferon-alpha stimulation (Figure 3 and Figure 18). Furthermore, Reactome pathways analysis indicated that those 14 genes were enriched in pathways related to immune system, (e.g. Regulation of IFNA signaling (HSA-912694, p(FDR)<0.025) or Cytokine signaling (HSA-1280215, p(FDR)<0.026)) and neurotransmitter regulation (e.g. Trafficking of AMPA receptors (HSA-399719, p(FDR)<0.025), glutamate binding and synaptic plasticity (HAS-399721, p(FDR)<0.025) or Neurotransmitter receptors and postsynaptic signal transmission (HSA-112314, p(FDR)<0.029)) (Figure 18). Interestingly, the Top pathway identified in the Reactome analysis (p(FDR)<0.012) is linked to the transcription factor Runx1, which has recently been shown to mediate the effect of chronic social defeat stress in a mouse depression model through regulation of the miR-30 Family of miRNAs[33].

**Target description**

**PRKCB:** Protein Kinase C Beta (PRKCB), which belongs to the family of serine- and threonine-specific protein kinases, is well known to play a crucial role in the immune system, as Mice knockout for PKCB develop immunodeficiency characterized by impaired B-cell activation, B cell Receptor response, and defects in T-cell-independent immune responses. PRKCB also act as a regulator of the HPA axis response to stress[34]. An association study of 44 candidate genes with depressive and anxiety symptoms in post-partum women revealed an association between PRKCB and major depression. Indeed, PRKCB interacts with and phosphorylates CREB1, which directly regulates the expression of several genes involved in stress response in the brain, such as BDNF, the BDNF receptor Trk-b, and the glucocorticoid receptor[35].

**MDM2:** MDM2 is a primary cellular inhibitor of p53, and might be a target for anticancer treatment. However, MDM2 also catalyzes ubiquitination of β-arrestin, which is a target for antidepressants. In the CNS, MDM2 is involved in AMPAR (glutamate receptor) surface expression during synaptic plasticity[36]. Interestingly, RNA editing in MDM2 has been shown to regulate protein levels. Indeed, increased RNA editing at the 3' UTR of MDM2 abolishes microRNA-mediated repression, which may increase its mRNA levels.

**PIAS1:** PIAS1 (Protein Inhibitor Of Activated STAT 1) function as a SUMO ligase which blocks the DNA binding activity of Stat1 and inhibited Stat1-mediated gene activation in response to interferon. Sumoylation of transcription factors (HSA-3232118) is one of the major Reactome pathways in the 14 genes selected for analysis in the prioritization cohort (Figure 19). PIAS1 selectively inhibits interferon-inducible genes and is an important player in innate immunity[37]. In addition, Pias1 interacts with the presynaptic Metabotropic Glutamate Receptor 8 (mGluR8) and add a post transcriptional modification by sumoylation, suggesting that PIAS1 might have regulatory functions in targeting and modulating mGluR8 receptor signaling.

**IFNAR1:** A wealth of studies have linked interferons to inflammation-induced changes in brain function and depression, at least in part though the effect of IL-6, CXCL10 or by induction of indoleamine 2,3-dioxygenase1 (IDO1)[38]. Moreover, it has been shown that polymorphisms in the promoter region of the IFN-alpha/beta receptor 1 (IFNAR1) can influence the risk of developing depression. Interferons are used for the treatment of viral hepatitis, hemato-proliferative disorders, autoimmune disorders and malignancies. However, irrespective of the indication for IFN therapy, IFNs are associated with a 30-70% risk of treatment-emergent depression. In a previous work we have shown that RNA editing biomarkers allowed discrimination between patients who developed a treatment-emergent depression and those who did not.

**IFNAR2:** Interferons act through their receptors; the interferon type I receptor (IFNaR) is composed of two subunits, IFNAR1 and IFNAR2. A polymorphism in the IFNAR2 gene (SNP 11876) showed an association with multiple sclerosis susceptibility (p = 0.035). Interestingly, the same study also identified a polymorphism in the IFNAR1 gene (SNP 18417), which is a member of the same receptor.

**LYN:** LYN encodes a non-receptor tyrosine-protein kinase that transmits signals from cell surface receptors. Lyn plays an important role in the regulation of innate and adaptive immune responses[39]. In the mammalian central nervous system, Lyn is highly expressed in the nucleus accumbens, cerebral cortex and thalamus. Moreover, Lyn participates in the control of N-methyl-D-aspartate receptor (NMDAR, an ionotropic glutamate receptor) receptor pathway. Indeed, behavioral tests showed that spontaneous motor activity is suppressed in Lyn-/- mice, due to enhancement of NMDA receptor signaling. Furthermore, Lyn physically interact with AMPA receptor, a member of the glutamate receptor family[40]. Following stimulation of the receptor, Lyn activates the mitogen-activated protein kinase (MAPK) signaling pathway, which in turn increases expression of brain-derived neurotrophic factor (BDNF). Importantly, activation of AMPA receptors along with release of BDNF and activation of downstream synaptogenic signaling pathways mediate the antidepressant effects of ketamine. Lyn kinase-glutamate receptor interactions undergo drastic adaptations in mood disorders such as major depressive disorder.

**AHR:** The aryl hydrocarbon receptor (AHR) is a highly conserved ligand-dependent transcription factor that senses environmental toxins and endogenous ligands. AHR also modulates immune cell differentiation and response[41]. AHR also controls both T(reg) and Th17 cell differentiation in a ligand-specific fashion. Activation AHR induces IDO and thus increases kynurenine levels. The role of Kynureine is well documented in the CNS, where it plays important roles in the pathophysiology of inflammation-induced depression. Pharmacological blockade of AHR rescued systemic inflammation-induced monocyte trafficking, neuroimmune disturbance, and depressive-like behavior in mice. RNA Editing affects the 3'UTR of the human AHR mRNA, thereby creating a microRNA recognition sequence which modulates AHR expression.

**RASSF1:** Ras association domain family member 1 (RASSF1) RASSF1A is a tumor suppressor gene whose inactivation has been implicated in a wide variety of sporadic human cancers and is epigenetically inactivated at high frequency in a variety of solid tumors. RASSF1A associates with MDM2 and enhances the self-ubiquitin ligase activity of MDM2[42]. Interestingly, a microarray analysis recently identified RASSF1A as a significantly downregulated gene in women with history of postpartum depression.

**GAB2:** Gab proteins function downstream of multiple receptors, such as the receptors for EGF, hepatocyte growth factor, and FGFs. GAB2 is an important component of neuronal differentiation induced by retinoic acid, partly by acting downstream of bFGF to mediate survival through PI3 kinase-AKT. GAB2 positively upregulated IL-4 and IL-13 expression in activated T cells, suggesting that GAB2 might be an important regulator of the human Th2 immune response[43].

**CAMK1D:** CAMK1D is a member of the calcium/calmodulin-dependent protein kinase 1 family and is involved in the chemokine signal transduction pathway that regulates granulocyte function[44]. In the CNS, CAMK1D is mostly expressed in the CA1 region of the hippocampus, where it is induced following Long-term potentiation (LTP), a long lasting enhancement of synaptic efficacy in the central nervous system. It could translocate to the nucleus in response to stimuli that trigger intracellular Ca2+ influx, e.g. glutamate, and activate CREB-dependent gene transcription. Noteworthy, SNP rs2724812 in CAMK1D has been associated to remission after selective serotonin reuptake inhibitors treatment by a deep learning approaches.

**KCNJ15:** Potassium voltage-gated channel subfamily J member 15 (KCNJ15) encodes an inward-rectifier type potassium channel, Kir4.2. In the CNS, the KIR4.2 channel is expressed late in development in both mice and human brain. Inwardly rectifying potassium channels (Kir channels) are important regulators of neuronal signaling and membrane excitability. Gating of Kir channel subunits plays crucial role in polygenic CNS diseases. Moreover, rs928771 in KCNJ15 was found to be associated with alterations in KCNJ15 transcript levels in whole blood; network analysis of hippocampus and blood transcriptome datasets suggested that suggests that the risk variants in the KCNJ15 locus might act through their regulatory effects on immune-related pathways[46].

**IL17RA:** The Interleukin 17 Receptor A (IL17RA) is a type I membrane glycoprotein, which is a similar structure to IFNAR1, that binds with low affinity to interleukin 17A. Interleukin 17A (IL17A) is a proinflammatory cytokine primarily secreted by activated Th17 lymphocytes. Upon biding to its cognate receptor, IL17 signals through ERK, MAPK and NFKB pathways, all of which have been implicated in depressive disorder.

Th17 cells are potent regulators of brain function as they target various brain resident cells including neurons, astrocytes, and microglia and participate in neuroinflammation[47]. IL17RA is highly expressed in blood-brain barrier (BBB) endothelial cells in multiple sclerosis lesions, where IL17 increases BBB permeability by disrupting tight junctions, thereby promoting CNS inflammation. Pro-inflammatory IL17 is also secreted by activated astrocytes and microglia and has detrimental effect on neurons. Flow cytometry analysis of PBMCs in unmedicated depressed subjects revealed a significant increase in peripheral Th17 cell number, and elevated serum concentration of IL-17, suggesting a potential role of Th17 cells in MDD patients. Similarly, Th17 cells are increased in the brain of mice after learned helplessness or chronic restraint stress, which are two common depression-like models. Inhibiting the production or function of Th17 cells reduces vulnerability to depression-like behavior in the same mouse models

**PTPRC:** Protein Tyrosine Phosphatase, Receptor Type C (PTPRC), also known as leukocyte-common antigen, or CD45, is an integral membrane protein tyrosine phosphatase, which contains an external segment and is capable of initiating transmembrane signaling in response to external ligands[48]. In the immune system, PTPRC is essential for activation of T and B cells by mediating cell-to-cell contacts and regulating protein-tyrosine kinases involved in signal transduction. PTPRC suppresses negatively regulates cytokine receptor signaling; selective disruption of PTPRC expression leads to enhanced interferon-receptor-mediated activation of JAK and STAT kinases proteins[49]. Remarkably, a point mutation in PTPRC (C-to-G nucleotide transition in position 77 of exon 4) has been associated with the development of multiple sclerosis, the most common demyelinating disease of the CNS. In the brain, anxiety-like behavior induced by repeated social defeat, a mouse model of depression, corresponded with an increase in brain macrophages overexpressing PTPRC. In the same mouse model of depression, classical benzodiazepines were shown to reverse symptoms and to block stress-induced accumulation of macrophages expressing high levels of PTPRC in the CNS. Furthermore, in patients diagnosed with major depressive disorder, PTPRC was higher in patients than in healthy subjects. PTPRC ratio decreased with antidepressant treatment such as venlafaxine or fluoxetine and was positively correlated with the severity of depression[50], suggesting that antidepressant treatment contributes to immune regulation in patients with major depressive disorder.

**PDE8A:** Glutamate or serotonin receptors are G-protein-coupled receptors (GPCRs) involved in a variety of psychiatric disorders, for which the spatial and temporal regulation of second messenger concentration is crucial for subsequent signaling. Phosphodiesterases (PDE) are key modulators of signal transduction and are involved in inflammatory cell activation, behavior, memory and cognition. PDE8A is a cAMP-specific phosphodiesterase expressed in the brain and blood. Altered RNA editing in PDE8A transcripts has been identified in in T cells of patients suffering from systemic lupus erythematosus, a chronic autoimmune disorder, as in normal T cells with IFN-$\alpha$. There is a two-fold decrease in the expression of phosphodiesterase 8A (PDE8A) in the temporal cortex of major depressive disorder (MDD)

patients[51]. We have recently shown brain region-specific alterations of RNA editing in PDE8A mRNA in suicide decedents, which provided the first immune response-related brain marker for suicide[30]. PDE8A RNA editing is also increased in SHSY-5Y neuroblastoma cells treated with interferon alpha, as in HCV patients undergoing antiviral combination therapy with IFN-$\alpha$ and ribavirin. Importantly, measurement of RNA editing in PDE8A allowed discrimination between the group of patients who developed a treatment-emergent depression and those who did not, suggesting that A-I RNA editing biomarkers could be useful to monitor treatment-emergent depression[31].

Here, we focused on transcriptome-wide RNA editing modifications on Unipolar and BD patients to identify editing variants that could be used as biomarkers to set up a clinically useful diagnostic assay for differential diagnosis of BD. In order to prioritize a subset of variants clinically useful for a diagnostic assay for the differential diagnosis of bipolar disorder, each of the target genes described above was tested individually in two separate 'prioritization' cohorts (n=35, Figure 20 and n=58, Figure 21) by measuring editing rate in unipolar and BD patients by NGS. In the first cohort each target was sequenced individually, while in the second cohort editing rates of biomarkers were measured by multiplexing 4 BMKs. A multivariate analysis statistical method, evaluating for each combination of RNA edited sites and/or isoforms, was performed to find the best combination of biomarkers. To potentiate diagnostic performance, while minimizing biomarker number, the best combination was calculated with different numbers of biomarkers.

The list of primers used for the detection of RNA editing sites is indicated in Table 1.

**Table 1:** Primers used for the detection of RNA editing sites

| PRIMER NAME | Gene Name | PCR product Size (bp) | Primer Sequence (5'->3') | Primer length (bp) | SEQ ID NO. |
|---|---|---|---|---|---|
| IFNAR1 FORWARD_1 | IFNAR1 | 259 | ATACGGGCAAGCTCTTAACTATT | 23 | 1 |
| IFNAR1 REVERSE_1 | | | AACCTCCACCTCCTGTTTTC | 20 | 2 |
| IFNAR1_MiSeqF | IFNAR1 | 472 | AAAATACGGGCAAGCTCTTAACT | 23 | 3 |
| IFNAR1_MiSeqR | | | CTGCACTTAGGCTCACAGGA | 20 | 4 |
| | | | | | |
| PDE8A Forward | PDE8A | 225 | ATGCAAGTTGTGGACATGGAG | 21 | 5 |
| PDE8A Reverse | | | TTCTGAAAACAATGGGCACCA | 21 | 6 |
| CAMK1DPCR0_F | CAMK1D | 577 | TGTGCAAAGGGCTCCATAC | 19 | 7 |
| CAMK1DPCR0_R | | | CCTTGCAAGTGTCCAGTAAAC | 21 | 8 9 |
| CAMK1DMPx_F1 | | 195 | CTAGGGTGAGGTGGTGCAAT | 20 | |
| CAMK1DMPx_R1 | | | CAAGGCCAGGAGTTCAAGAC | 20 | 10 |
| | | | | | |
| LYN_F_PCR0 | LYN | 358 | TTTAAAAGCTTCTCCAGTCAGC | 22 | 11 |
| LYN_R_PCR0 | | | CAAGACTGAGCATACATTATATTCACC | 27 | 12 |
| LYN_MPx_F1 | | 193 | TTTAAAAGCTTCTCCAGTCAGC | 22 | 13 |
| LYN_MPx_R1 | | | TCCTGAGTAGCTGGGACCAT | 20 | 14 |
| | | | | | |
| PRKCB_MPx_F1 | PRKCB | 176 | CAGCACATTCTGTTGCCTTC | 20 | 15 |
| PRKCB_MPx_R2 | | | GTGACTTTGCCCCTCATTTG | 20 | 16 |
| | | | | | |
| KCNJ15_MPx_F1 | KCNJ15 | 160 | GGCCACTCTGTACTATGCAAATC | 23 | 17 |
| KCNJ15_MPx_R2 | | | TGAATTTCAGATGGACAGCAA | 21 | 18 |
| | | | | | |
| MDM2_F5 MPx-Rev | MDM2 | 302 | AAGCCCATCCTCGAACTACAG | 21 | 19 |
| MDM2_R5 MPx-Rev | | | TTGCTCTGTCACCTGGACTG | 20 | 20 |
| | | | | | |
| GAB2 MPx_F1 | GAB2 | 154 | GAACCAGGCTAGGTGCAATG | 20 | 21 |
| GAB2 MPx_R2 | | | CCATGCCCTGGTAATTTTTG | 20 | 22 |

(continued)

| PRIMER NAME | Gene Name | PCR product Size (bp) | Primer Sequence (5'->3') | Primer length (bp) | SEQ ID NO. |
|---|---|---|---|---|---|
| | | | | | |
| AHR-F1 | AHR | 409 | TCTGATGGAATCCCAAACTGAGA | 23 | 23 |
| AHR_F2 | | | TCAGAGATATTTTGTCCGAAGCCA | 24 | 24 |
| | | | | | |
| IFNAR2 MPx_F1_rev | IFNAR2 | 344 | CCCACAGTTTCAGAGGTGGT | 18 | 25 |
| IFNAR2 MPx_R1_rev | | | GGCTCACCCCTGTAATCC | 20 | 26 |
| | | | | | |
| IL17RA_Forward1 | IL17RA | 172 | GGGATGTAAACGCTGAATGG | 20 | 27 |
| IL17RA_Reverse1 | | | ATGTGCCACCATGACTGACT | 20 | 28 |
| | | | | | |
| RASSF1_F1 | | 480 | CCCTTCCGGCAAGAATCCAG | 20 | 29 |
| RASSF1_R1 | | | ACCCAAGCTGCATAAAAGGC | 20 | 30 |
| | | | | | |
| PIAS1_F1 | PIAS1 | 407 | GCAACCTTAGATCTCAATGGCTAA | 24 | 31 |
| PIAS1_R2 | | | AGCAGAGTCACAGTCTACAGC | 21 | 32 |
| | | | | | |
| PTPRC-MiSeqF | PTPTC | 505 | CCGGGGCTCATTTATAGGAT | 20 | 33 |
| PTPRC-MiSeqR | | | TGCTAGATACCGCCACATTG | 20 | 34 |
| PTPRC F rd2-1 | PTPRC | 215 | TAAGGACGGAGTTCGAGACC | 20 | 35 |
| PTPRC R rd2-1 | | | TGAGAGAGTTTTGCTCTGTGG | 21 | 36 |

As it was important to validate the results obtained in the RNA-Seq study, we first ran a differential analysis of each target on the prioritization cohorts. As shown in the tables below, all the sites and isoforms identified in the RNA-Seq study were found significant in a subsequent analysis on a larger population.

**Table 2A-2B**: Differential analysis of editing sites and isoforms identified in the RNA-Seq study in the patients of the first prioritization cohort analyzed in single-plex.

Shown are examples of RNA-Seq data of different targets with target name, differentially edited site or isoform, p-value and fold change of the edited site or isoform, AUC ROC, the mean value of editing at the considered site and population number.

**Table 2A**

| BMQnames | Chr:Pos (Hg38) | Site | p-Value | FoldChange | AUC ROC | % Editing | Populations |
|---|---|---|---|---|---|---|---|
| PTPRC | 1:198680333 | X227 | 0.0184 | 1.075001787 | 0.6924 | 18.68 | N= 35 [ 16 (0) ; 19 (1)] |
| PTPRC | 1:198680346 | X240 | 0.0314 | 1.071854346 | 0.7072 | 26.04 | N= 35 [16 (0) ; 19(1)] |
| PIAS1 | 15:68138001 | X201 | 0.0062 | 1.136535407 | 0.7500 | 0.65 | N= 35 [16 (0) ; 19 (1)] |
| IFNAR1 | 21:33355744 | X66 | 0.0054 | 1.263704007 | 0.7796 | 0.55 | N= 35 [ 16 (0) ; 19 (1)] |
| IFNAR1 | 21:33355754 | X76 | 0.0187 | 1.132685085 | 0.7204 | 4.80 | N= 35 [ 16 (0) ; 19 (1)] |
| IFNAR1 | 21:33355762 | X84 | 0.0058 | 1.153392283 | 0.7352 | 11.19 | N= 35 [ 16 (0) ; 19 (1)] |
| IFNAR1 | 21:33355763 | X85 | 0.0145 | 1.145854589 | 0.7368 | 6.65 | N= 35 [16 (0) ; 19(1)] |
| IFNAR1 | 21:33355789 | X111 | 0.0139 | 1.103258478 | 0.7303 | 19.89 | N= 35 [16 (0) ; 19(1)] |
| IFNAR1 | 21:33355793 | X115 | 0.0058 | 1.161013737 | 0.7566 | 11.51 | N= 35 [16 (0) ; 19(1)] |

(continued)

| BMQnames | Chr:Pos (Hg38) | Site | p-Value | FoldChange | AUC ROC | % Editing | Populations |
|---|---|---|---|---|---|---|---|
| IFNAR1 | 21:33355798 | X120 | 0.0116 | 1.180535533 | 0.7368 | 1.53 | N= 35 [16 (0) ; 19(1)] |
| IFNAR1 | 21:33355801 | X123 | 0.0013 | 1.224980021 | 0.7747 | 3.23 | N= 35 [16 (0) ; 19(1)] |
| IFNAR1 | 21:33355806 | X128 | 0.0032 | 1.210179599 | 0.7813 | 2.11 | N= 35 [16 (0) ; 19(1)] |
| IFNAR1 | 21:33355807 | X129 | 0.0101 | 1.104729968 | 0.7401 | 21.19 | N= 35 [16 (0) ; 19(1)] |
| IFNAR1 | 21:33355809 | X131 | 0.0052 | 1.232781797 | 0.7451 | 1.34 | N= 35 [16 (0) ; 19(1)] |
| IFNAR1 | 21:33355820 | X142 | 0.0108 | 1.152804642 | 0.7451 | 1.40 | N= 35 [16 (0) ; 19(1)] |
| IFNAR1 | 21:33355829 | X151 | 0.0069 | 1.181169484 | 0.7401 | 6.04 | N= 35 [16 (0) ; 19(1)] |
| IFNAR1 | 21:33355830 | X152 | 0.0173 | 1.109572564 | 0.7138 | 22.08 | N= 35 [16 (0) ; 19(1)] |
| IFNAR1 | 21:33355838 | X160 | 0.0067 | 1.149938391 | 0.7467 | 8.08 | N= 35 [16 (0) ; 19(1)] |
| IFNAR1 | 21:33355839 | X161 | 0.0208 | 1.135470598 | 0.7188 | 4.12 | N= 35 [ 16 (0) ; 19 (1)] |
| IFNAR1 | 21:33355840 | X162 | 0.0113 | 1.1268041 | 0.7451 | 10.56 | N= 35 [16 (0) ; 19 (1)] |
| IFNAR1 | 21:33355860 | X182 | 0.009 | 1.180765679 | 0.7500 | 4.58 | N= 35 [16 (0) ; 19 (1)] |
| IFNAR1 | 21:33355863 | X185 | 0.0031 | 1.21778062 | 0.7862 | 2.19 | N= 35 [16 (0) ; 19 (1)] |
| IFNAR1 | 21:33355865 | X187 | 0.0014 | 1.196153448 | 0.8240 | 2.11 | N= 35 [16 (0) ; 19 (1)] |
| IFNAR1 | 21:33355869 | X191 | 0.0036 | 1.192181699 | 0.7730 | 7.98 | N= 35 [16 (0) ; 19 (1)] |
| IFNAR1 | 21:33355905 | X227 | 0.0058 | 1.14365726 | 0.7845 | 5.72 | N= 35 [16 (0) ; 19 (1)] |
| IFNAR1 | 21:33355909 | X231 | 0.0055 | 1.150637009 | 0.7632 | 10.90 | N= 35 [16 (0) ; 19 (1)] |
| IL17RA | 22:17110596 | X100 | 0.0356 | 1.129120379 | 0.6924 | 0.39 | N= 35 [16 (0) ; 19 (1)] |
| IL17RA | 22:17110628 | X132 | 0.0438 | 1.154264973 | 0.6793 | 0.24 | N= 35 [16 (0) ; 19 (1)] |
| RASSF1 | 3:50333653 | X192 | 0.0112 | 1.130138254 | 0.7401 | 1.33 | N= 35 [16 (0) ; 19 (1)] |
| RASSF1 | 3:50333747 | X98 | 0.0354 | 1.084940073 | 0.7385 | 2.11 | N= 35 [16 (0) ; 19 (1)] |
| LYN | 8:56012452 | X47 | 0.0427 | 1.131762974 | 0.7039 | 1.53 | N= 35 [16 (0) ; 19 (1)] |
| LYN | 8:56012468 | X63 | 0.0165 | 1.106424238 | 0.7368 | 8.54 | N= 35 [16 (0) ; 19 (1)] |
| LYN | 8:56012469 | X64 | 0.0316 | 1.113571193 | 0.7237 | 4.98 | N= 35 [16 (0) ; 19 (1)] |
| LYN | 8:56012521 | X116 | 0.0433 | 1.173270254 | 0.7023 | 1.03 | N= 35 [16 (0) ; 19 (1)] |
| LYN | 8:56012553 | X148 | 0.0207 | 1.079245173 | 0.7303 | 18.89 | N= 35 [16 (0) ; 19 (1)] |
| LYN | 8:56012556 | X151 | 0.0122 | 1.105001887 | 0.7467 | 3.59 | N= 35 [ 16 (0) ; 19 (1)] |
| LYN | 8:56012558 | X153 | 0.0101 | 1.131044953 | 0.7516 | 3.99 | N= 35 [16 (0) ; 19 (1)] |
| LYN | 8:56012567 | X162 | 0.0266 | 1.116046561 | 0.7138 | 1.96 | N= 35 [16 (0) ; 19 (1)] |
| LYN | 8:56012568 | X163 | 0.0215 | 1.121045291 | 0.7138 | 1.91 | N= 35 [ 16 (0) ; 19 (1)] |
| LYN | 8:56012572 | X167 | 0.0493 | 1.098662612 | 0.6760 | 5.01 | N= 35 [ 16 (0) ; 19 (1)] |
| LYN | 8:56012596 | X191 | 0.014 | 1.066625025 | 0.7434 | 31.43 | N= 35 [ 16 (0) ; 19 (1)] |
| LYN | 8:56012604 | X199 | 0.0398 | 1.105936897 | 0.7056 | 3.05 | N= 35 [ 16 (0) ; 19 (1)] |
| LYN | 8:56012608 | X203 | 0.0142 | 1.130472736 | 0.7500 | 4.68 | N= 35 [ 16 (0) ; 19 (1)] |
| LYN | 8:56012612 | X207 | 0.0269 | 1.112587406 | 0.7039 | 8.00 | N= 35 [16 (0) ; 19 (1)] |
| LYN | 8:56012660 | X255 | 0.0206 | 1.09554913 | 0.7253 | 11.18 | N= 35 [16 (0) ; 19 (1)] |
| LYN | 8:56012668 | X263 | 0.0282 | 1.077150344 | 0.6924 | 12.11 | N= 35 [ 16 (0) ; 19 (1)] |

(continued)

| BMQnames | Chr:Pos (Hg38) | Site | p-Value | FoldChange | AUC ROC | % Editing | Populations |
|----------|----------------|------|---------|------------|---------|-----------|-------------|
| LYN | 8:56012689 | X284 | 0.0033 | 1.162082677 | 0.7648 | 4.56 | N= 35 [ 16 (0) ; 19 (1)] |
| LYN | 8:56012699 | X294 | 0.0097 | 1.228499121 | 0.7352 | 1.72 | N= 35 [16 (0) ; 19(1)] |

**Table 2B**

| Target | Isoforms | p-value | FoldChange | AUC ROC | % Editing | Populations |
|--------|----------|---------|------------|---------|-----------|-------------|
| AHR | JKMNTV | 0.0270 | 1.77 | 0.719 | 0.33 | N= 35 [16 (0); 19(1)] |
| IFNAR1 | ABCDE | 0.0115 | 1.16 | 0.740 | 1.16 | N= 35 [16 (0); 19(1)] |
| IFNAR1 | ABCDEFGH | 0.0026 | 1.29 | 0.776 | 1.08 | N= 35 [16 (0); 19(1)] |
| IFNAR1 | ABCDEFH | 0.0116 | 1.21 | 0.727 | 1.40 | N= 35 [16 (0); 19(1)] |
| IFNAR1 | ABCDEGH | 0.0007 | 1.34 | 0.816 | 0.52 | N= 35 [ 16 (0); 19(1)] |
| IFNAR1 | ABCDEH | 0.0062 | 1.25 | 0.737 | 0.66 | N= 35 [16 (0); 19(1)] |
| IFNAR1 | BCDEFGH | 0.0010 | 1.30 | 0.798 | 0.29 | N= 35 [16 (0); 19(1)] |
| IFNAR1 | BCDEH | 0.0040 | 1.21 | 0.760 | 0.27 | N= 35 [16 (0); 19(1)] |
| IFNAR1 | GH | 0.0353 | 1.11 | 0.707 | 0.79 | N= 35 [16 (0); 19(1)] |
| IFNAR1 | NoEdit | 0.0236 | 0.97 | 0.727 | 67.71 | N= 35 [16 (0); 19(1)] |
| LYN | ABC | 0.0267 | 1.12 | 0.717 | 0.46 | N= 35 [16 (0) ; 19(1)] |
| LYN | ABCD | 0.0252 | 1.19 | 0.738 | 0.22 | N= 35 [16 (0) ; 19(1)] |
| LYN | ABCDMNOP | 0.0224 | 1.22 | 0.702 | 0.44 | N= 35 [16 (0) ; 19(1)] |
| LYN | ACD | 0.0400 | 1.10 | 0.714 | 0.46 | N= 35 [16 (0) ; 19(1)] |
| LYN | ACDMNOP | 0.0105 | 1.22 | 0.743 | 0.44 | N= 35 [16 (0) ; 19(1)] |
| LYN | CDMNOP | 0.0159 | 1.18 | 0.743 | 0.53 | N= 35 [16 (0) ; 19(1)] |
| LYN | D | 0.0341 | 1.04 | 0.740 | 10.52 | N= 35 [16 (0) ; 19(1)] |
| LYN | DMNOP | 0.0148 | 1.18 | 0.742 | 0.47 | N= 35 [16 (0) ; 19(1)] |
| LYN | NoEdit | 0.0162 | 0.96 | 0.724 | 58.17 | N= 35 [16 (0) ; 19(1)] |
| MDM2 | GTAX | 0.0014 | 1.20 | 0.849 | 0.22 | N= 35 [16 (0) ; 19(1)] |
| MDM2 | GTVA | 0.0242 | 0.88 | 0.702 | 0.25 | N= 35 [16 (0) ; 19(1)] |
| PTPRC | GAHILM | 0.0458 | 1.25 | 0.717 | 0.58 | N= 35 [16 (0) ; 19(1)] |
| PTPRC | HI | 0.0228 | 1.11 | 0.727 | 1.67 | N= 35 [16 (0) ; 19(1)] |
| PTPRC | HM | 0.0150 | 1.37 | 0.794 | 0.20 | N= 35 [16 (0) ; 19(1)] |
| PTPRC | LM | 0.0422 | 0.86 | 0.702 | 0.56 | N= 35 [16 (0) ; 19(1)] |
| RASSF1 | EJ | 0.0362 | 0.94 | 0.714 | 0.70 | | N= 35 [ 16 (0) ; 19 (1)] |

**Table** 3: Differential analysis of editing sites and isoforms identified in the RNA-Seq study in the patients of the second prioritization cohort analyzed with multiplex sequencing.

Shown are examples of RNA-Seq data of different targets with target name, differentially edited site, p-value and fold change of the edited site, AUC ROC, the mean value of editing at the considered site and population number.

| Num | Target | Chr:Pos (hg38) | Sites | p-Value | Fold-Change | AUC ROC | % Editing | Population | Sex |
|---|---|---|---|---|---|---|---|---|---|
| 1 | PTPRC | 1: 198680346 | X240 | 0.0410 | 1.04 | 0.656 | 22.2 | N= 58 [ 29 (0) ; 29 (1)] | M+ F |
| 2 | CAMK1D | 10: 12378356 | X64 | 0.0024 | 1.11 | 0.804 | 7.7 | N=31 [15 (0) ; 16 (1)] | F only |
| 3 | CAMK1D | 10: 12378376 | X84 | 0.0120 | 1.09 | 0.788 | 22.7 | N=31 [15 (0) ; 16 (1)] | F only |
| 4 | CAMK1D | 10: 12378393 | X101 | 0.0294 | 1.08 | 0.735 | 7.4 | N= 31 [ 15 (0) ; 16 (1)] | F only |
| 5 | CAMK1D | 10: 12378415 | X123 | 0.0064 | 1.10 | 0.794 | 25.6 | N=31 [15 (0) ; 16 (1)] | F only |
| 6 | CAMK1D | 10: 12378423 | X131 | 0.0190 | 0.82 | 0.745 | 1.2 | N= 27 [ 14 (0) ; 13 (1)] | M only |
| 7 | GAB2 | 11: 78330899 | X141 | 0.0165 | 1.04 | 0.754 | 35.4 | N=31 [15 (0) ; 16 (1)] | F only |
| 8 | MDM2 | 12: 68821522 | X143 | 0.0150 | 0.71 | 0.760 | 0.7 | N= 31 [ 15 (0) ; 16 (1)] | F only |
| 9 | MDM2 | 12: 68821573 | X194 | 0.0166 | 1.06 | 0.752 | 5.7 | N=31 [15 (0) ; 16 (1)] | F only |
| 10 | PDE8A | 15: 85212906 | X116278 | 0.0496 | 0.77 | 0.723 | 0.5 | N= 27 [ 14 (0) ; 13(1)] | M only |
| 11 | PDE8A | 15: 85212909 | X116281 | 0.0482 | 1.21 | 0.660 | 0.6 | N=31 [15 (0) ; 16 (1)] | F only |
| 12 | IFNAR1 | 21: 33355744 | X66 | 0.0413 | 1.19 | 0.656 | 0.5 | N= 58 [ 29 (0) ; 29 (1)] | M+ F |
| 13 | IFNAR1 | 21: 33355762 | X84 | 0.0464 | 1.15 | 0.700 | 10.1 | N=31 [ 15 (0) ; 16 (1)] | F only |
| 14 | IFNAR1 | 21: 33355763 | X85 | 0.0386 | 1.11 | 0.667 | 5.9 | N= 58 [ 29 (0) ; 29 (1)] | M+ F |
| 15 | IFNAR1 | 21: 33355789 | X111 | 0.0414 | 1.06 | 0.654 | 17.3 | N= 58 [ 29 (0) ; 29 (1)] | M+ F |
| 16 | IFNAR1 | 21: 33355807 | X129 | 0.0473 | 1.10 | 0.696 | 18.9 | N= 31 [ 15 (0) ; 16 (1)] | F only |
| 17 | IFNAR1 | 21: 33355829 | X151 | 0.0247 | 1.18 | 0.744 | 5.5 | N=31 [15 (0) ; 16 (1)] | F only |
| 18 | IFNAR1 | 21: 33355830 | X152 | 0.0481 | 1.06 | 0.651 | 19.8 | N= 58 [ 29 (0) ; 29 (1)] | M+ F |

Combinations of biomarkers were then analyzed to identify the best diagnostic performance for differential diagnosis of Bipolar Disorder, with different numbers of biomarkers. In the first cohort, when only 2 sites from 2 different targets were used, we obtained an AUC of 0.812 (Sp 81.3%; Se 79.0%, Figure 4A). Another combination of 3 sites, using 2 BMKs, gave even better performances, with an AUC of 0.872 (Sp 87.5%; Se 79.0%, Figure 4B). The equation used for this combination is the following:

$$Z = + (2,03684329038398)* IFNAR1\_X115\_G + ( 12,2505281183792 )* LYN\ X151\_F + ( -0,765269706061865 )* IFNAR1\_X111\_A$$

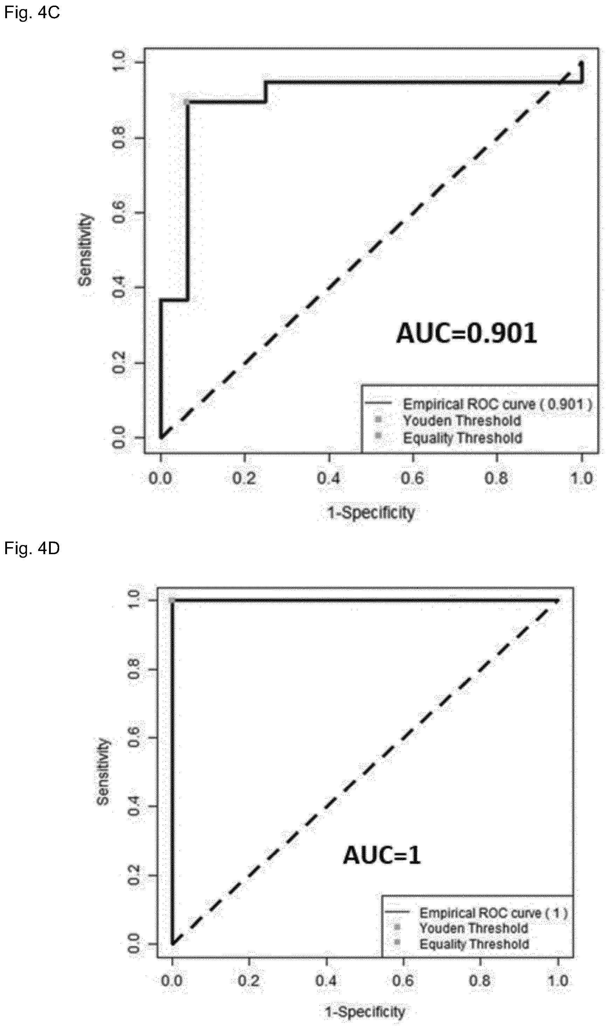

When the number of differentially edited sites was increased to 4, with 3 different targets, the AUC was 0.901 (Sp 93.8%; Se 89.5%, Figure 4C). Moreover, when up to 24 differentially edited sites, all contained in only three different biomarkers, were combined and analyzed, we obtained an outstanding performance, with an AUC of 1.00, with excellent specificity and sensitivity (100% and 100%, respectively, Figure 4D). The equation used for this combination is the following:

Z=: + ( 18131,4993489419 )* LYN X191_B + (-1,0712864185386 )* PTPRC_X240_I + ( -4,64009201823763e-05 )* IFNAR1_X152_C + ( 0,996774248934566 )* IFNAR1_X129_B + ( 0,0381530674132845 )* PTPRC_X227_H + ( 1885,81023403163 )* LYN_X148_A + ( -0,736314805340674 )* IFNAR1_X111_A + ( 4390,83243262018 )* LYN_X263_O + ( 6,2909637469539 )* IFNAR1_X115_G + ( 13,9766314855872 )* IFNAR1_X84_F + ( 15,0973299759625 )* IFNAR1_X231_D + ( -32,585224895614 )* IFNAR1_X162_E + ( -310,251286436275 )* LYN_X207_M + ( - 16,457803229942 )* LYN_X63_C + ( -2,01190201250814 )* IFNAR1_X191_nan + ( 3,99543996199355 )* IFNAR1_X160_nan + ( -5,82765669149742 )* IFNAR1_X85_nan + ( 0,883565177670863 )* IFNAR1_X151_nan + ( 0,655717782231297 )* IFNAR1 X227_H + ( -16,1663006759008 )* LYN_X167_I + (-72,6085985517623 )* LYN X64_D + ( 26,1324832381722 )* LYN_X284_P + ( - 19,0669113948407 )* IFNAR1_X76_nan + ( -33,3105655669062 )* IFNAR1_X182_nan

The list of all combination tested in this cohort, all of which included the 35 patients of the cohort, is presented in Table 4. All combinations were statistically significant between control and cases with a p value <0.01.

**Table 4:** Diagnostic performances obtained with patients of the first prioritization cohort analyzed in single-plex.

| Num | Combinaison mROC | Combi | ID | AUC ROC | CI95% | Threshold | Acc (%) | Sp(%) | Se(%) | VPP (%) | VPN (%) | Population |
|---|---|---|---|---|---|---|---|---|---|---|---|---|
| 1 | PIAS1_X201_L + LYN_X191_B | C2 | 156 | 0.826 | [0,686 ; 0,965] | 2096.25 | 80.0 | 68.8 | 89.5 | 77.3 | 84.6 | N= 35 [16 (1); 19 (2)] |
| 2 | IFNAR1_X227_H + PIAS1_X201_L | C2 | 6 | 0.816 | [0,664 ; 0,968] | 0.00 | 82.9 | 68.8 | 94.7 | 78.3 | 91.7 | N= 35 [16 (1); 19 (2)] |
| 3 | IFNAR1_X227_H + LYN_X284_P | C2 | 1 | 0.812 | [0,669 ; 0,956] | 5.08 | 80.0 | 81.3 | 79.0 | 83.3 | 76.5 | N= 35 [16 (1); 19 (2)] |
| 4 | IFNAR1_X231_D + IFNAR1_X111_A | C2 | 66 | 0.809 | [0,662 ; 0,957] | 4.11 | 80.0 | 81.3 | 79.0 | 83.3 | 76.5 | N= 35 [16 (1); 19 (2)] |
| 5 | PIAS1_X201_L + LYN_X47_Q | C2 | 168 | 0.806 | [0,658 ; 0,954] | -0.82 | 80.0 | 75.0 | 84.2 | 80.0 | 80.0 | N= 35 [16 (1); 19 (2)] |
| 6 | IFNAR1_X231_D + IFNAR1_X152_C | C2 | 70 | 0.803 | [0,65 ; 0,956] | 6.34 | 80.0 | 93.8 | 68.4 | 92.9 | 71.4 | N= 35 [16 (1); 19 (2)] |
| 7 | IFNAR1_X227_H + IFNAR1_X111_A | C2 | 13 | 0.786 | [0,626 ; 0,946] | 1.01 | 80.0 | 87.5 | 73.7 | 87.5 | 73.7 | N= 35 [16 (1); 19 (2)] |
| 8 | LYN_X191_B + PTPRC_X240_I | C2 | 227 | 0.766 | [0,597 ; 0,936] | 1678.04 | 80.0 | 68.8 | 89.5 | 77.3 | 84.6 | N= 35 [16 (1); 19 (2)] |
| 9 | IFNAR1_X129_B + IFNAR1_X111_A | C2 | 238 | 0.757 | [0,579 ; 0,934] | 5.64 | 82.9 | 68.8 | 94.7 | 78.3 | 91.7 | N= 35 [16 (1); 19 (2)] |
| 10 | IFNAR1_X115_G + LYN_X151_F + IFNAR1_X111_A | C3 | 1129 | 0.872 | [0,756 ; 0,987] | 9.62 | 82.9 | 87.5 | 79.0 | 88.2 | 77.8 | N= 35 [16 (1); 19 (2)] |
| 11 | PIAS1_X201_L + LYN_X191_B + LYN_X199_K | C3 | 1943 | 0.852 | [0,719; 0,985] | 4285.00 | 82.9 | 75.0 | 89.5 | 81.0 | 85.7 | N= 35 [16 (1); 19 (2)] |
| 12 | IFNAR1_X227_H + LYN_X284_P + LYN_X207_M | C3 | 21 | 0.849 | [0,71 ; 0,988] | -36.88 | 85.7 | 93.8 | 79.0 | 93.8 | 79.0 | N= 35 [16 (1); 19 (2)] |
| 13 | PIAS1_X201_L + LYN_X148_A + LYN_X199_K | C3 | 2023 | 0.845 | [0,704 ; 0,987] | 1373.92 | 85.7 | 81.3 | 89.5 | 85.0 | 86.7 | N= 35 [16 (1); 19 (2)] |
| 14 | IFNAR1_X115_G + PIAS1_X201_L + IFNAR1_X111_A | C3 | 1108 | 0.842 | [0,701 ; 0,983] | -0.97 | 85.7 | 81.3 | 89.5 | 85.0 | 86.7 | N= 35 [16 (1); 19 (2)] |

| Num | Combinaison mROC | Combi | ID | AUC ROC | C195% | Threshold | Acc (%) | Sp(%) | Se(%) | VPP (%) | VPN (%) | Population |
|---|---|---|---|---|---|---|---|---|---|---|---|---|
| 15 | LYN_X284_P + PIAS1_X201_L + LYN_X207_M | C3 | 492 | 0.836 | [ 0,699 ; 0,972 ] | -21.34 | 82.9 | 87.5 | 79.0 | 88.2 | 77.8 | N= 35 [ 16 (1); 19 (2)] |
| 16 | LYN_X153_G + IFNAR1_X129_B + IFNAR1_X111_A | C3 | 1462 | 0.829 | [ 0,673 ; 0,985 ] | 8.69 | 85.7 | 81.3 | 89.5 | 85.0 | 86.7 | N= 35 [ 16 (1); 19 (2)] |
| 17 | IFNAR1_X115_G + IFNAR1_X111_A + PTPRC_X240_I | C3 | 1241 | 0.822 | [ 0,672 ; 0,972 ] | 3.43 | 82.9 | 81.3 | 84.2 | 84.2 | 81.3 | N= 35 [ 16 (1); 19 (2)] |
| 18 | IFNAR1_X115_G + IFNAR1_X111_A + PTPRC_X240_I + LYN_X263_O | C4 | 10614 | 0.901 | [ 0,778 ; 1] | 316.50 | 91.4 | 93.8 | 89.5 | 94.4 | 88.2 | N= 35 [ 16 (1); 19 (2)] |
| 19 | IFNAR1_X231_D + PIAS1_X201_L + LYN_X151_F + LYN_X162 _Y | C4 | 7041 | 0.898 | [ 0,784 ; 1] | 9.73 | 88.6 | 93.8 | 84.2 | 94.1 | 83.3 | N= 35 [ 16 (1); 19 (2)] |
| 20 | IFNAR1_X115_G + PIAS1_X201_L + LYN_X191_B + IFNAR1_X111_A | C4 | 9375 | 0.895 | [ 0,789 ; 1] | 3657.05 | 85.7 | 87.5 | 84.2 | 88.9 | 82.4 | N= 35 [ 16 (1); 19 (2)] |
| 21 | IFNAR1_X115_G + PIAS1_X201_L + IFNAR1_X111_A + LYN_X148_A | C4 | 9442 | 0.891 | [ 0,778 ; 1] | 1071.10 | 85.7 | 87.5 | 84.2 | 88.9 | 82.4 | N= 35 [ 16 (1); 19 (2)] |
| 22 | IFNAR1_X115_G + IFNAR1_X111_A + LYN_X148_A + PTPRC_X227_H | C4 | 10552 | 0.891 | [ 0,779 ; 1] | 1104.69 | 85.7 | 87.5 | 84.2 | 88.9 | 82.4 | N= 35 [ 16 (1); 19 (2)] |
| 23 | IFNAR1_X231_D + PIAS1_X201_L + LYN_X148_A + LYN_X199_K | C4 | 7170 | 0.888 | [ 0,767 ; 1] | 1465.90 | 88.6 | 87.5 | 89.5 | 89.5 | 87.5 | N= 35 [ 16 (1); 19 (2)] |
| 24 | IFNAR1_X115_G + LYN_X153_G + PIAS1_X201_L + IFNAR1_X111_A | C4 | 8831 | 0.882 | [ 0,757 ; 1] | 1.36 | 85.7 | 93.8 | 79.0 | 93.8 | 79.0 | N= 35 [ 16 (1); 19 (2)] |
| 25 | IFNAR1_X227_H + LYN_X284_P + LYN_X151_F + LYN_X207_M | C4 | 135 | 0.878 | [ 0,752 ; 1] | -52.29 | 88.6 | 87.5 | 89.5 | 89.5 | 87.5 | N= 35 [ 16 (1); 19 (2)] |
| 26 | IFNAR1_X227_H + IFNAR1_X115_G + PIAS1_X201_L + IFNAR1_X111_A | C4 | 730 | 0.862 | [ 0,723 ; 1] | -2.98 | 88.6 | 87.5 | 89.5 | 89.5 | 87.5 | N= 35 [ 16 (1); 19 (2)] |
| 27 | IFNAR1_X227_H + IFNAR1_X115_G + IFNAR1_X111_A + PTPRC_X240_I | C4 | 863 | 0.839 | [ 0,686 ; 0,991 ] | 1.79 | 88.6 | 87.5 | 89.5 | 89.5 | 87.5 | N= 35 [ 16 (1); 19 (2)] |

(continued)

| Num | Combinaison mROC | Combi | ID | AUC ROC | C195% | Threshold | Acc (%) | Sp(%) | Se(%) | VPP (%) | VPN (%) | Population |
|---|---|---|---|---|---|---|---|---|---|---|---|---|
| 28 | LYN_X191_B + PTPRC_X240_I + IFNAR1_X152_C + IFNAR1_X129_B + PTPRC_X227_H + LYN_X148_A + IFNAR1_X111_A + LYN_X263_O + IFNAR1_X115_G + IFNAR1_X84_F + IFNAR1_X231_D + IFNAR1_X162_E + LYN_X207_M + LYN_X63_C + IFNAR1_X191 + IFNAR1_X160 + IFNAR1_X85 + IFNAR1_X151 + IFNAR1_X227_H + LYN_X167_I + LYN_X64_D + LYN_X284_P + IFNAR1_X76 + IFNAR1_X182 | C24 | 15 | 1.000 | [ 1 ; 1 ] | 12793.7 | 100.0 | 100.0 | 100.0 | 100.0 | 100.0 | N= 35 [ 16 (1); 19 (2)] |

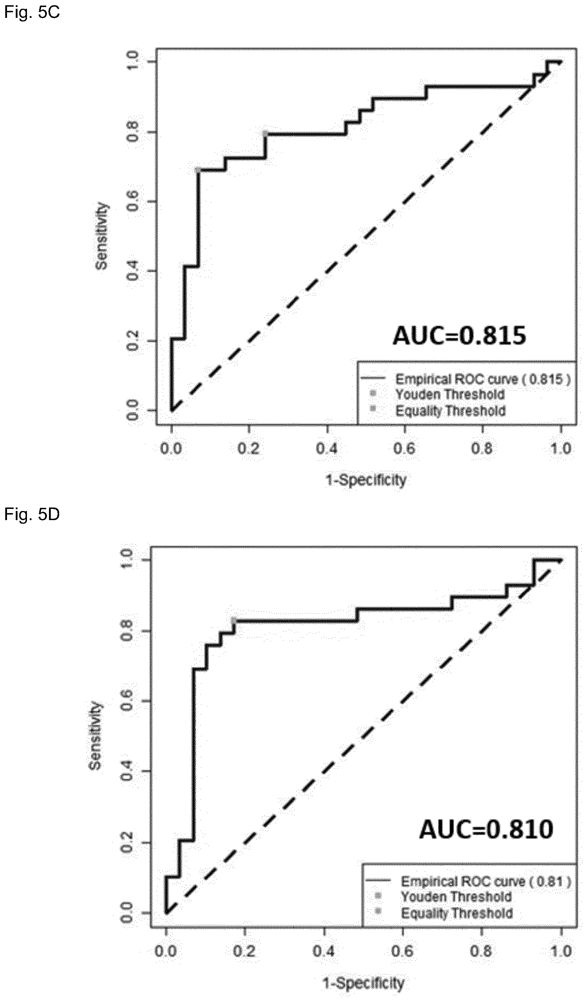

In the second prioritization cohort, using multiplexed sequencing, when only 3 sites in 2 different BMK were chosen, the AUC was 0.759 (Sp 89.7%; Se 69.0%, Figure 5A). When 4 sites in 2 BMKs were combined, the AUC increased to 0.772 (Sp 82.8%; Se 75.9%, Figure 5B). With combinations of 5 and 6 sites, the AUC rose to 0.815 and 0.810, with specificities of 81.0% and 82.8% and sensitivities of 93.1% and 82.8%, respectively (Figure 5C and D). When the combination was increased to include 14 sites, the AUC was above 0.85 (AUC 0.860, Sp 89.7%, Se 82.8%, Figure 5E). For this combination, the equation used is the following:

Z= + ( 0.510073028938919 )* IFNAR1_Site85 + ( 0.000530246141972222 )* PTPRC_Site240 + ( 1.61121867548568 )* IFNAR1_Site66 + ( 0.000171269688832027 )* IFNAR1_Site152_C + ( -0.000399273936550938 )* PRKCB_Site152_L - ( 1.99301166582376 )* GAB2_Site159 + ( 0.496425680926631 )* IFNAR1_Site151 + (-0.343700289838798)* PRKCB_Site99 + ( 2.72505005033054 )* PRKCB_Site94 + ( -0.0231770987287983 )* IFNAR1_Site129_B + ( 0.00239888123061141 )* PTPRC_Site227_H + ( -0.471278382211274 )* IFNAR1_Site78 + ( - 0.0500319554438776 )* PTPRC_Site226_A + ( - 0.852148596678129 )* IFNAR1_Site101

The list of all combination tested in the second prioritization cohort is presented in Table 5. All combinations were statistically significant between control and cases with a p value <0.01.

**Table 5:** Diagnostic performances obtained with patients of the second prioritization cohort analyzed with multiplex sequencing

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 1 | IFNAR1_Site85 + IFNAR1_Site129_B | C2 | 10 | 0.703 | [0.559 ; 0.847 ] | 0.3872 | 74.1 | 79.3 | 69.0 | 76.9 | 71.9 | N= 58 [ 29 (1); 29 (2)] |
| 2 | IFNAR1_Site85 + GAB2_Site159 | C2 | 6 | 0.719 | [0.581 ; 0.858 ] | 1.3141 | 72.4 | 72.4 | 72.4 | 72.4 | 72.4 | N= 58 [ 29 (1); 29 (2)] |
| 3 | GAB2_Site159 + PTPRC_Site227_H | C2 | 80 | 0.709 | [ 0.57 ; 0.848 ] | 1.5524 | 72.4 | 79.3 | 65.5 | 76.0 | 69.7 | N= 58 [ 29 (1); 29 (2)] |
| 4 | PTPRC_Site240 + GAB2_Site159 | C2 | 20 | 0.722 | [0.585 ; 0.859 ] | 1.4809 | 70.7 | 75.9 | 65.5 | 73.1 | 68.8 | N= 58 [ 29 (1); 29 (2)] |
| 5 | IFNAR1_Site85 + PRKCB_Site94 | C2 | 9 | 0.698 | [0.557 ; 0.839 ] | 0.9817 | 70.7 | 72.4 | 69.0 | 71.4 | 70.0 | N= 58 [ 29 (1); 29 (2)] |
| 6 | IFNAR1_Site85 + PRKCB_Site94 + IFNAR1_Site84 F | C3 | 90 | 0.706 | [0.559 ; 0.854 ] | 0.3811 | 79.3 | 89.7 | 69.0 | 87.0 | 74.3 | N= 58 [ 29 (1); 29 (2)] |
| 7 | IFNAR1_Site85 + GAB2_Site159 + IFNAR1_Site129_B | C3 | 64 | 0.759 | [0.627 ; 0.89 ] | 1.141 | 79.3 | 89.7 | 69.0 | 87.0 | 74.3 | N= 58 [ 29 (1); 29 (2)] |
| 8 | IFNAR1_Site85 + IFNAR1_Site111_A + GAB2_Site159 | C3 | 30 | 0.741 | [0.603 ; 0.879 ] | 1.1973 | 77.6 | 86.2 | 69.0 | 83.3 | 73.5 | N= 58 [ 29 (1); 29 (2)] |
| 9 | IFNAR1_Site85 + PTPRC_Site240 + IFNAR1_Site129_B | C3 | 9 | 0.744 | [0.608 ; 0.881 ] | 0.9526 | 75.9 | 75.9 | 75.9 | 75.9 | 75.9 | N= 58 [ 29 (1); 29 (2)] |
| 10 | IFNAR1_Site85 + GAB2_Site159 + IFNAR1_Site151 + IFNAR1_Site129_B | C4 | 338 | 0.780 | [0.652 ; 0.908 ] | 1.2687 | 81.0 | 93.1 | 69.0 | 90.9 | 75.0 | N= 58 [ 29 (1); 29 (2)] |
| 11 | IFNAR1_Site85 + IFNAR1_Site66 + GAB2_Site159 + IFNAR1_Site129_B | C4 | 128 | 0.772 | [0.642 ; 0.901 ] | -0.184 | 79.3 | 82.8 | 75.9 | 81.5 | 77.4 | N= 58 [ 29 (1); 29 (2)] |
| 12 | IFNAR1_Site85 + GAB2_Site159 + PRKCB_Site94 + IFNAR1_Site129_B | C4 | 351 | 0.772 | [0.643 ; 0.9 ] | 1.2987 | 79.3 | 89.7 | 69.0 | 87.0 | 74.3 | N= 58 [ 29 (1); 29 (2)] |
| 13 | IFNAR1_Site85 + IFNAR1_Site111_A + GAB2_Site159 + PTPRC_Site226_A | C4 | 197 | 0.746 | [0.607 ; 0.884 ] | 1.4803 | 79.3 | 96.6 | 62.1 | 94.7 | 71.8 | N= 58 [ 29 (1); 29 (2)] |
| 14 | IFNAR1_Site85 + GAB2_Site159 + IFNAR1_Site129_B + IFNAR1_Site78 | C4 | 358 | 0.760 | [0.629 ; 0.891 ] | 1.1449 | 79.3 | 89.7 | 69.0 | 87.0 | 74.3 | N= 58 [ 29 (1); 29 (2)] |

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 15 | IFNAR1_Site85 + GAB2_Site159 + IFNAR1_Site129_B + IFNAR1_Site101 | C4 | 360 | 0.759 | [0.627 ; 0.89 ] | 1.1525 | 79.3 | 89.7 | 69.0 | 87.0 | 74.3 | N= 58 [ 29 (1); 29 (2)] |
| 16 | IFNAR1_Site85 + GAB2_Site159 + IFNAR1_Site129_B + PTPRC_Site227_H | C4 | 357 | 0.762 | [0.631 ; 0.893 ] | 1.3882 | 77.6 | 82.8 | 72.4 | 80.8 | 75.0 | N= 58 [ 29 (1); 29 (2)] |
| 17 | IFNAR1_Site85 + IFNAR1_Site111_A + GAB2_Site159 + IFNAR1_Site129_B | C4 | 194 | 0.760 | [ 0.63 ; 0.89 ] | 1.0227 | 77.6 | 79.3 | 75.9 | 78.6 | 76.7 | N= 58 [ 29 (1); 29 (2)] |
| 18 | IFNAR1_Site85 + GAB2_Site159 + IFNAR1_Site151 + IFNAR1_Site129_B + IFNAR1_Site78 | C5 | 1170 | 0.788 | [0.665 ; 0.912 ] | 1.2865 | 81.0 | 93.1 | 69.0 | 90.9 | 75.0 | N= 58 [ 29 (1); 29 (2)] |
| 19 | IFNAR1_Site85 + IFNAR1_Site111_A + GAB2_Site159 + IFNAR1_Site151 + IFNAR1_Site129_B | C5 | 753 | 0.779 | [0.651 ; 0.907 ] | 1.3754 | 81.0 | 93.1 | 69.0 | 90.9 | 75.0 | N= 58 [ 29 (1); 29 (2)] |
| 20 | IFNAR1_Site85 + GAB2_Site159 + IFNAR1_Site151 + IFNAR1_Site129_B + PTPRC_Site226_A | C5 | 1171 | 0.779 | [0.651 ; 0.907 ] | 1.422 | 81.0 | 96.6 | 65.5 | 95.0 | 73.7 | N= 58 [ 29 (1); 29 (2)] |
| 21 | IFNAR1_Site85 + PTPRC_Site240 + IFNAR1_Site66 + GAB2_Site159 + IFNAR1_Site129_B | C5 | 37 | 0.815 | [0.697 ; 0.932 ] | 0.5012 | 81.0 | 93.1 | 69.0 | 90.9 | 75.0 | N= 58 [ 29 (1); 29 (2)] |
| 22 | IFNAR1_Site85 + GAB2_Site159 + IFNAR1_Site151 + IFNAR1_Site129_B + PTPRC_Site227_H | C5 | 1169 | 0.791 | [0.665 ; 0.917 ] | 1.7748 | 81.0 | 93.1 | 69.0 | 90.9 | 75.0 | N= 58 [ 29 (1); 29 (2)] |
| 23 | IFNAR1_Site85 + IFNAR1_Site66 + GAB2_Site159 + IFNAR1_Site129_B + PTPRC_Site227_H | C5 | 552 | 0.787 | [0.662 ; 0.912 ] | 0.1562 | 79.3 | 82.8 | 75.9 | 81.5 | 77.4 | N= 58 [ 29 (1); 29 (2)] |
| 24 | IFNAR1_Site85 + IFNAR1_Site66 + GAB2_Site159 + IFNAR1_Site129_B + PTPRC_Site226_A | C5 | 554 | 0.772 | [0.642 ; 0.901 ] | -0.1973 | 79.3 | 82.8 | 75.9 | 81.5 | 77.4 | N= 58 [ 29 (1); 29 (2)] |
| 25 | IFNAR1_Site85 + PTPRC_Site240 + GAB2_Site159 + IFNAR1_Site151 + IFNAR1_Site129_B | C5 | 247 | 0.810 | [0.689 ; 0.931 ] | 1.7106 | 79.3 | 75.9 | 75.9 | 75.9 | 75.9 | N= 58 [ 29 (1); 29 (2)] |

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 26 | IFNAR1_Site85 + GAB2_Site159 + IFNAR1_Site151 + IFNAR1_Site129_B + PTPRC_Site227_H + PTPRC_Site226_A | C6 | 2789 | 0.810 | [0.684 ; 0.936 ] | 1.3304 | 82.8 | 82.8 | 82.8 | 82.8 | 82.8 | N= 58 [ 29 (1); 29 (2)] |
| 27 | IFNAR1_Site85 + PTPRC_Site240 + GAB2_Site159 + IFNAR1_Site151 + PRKCB_Site99 + IFNAR1_Site129_B | C6 | 793 | 0.817 | [0.699 ; 0.935 ] | 1.7361 | 81.0 | 82.8 | 79.3 | 82.1 | 80.0 | N= 58 [ 29 (1); 29 (2)] |
| 28 | IFNAR1_Site85 + PTPRC_Site240 + IFNAR1_Site66 + IFNAR1_Site111_A + GAB2_Site159 + IFNAR1_Site129_B | C6 | 25 | 0.815 | [0.697 ; 0.932 ] | 0.5755 | 81.0 | 89.7 | 72.4 | 87.5 | 76.5 | N= 58 [ 29 (1); 29 (2)] |
| 29 | IFNAR1_Site85 + PTPRC_Site240 + GAB2_Site159 + IFNAR1_Site151 + IFNAR1_Site129_B + PTPRC_Site226_A | C6 | 807 | 0.812 | [0.693 ; 0.931 ] | 1.3813 | 81.0 | 82.8 | 79.3 | 82.1 | 80.0 | N= 58 [ 29 (1); 29 (2)] |
| 30 | IFNAR1_Site85 + PTPRC_Site240 + GAB2_Site159 + PRKCB_Site94 + IFNAR1_Site129_B + PTPRC_Site226_A | C6 | 843 | 0.812 | [0.697 ; 0.927 ] | 0.9701 | 81.0 | 79.3 | 82.8 | 80.0 | 82.1 | N= 58 [ 29 (1); 29 (2)] |
| 31 | IFNAR1_Site85 + IFNAR1_Site66 + GAB2_Site159 + IFNAR1_Site129_B + PTPRC_Site227_H + PTPRC_Site226_A | C6 | 1572 | 0.801 | [ 0.68 ; 0.923 ] | -0.6985 | 81.0 | 86.2 | 75.9 | 84.6 | 78.1 | N= 58 [ 29 (1); 29 (2)] |
| 32 | IFNAR1_Site85 + PTPRC_Site240 + IFNAR1_Site66 + GAB2_Site159 + PRKCB_Site94 + IFNAR1_Site129_B | C6 | 182 | 0.819 | [0.703 ; 0.936 ] | 0.5214 | 81.0 | 86.2 | 75.9 | 84.6 | 78.1 | N= 58 [ 29 (1); 29 (2)] |
| 33 | IFNAR1_Site85 + PTPRC_Site240 + IFNAR1_Site66 + GAB2_Site159 + IFNAR1_Site151 + IFNAR1_Site129_B | C6 | 169 | 0.816 | [0.697 ; 0.934 ] | 1.3532 | 81.0 | 89.7 | 72.4 | 87.5 | 76.5 | N= 58 [ 29 (1); 29 (2)] |
| 34 | IFNAR1_Site85 + IFNAR1_Site66 + GAB2_Site159 + IFNAR1_Site151 + IFNAR1_Site129_B + PTPRC_Site227_H | C6 | 1520 | 0.798 | [0.674 ; 0.921 ] | 1.1937 | 81.0 | 93.1 | 69.0 | 90.9 | 75.0 | N= 58 [ 29 (1); 29 (2)] |
| 35 | IFNAR1_Site85 + PTPRC_Site240 + IFNAR1_Site66 + GAB2_Site159 + IFNAR1_Site129_B + PTPRC_Site227_H | C6 | 188 | 0.815 | [0.698 ; 0.931 ] | 0.1956 | 79.3 | 79.3 | 79.3 | 79.3 | 79.3 | N= 58 [ 29 (1); 29 (2)] |

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 36 | IFNAR1_Site85 + PTPRC_Site240 + IFNAR1_Site66 + GAB2_Site159 + PRKCB_Site99 + IFNAR1_Site129_B | C6 | 176 | 0.812 | [0.694 ; 0.93 ] | 0.5147 | 79.3 | 79.3 | 79.3 | 79.3 | 79.3 | N= 58 [ 29 (1); 29 (2)] |
| 37 | IFNAR1_Site85 + PTPRC_Site240 + IFNAR1_Site66 + IFNAR1_Site152_C + PRKCB_Site152_L + GAB2_Site159 + IFNAR1_Site151 + PRKCB_Site99 + PRKCB_Site94 + IFNAR1_Site129_B + PTPRC_Site227_H + IFNAR1_Site78 + PTPRC_Site226_A + IFNAR1_Site101 | C14 | 67 | 0.860 | [0.755 ; 0.964 ] | 0.166 | 86.2 | 89.7 | 82.8 | 88.9 | 83.9 | N= 58 [ 29 (1); 29 (2)] |
| 38 | IFNAR1_Site85 + PTPRC_Site240 + IFNAR1_Site66 + IFNAR1_Site111_A + PRKCB_Site152_L + GAB2_Site159 + IFNAR1_Site151 + PRKCB_Site99 + PRKCB_Site94 + IFNAR1_Site129_B + PTPRC_Site227_H + IFNAR1_Site78 + PTPRC_Site226_A + IFNAR1_Site101 | C14 | 56 | 0.866 | [0.767 ; 0.964 ] | 0.7721 | 84.5 | 82.8 | 86.2 | 83.3 | 85.7 | N= 58 [ 29 (1); 29 (2)] |
| 39 | IFNAR1_Site85 + PTPRC_Site240 + IFNAR1_Site66 + IFNAR1_Site152_C + PRKCB_Site152_L + GAB2_Site159 + IFNAR1_Site151 + PRKCB_Site99 + PRKCB_Site94 + IFNAR1_Site129_B + IFNAR1_Site78 + PTPRC_Site226_A + IFNAR1_Site101 + IFNAR1_Site84_F | C14 | 71 | 0.864 | [0.765 ; 0.964 ] | -0.7182 | 84.5 | 86.2 | 82.8 | 85.7 | 83.3 | N= 58 [ 29 (1); 29 (2)] |
| 40 | IFNAR1_Site85 + PTPRC_Site240 + IFNAR1_Site66 + IFNAR1_Site111_A + IFNAR1_Site152_C + PRKCB_Site152_L + GAB2_Site159 + IFNAR1_Site151 + PRKCB_Site94 + IFNAR1_Site129_B + PTPRC_Site227_H + IFNAR1_Site78 + PTPRC_Site226_A + IFNAR1_Site101 | C14 | 22 | 0.862 | [0.764 ; 0.96 ] | 1.4897 | 84.5 | 82.8 | 86.2 | 83.3 | 85.7 | N= 58 [ 29 (1); 29 (2)] |

(continued)

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 41 | IFNAR1_Site85 + PTPRC_Site240 + IFNAR1_Site66 + IFNAR1_Site111_A + IFNAR1_Site152_C + GAB2_Site159 + IFNAR1_Site151 + PRKCB_Site99 + PRKCB_Site94 + IFNAR1_Site129_B + PTPRC_Site227_H + IFNAR1_Site78 + PTPRC_Site226_A + IFNAR1_Site84_F | C14 | 47 | 0.862 | [0.764 ; 0.96 ] | -1.1144 | 84.5 | 79.3 | 89.7 | 81.3 | 88.5 | N= 58 [ 29 (1); 29 (2)] |
| 42 | IFNAR1_Site85 + PTPRC_Site240 + IFNAR1_Site66 + IFNAR1_Site152_C + PRKCB_Site152_L + GAB2_Site159 + IFNAR1_Site151 + PRKCB_Site99 + PRKCB_Site94 + IFNAR1_Site129_B + PTPRC_Site227_H + IFNAR1_Site78 + PTPRC_Site226_A + IFNAR1_Site84_F | C14 | 68 | 0.862 | [0.761 ; 0.963 ] | -0.8369 | 84.5 | 86.2 | 82.8 | 85.7 | 83.3 | N= 58 [ 29 (1); 29 (2)] |
| 43 | IFNAR1_Site85 + PTPRC_Site240 + IFNAR1_Site66 + IFNAR1_Site111_A + PRKCB_Site152_L + GAB2_Site159 + IFNAR1_Site151 + PRKCB_Site99 + PRKCB_Site94 + IFNAR1_Site129_B + PTPRC_Site227_H + IFNAR1_Site78 + PTPRC_Site226_A + IFNAR1_Site84_F | C14 | 57 | 0.864 | [0.767 ; 0.962 ] | -0.9497 | 84.5 | 79.3 | 89.7 | 81.3 | 88.5 | N= 58 [ 29 (1); 29 (2)] |
| 44 | IFNAR1_Site85 + PTPRC_Site240 + IFNAR1_Site66 + IFNAR1_Site111_A + IFNAR1_Site152_C + PRKCB_Site152_L + GAB2_Site159 + IFNAR1_Site151 + PRKCB_Site94 + IFNAR1_Site129_B + PTPRC_Site227_H + IFNAR1_Site78 + PTPRC_Site226_A + IFNAR1_Site84_F | C14 | 23 | 0.861 | [0.763 ; 0.959 ] | -0.5853 | 84.5 | 79.3 | 89.7 | 81.3 | 88.5 | N= 58 [ 29 (1); 29 (2)] |

Altogether, these data suggest that the different biomarkers in the panel of 14 genes selected in the prioritization cohorts could be used for the differential diagnosis of Bipolar Disorder.

A panel of the 8 most interesting biomarkers was selected and sequenced in a multiplex format to further validate our results in a large cohort (validation cohort) of 297 subjects, of which 160 were Unipolar depressive patients. The Bipolar disorder population was comprised of 96 Bipolar Patients in an actual depressive episode (Figure 22) and 41 Euthymic patients, which were not taken into account in this analysis for differential diagnosis of Bipolar depression. Thus, a total population of depressed patients (n=256), either suffering from Unipolar or bipolar Depression was analyzed.

All BMKs were combined with each other to evaluate the potential increase in sensibility and specificity using RandomForest (RF). Analysis of the RNA editing variants of 6 BMKs (GAB2, IFNAR1, KCNJ15, LYN, MDM2 and PRKCB) gave an AUC ROC curve of 0.934 (CI 95%: [0.880 - 0.987]), with a sensitivity of 91.2% and a specificity of 84.6% (Figure 29 A) on test dataset, for the first model adjusted by age, sex, psychiatric treatment and addiction.

**Table 5.1 :RandomForest model adjusted by age, sex, psychiatric treatment and addictions Model1, see Figure 29A)**

| List of target in RandomForest model1 | List of A-I RNA editing variants in RandomForest model1 |
|---|---|
| IFNAR1, GAB2, KCNJ15, MDM2, LYN, PRKCB | IFNAR1.1_ABCG, IFNAR1.1_Site33355798, IFNAR1.1_Site33355806_M, IFNAR1.1_Site33355809, IFNAR1.1_Site33355817, IFNAR1.1_Site33355820_O, FNAR1.1_Site33355829_I, GAB2_ADEJ, GAB2_BDEH, GAB2_BDJ, GAB2_DEH, GAB2_DEJ, GAB2_Site78330929_J, GAB2_Site78331080, KCNJ15_ACEHKL, KCNJ15_ACEKLMN, KCNJ15_Site38287584, KCNJ15_Site38287590, KCNJ15_Site38287594, MDM2_AIKTVW, MDM2_ASVX, MDM2_BG, MDM2_G, MDM2_HPQSVX, MDM2_I, MDM2_SV, MDM2_Site68821570, MDM2_Site68821571, MDM2_Site68821572_T, MDM2_Site68821576_V, MDM2_Site68821586_W, MDM2_Site68821612_A, MDM2_Site68821616_X, MDM2_Site68821625, MDM2_Site68821643, PRKCB_AD, PRKCB_M, PRKCB_Site23920874, PRKCB_Site23920921_L, LYN_AF |

In addition, a second random Forest model adjusted only by age and sex using 6 BMKs (CAMK1D, IFNAR1, GAB2, MDM2, KCNJ15 and PRKCB) gave an AUC ROC curve of 0.899 (CI 95%: [0.826 - 0.971]), with a sensitivity of 84.8% and a specificity of 83.7% (Figure 29 B) on test dataset.

**Table 5.2 :RandomForest model adjusted by age and sex (Model2, see Figure 29B)**

| List of target in RandomForest model2 | List of A-I RNA editing variants in randomForest model2 |
|---|---|
| CAMK1D, IFNAR1, GAB2, MDM2, KCNJ15, PRKCB | CAMK1D_AS, CAMK1D_Site12378440, IFNAR1.1_ABCG, IFNAR1.1_Site33355798, IFNAR1.1_Site33355806_M , IFNAR1.1_Site33355809, IFNAR1.1_Site33355817, IFNAR1.1_Site33355820_O, IFNAR1.1_Site33355829_I, GAB2_BDEH, GAB2_BDJ, GAB2_CD, GAB2_CDEJ, GAB2_DEHJ, GAB2_Site78330929_J, GAB2_Site78331080, MDM2_ASVX, MDM2_SV, MDM2_SVW, MDM2_Site68821571, MDM2_Site68821572_T, MDM2_Site68821576_V, MDM2_Site68821584, MDM2_Site68821586_W, MDM2_Site68821602, MDM2_Site68821612_A, MDM2_Site68821616_X, MDM2_Site68821625, MDM2_Site68821643, PRKCB_M, PRKCB_Site23920874, PRKCB_Site23920921_L, KCNJ15_AF |

Both models allow a clear separation of unipolar from bipolar patients

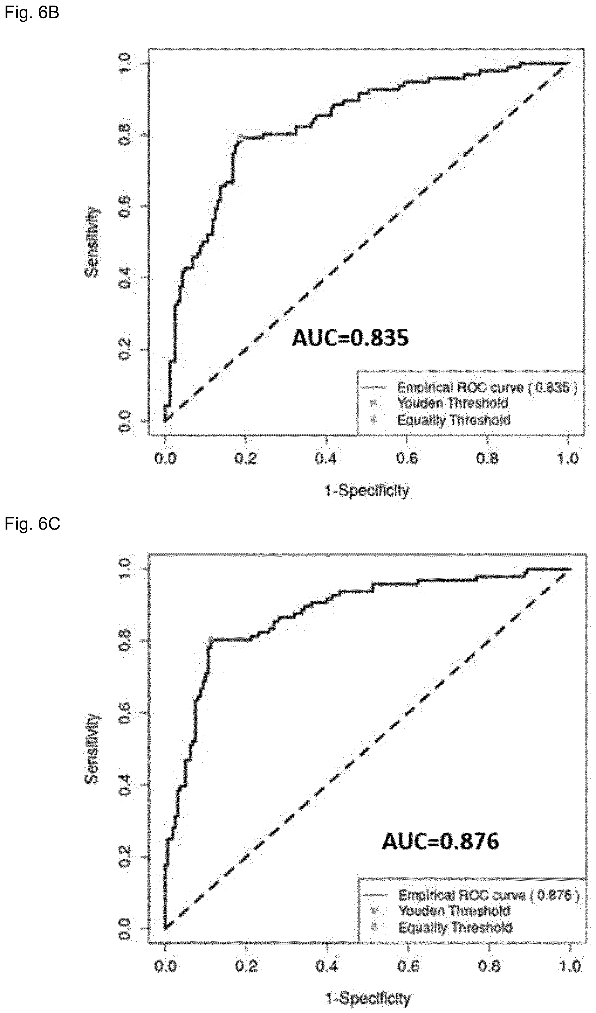

In addition when we analyzed the editing sites of the whole population and combined 29 editing variants in 6 BMKs using mROC approach, we obtained an AUC of 0.829 (Sp 81.3%; Se 78.1%, Figure 6A). When 6 biomarkers included in the multiplex were used and 32 differentially edited sites were combined, the AUC rose to 0.835, with a Specificity of 81.9% and Sensitivity of 80.2% (Figure 6B) using also mROC method. When one more biomarker was added and the number of editing sites in those 7 BMKs was further increased to reach 44 sites, all of which were sequenced in multiplex in a single experiment, the AUC further increased to 0.876 (Sp 88.8%; Se 80.2%, Figure 6C). In the latter case, the equation used is the following:

$$
\begin{aligned}
Z = & + (0.329681470592662)* \text{ MDM2\_Site68821571} + (0.236048209760787)* \text{ CAMK1D\_Site12378440} + \\
& (0.399861222643987)* \text{MDM2\_ASVX} + (0.0160482522095354)* \text{MDM2\_Site68821612\_A} + (0.375844895908091)* \\
& \text{PRKCB\_Site23920921\_L} + (0.116863680480038)* \text{MDM2\_Site68821643} - (0.235485105099063)* \text{PRKCB\_M} + (- \\
& 0.49839847485377)* \quad \text{MDM2\_Site68821576\_V} \quad + \quad (-0.124892097355352)* \quad \text{MDM2\_Site68821616\_X} \quad + \\
& (0.28431910435528)* \text{ MDM2\_SV} + (0.066416717861871)* \text{ MDM2\_Site68821625} + (0.0585507722904674)* \\
& \text{IFNAR1.1\_Site33355798} - (0.220791132716944)* \text{ GAB2\_Site78331080} + (0.373002573187011)* \text{ IFNAR1.1} \\
& \text{Site33355829\_I} + (0.177845820560045)* \text{ IFNAR1.1\_Site33355809} + (0.111074557540484)* \text{ KCNJ15\_AF} + \\
& (0.0432162523917806)* \quad \text{MDM2\_Site68821586\_W} \quad + \quad (0.0198450297310912)* \quad \text{MDM2\_SVW} \quad + \\
& (0.185938219857141)* \text{ PRKCB\_Site23920874} + (0.258962106244043)* \text{ GAB2\_CDEJ} + (0.0733750604485723)* \\
& \text{CAMK1D\_AS} + (0.128439671600768)* \text{ MDM2\_Site68821584} + (0.2412371608579)* \text{ IFNAR1.1\_ABCG} - \\
& (0.176406879704957)* \text{ PRKCB\_AD} + (0.239625600163975)* \text{ IFNAR1.1\_Site33355817} - (0.136223551823182)* \\
& \text{CAMK1D\_DE} \quad + \quad (0.134350140951696)* \quad \text{MDM2\_STX} \quad - \quad (0.112627144618545)* \quad \text{GAB2\_CD} \quad + \quad (- \\
& 0.0683274951449568)* \text{ MDM2\_SWX} + (-0.153964487695514)* \text{ MDM2\_Site68821602} + (0.0876023949116742)* \\
& \text{MDM2\_Site68821563} + (-0.51644072467136)* \text{ IFNAR1.1\_Site33355838\_P} - (0.248333456561587)* \text{ GAB2\_BDEH} \\
& + \quad (0.134133017079705)* \quad \text{IFNAR1.1\_Site33355806\_M} \quad + \quad (0.105132967365637)* \quad \text{MDM2\_WX} \quad + \\
& (0.201724555879812)* \quad \text{GAB2} \quad \text{DEHJ} \quad - \quad (0.14489914590203)* \quad \text{GAB2\_AE} \quad + \quad (-0.309844742843455)* \\
& \text{GAB2\_Site78330929\_J} \quad + \quad (-0.03435818038963)* \quad \text{MDM2\_AS} \quad + \quad (-0.0215011144074209)* \quad \text{LYN\_AF} \quad + \quad (- \\
& 0.196845543135798)* \quad \text{PRKCB\_Site23920844\_B} \quad + \quad (-0.349471597671111)* \quad \text{IFNAR1.1\_Site33355830\_C} \quad + \\
& (0.131993541547468)* \text{ GAB2\_BCDEJ} - (0.146581798182588)* \text{ PRKCB\_K}
\end{aligned}
$$

The list of all combination tested in the second prioritization cohort, all of which included the 86 patients, is presented in Table 6. All combinations were statistically significant between control and cases with a p value $< 10^{-4}$.

**Table 6:** Diagnostic performances obtained with depressed patients (N=256) of the validation cohort analyzed with multiplex sequencing

| 0 | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 1 | MDM2_Site68821571<br><br>CAMK1D_Site12378440<br>MDM2_ASVX<br>MDM2_Site68821612_A<br>PRKCB_Site23920921_L<br>MDM2_Site68821643<br>PRKCB_M<br>MDM2_Site68821576_V<br>MDM2_Site68821616_X<br>MDM2_SV<br>MDM2_Site68821625<br>IFNAR1.1_Site33355798<br>GAB2_Site78331080<br>IFNAR1.1_Site33355829_I<br>IFNAR1.1_Site33355809<br>KCNJ15_AF<br>MDM2_Site68821586_W<br>MDM2_SVW<br>PRKCB_Site23920874<br>GAB2_CDEJ<br>CAMK1D_AS<br>MDM2_Site68821584<br>IFNAR1.1_ABCG PRKCB_AD<br>IFNAR1.1_Site33355817<br>CAMK1D_DE<br>MDM2_STX GAB2_CD<br>MDM2_SWX<br>MDM2_Site68821602<br>MDM2_Site68821563<br>IFNAR1.1_Site33355838_P<br>GAB2_BDEH | C44 | 7490 | 0.876 | [ 0.831 ; 0.922 ] | 0.3342 | 85.6 | 88.8 | 80.2 | 81.1 | 88.2 | N= 256 [ 160 (1); 96 (2)] |

| 0 | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| | IFNAR1.1_Site33355806_M MDM2_WX GAB2_DEHJ GAB2_AE GAB2_Site78330929_J MDM2_AS LYN_AF PRKCB_ Site23920844_B IFNAR1,1_Site33355830_C GAB2_BCDEJ PRKCB_K | | | | | | | | | | | |
| 2 | MDM2_Site68821571 CAMK1D_Site12378440 MDM2_ASVX MDM2_Site68821612_A PRKCB_Site23920921_L MDM2_Site68821643 PRKCB_M MDM2_Site68821576_V MDM2_Site68821616_X MDM2_SV IFNAR1.1_Site33355820_O MDM2_Site68821625 IFNAR1.1_Site33355798 GAB2 Site78331080 IFNAR1.1_Site33355829_I IFNAR1.1_Site33355809 MDM2_Site68821586_W MDM2_SVW PRKCB_Site23920874 MDM2_Site68821572_T GAB2_CDEJ GAB2_BDJ CAMK1D_AS | C44 | 4162 | 0.874 | [ 0.829 ; 0.919] | 0.2976 | 84.8 | 85.6 | 83.3 | 77.7 | 89.5 | N= 256 [ 160 (1); 96 (2)] |

| 0 | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| | MDM2_Site68821584 IFNAR1.1_ABCG PRKCB_AD IFNAR1.1_Site33355817 CAMK1D_DE MDM2_STX GAB2_CD MDM2_SWX MDM2_Site68821563 IFNAR1.1_Site33355838_P GAB2_BDEH MDM2_WX GAB2_DEHJ GAB2_AE GAB2_Site78330929_J MDM2_AS LYN_AF PRKCB_ Site23920844_B IFNAR1.1_Site33355830_C GAB2_BCDEJ PRKCB_K | | | | | | | | | | | |
| 3 | MDM2_Site68821571 CAMK1D_Site12378440 MDM2_ASVX MDM2_Site68821612_A PRKCB_Site23920921_L MDM2_Site68821643 PRKCB_M MDM2_Site68821576_V MDM2_Site68821616_X MDM2_SV IFNAR1.1_Site33355820_O MDM2_Site68821625 IFNAR1.1_Site33355798 | C44 | 937 | 0.873 | [ 0.828 ; 0.919] | 0.3791 | 85.2 | 87.5 | 81.3 | 79.6 | 88.6 | N= 256 [ 160 (1); 96 (2)] |

EP 4 055 191 B1

37

| 0 | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| | GAB2_Site78331080 IFNAR1.1_Site33355829_I IFNAR1.1_Site33355809 KCNJ15_AF MDM2_Site68821586_W MDM2_SVW PRKCB_Site23920874 MDM2_Site68821572_T GAB2_CDEJ GAB2_BDJ CAMK1D_AS MDM2_Site68821584 IFNAR1.1_ABCG PRKCB_AD IFNAR1.1_Site33355817 MDM2_STX MDM2_SWX MDM2_Site68821602 MDM2_Site68821563 IFNAR1.1_Site33355838_P GAB2_BDEH IFNAR1.1_Site33355806_M MDM2_WX GAB2_DEHJ GAB2_AE GAB2_Site78330929_J MDM2_AS LYN_AF PRKCB_ Site23920844_B IFNAR1,1_Site33355830_C GAB2_BCDEJ | | | | | | | | | | | |
| 4 | MDM2_Site68821571<br><br>CAMK1D_Site12378440 MDM2_ASVX MDM2_Site68821612_A | C32 | 4082 | 0.836 | [ 0.785 ; 0.887] | 0.2281 | 81.3 | 81.9 | 80.2 | 72.6 | 87.3 | N= 256 [ 160 (1); 96 (2)] |

| 0 | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| | MDM2_Site68821643 PRKCB_M MDM2_Site68821576_V MDM2_Site68821616_X MDM2_SV IFNAR1.1_Site33355820_O MDM2_Site68821625 IFNAR1.1_Site33355798 GAB2_Site78331080 IFNAR1.1_Site33355829_I IFNAR1.1_Site33355809 KCNJ15_AF MDM2_Site68821586_W MDM2_SVW PRKCB_Site23920874 MDM2_Site68821572_T GAB2_CDEJ GAB2_BDJ CAMK1D_AS MDM2_Site68821584 IFNAR1.1_ABCG PRKCB_AD CAMK1D_DE MDM2_STX GAB2_CD MDM2_Site68821602 IFNAR1.1_Site33355830_C MDM2_SWX | | | | | | | | | | | |
| 5 | MDM2_Site68821571 CAMK1D_Site12378440 MDM2_ASVX MDM2_Site68821612_A MDM2_Site68821643 PRKCB_M | C32 | 4375 | 0.832 | [0.78; 0.884] | 0.2504 | 80.9 | 82.5 | 78.1 | 72.8 | 86.3 | N= 256 [ 160 (1); 96 (2)] |

| 0 | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| | MDM2_Site68821576_V<br>MDM2_Site68821616_X<br>IFNAR1.1_Site33355820_O<br>MDM2_Site68821625<br>IFNAR1.1_Site33355798<br>GAB2_Site78331080<br>IFNAR1.1_Site33355829_I<br>IFNAR1.1_Site33355809<br>KCNJ15_AF<br>MDM2_Site68821586_W<br>MDM2_SVW<br>PRKCB_Site23920874<br>GAB2_CDEJ<br>GAB2_BDJ CAMK1D_AS<br>MDM2_Site68821584<br>IFNAR1.1_ABCG<br>PRKCB_AD<br>IFNAR1.1_Site33355817<br>CAMK1D_DE MDM2_STX<br>GAB2_CD<br>MDM2_Site68821602<br>IFNAR1.1_Site33355830_C<br>MDM2_SWX<br>PRKCB_Site23920844_B | | | | | | | | | | | |
| 6 | MDM2_Site68821571<br><br>CAMK1D_Site12378440<br>MDM2_ASVX<br>MDM2_Site68821612_A<br>MDM2_Site68821643<br>PRKCB_M<br>MDM2_Site68821576_V | C32 | 4239 | 0.835 | [ 0.784 ;<br>0.887 ] | 0.2376 | 80.5 | 81.3 | 79.2 | 71.7 | 86.7 | N= 256 [ 160 (1);<br>96 (2)] |

EP 4 055 191 B1

| 0 | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| | MDM2_Site68821616_X<br>MDM2_SV<br>IFNAR1.1_Site33355820_O<br>MDM2_Site68821625<br>IFNAR1.1_Site33355798<br>GAB2_Site78331080<br>IFNAR1.1_Site33355829_I<br>KCNJ15_AF<br>MDM2_Site68821586_W<br>MDM2_SVW<br>PRKCB_Site23920874<br>MDM2_Site68821572_T<br>GAB2_CDEJ<br>GAB2_BDJ CAMK1D_AS<br>MDM2_Site68821584<br>IFNAR1.1_ABCG<br>PRKCB_AD CAMK1D_DE<br>MDM2_STX<br>GAB2_CD<br>MDM2_Site68821602<br>IFNAR1.1_Site33355830_C<br>MDM2_SWX<br>PRKCB_Site23920844_B | | | | | | | | | | | |
| 7 | MDM2_Site68821571<br><br>CAMK1D_Site12378440<br>MDM2_ASVX<br>MDM2_Site68821612_A<br>MDM2_Site68821643<br>PRKCB_M<br>MDM2_Site68821576_V<br>MDM2_Site68821616_X<br>MDM2_SV | C29 | 2926 | 0.829 | [ 0.776 ; 0.881 ] | 0.1964 | 80.1 | 81.3 | 78.1 | 71.4 | 86.1 | N= 256 [ 160 (1); 96 (2)] |

EP 4 055 191 B1

41

(continued)

| 0 | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| | IFNAR1.1_Site33355820_O MDM2_Site68821625 IFNAR1.1_Site33355798 GAB2_Site78331080 IFNAR1.1_Site33355829_I IFNAR1.1_Site33355809 KCNJ15_AF MDM2_Site68821586_W MDM2_SVW PRKCB_Site23920874 MDM2_Site68821572_T GAB2_CDEJ GAB2_BDJ CAMK1D_AS MDM2_Site68821584 IFNAR1.1_ABCG PRKCB_AD IFNAR1.1_Site33355817 CAMK1D_DE MDM2_STX | | | | | | | | | | | |
| 8 | MDM2_Site68821571 CAMK1D_Site12378440 MDM2_ASVX MDM2_Site68821612_A MDM2_Site68821643 PRKCB_M MDM2_Site68821576_V MDM2_Site68821616_X MDM2_SV IFNAR1.1_Site33355820_O MDM2_Site68821625 IFNAR1.1_Site33355798 GAB2_Site78331080 | C29 | 3063 | 0.828 | [ 0.775 ; 0.881 ] | 0.294 | 80.9 | 85.6 | 72.9 | 75.3 | 84.1 | N= 256 [160 (1); 96 (2)] |

42

| 0 | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| | IFNAR1.1_Site33355809 KCNJ15_AF MDM2_Site68821586_W MDM2_SVW PRKCB_Site23920874 MDM2_Site68821572_T GAB2_CDEJ GAB2_BDJ CAMK1D_AS MDM2_Site68821584 IFNAR1.1_ABCG PRKCB_AD IFNAR1.1_Site33355817 CAMK1D_DE MDM2_STX MDM2_Site68821602 | | | | | | | | | | | |
| 9 | MDM2_Site68821571<br><br>CAMK1D_Site12378440 MDM2_ASVX MDM2_Site68821612_A PRKCB_Site23920921_L MDM2_Site68821643 PRKCB_M MDM2_Site68821576_V MDM2_Site68821616_X MDM2_SV IFNAR1.1_Site33355820_O IFNAR1.1_Site33355798 GAB2_Site78331080 IFNAR1.1_Site33355809 KCNJ15_AF MDM2_Site68821586 _W MDM2_SVW PRKCB_Site23920874 | C29 | 1277 | 0.829 | [ 0.778 ; 0.881 ] | 0.1885 | 78.9 | 78.1 | 80.2 | 68.8 | 86.8 | N= 256 [ 160 (1); 96 (2)] |

EP 4 055 191 B1

| 0 | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
|  | MDM2_Site68821572_T GAB2_CDEJ GAB2_BDJ CAMK1D_AS MDM2_Site68821584 IFNAR1.1_ABCG PRKCB_AD IFNAR1.1_Site33355817 CAMK1D_DE MDM2_STX GAB2_CD |  |  |  |  |  |  |  |  |  |  |  |
| 10 | CAMK1D_Site12378440 PRKCB_Site23920921_L MDM2_Site68821576_V MDM2_Site68821616_X MDM2_SV IFNAR1.1_Site33355829_I MDM2_Site68821586_W MDM2_Site68821572_T CAMK1D_AS MDM2_SWX MDM2_Site68821602 MDM2_Site68821563 IFNAR1.1_Site33355838_P IFNAR1.1_Site33355806_M MDM2_WX MDM2_AS PRKCB_ Site23920844_B IFNAR1.1_Site33355830_C | C18 | 1297 | 0.728 | [ 0.663 ; 0.792 ] | 0.088 | 74.2 | 76.9 | 69.8 | 64.4 | 80.9 | N= 256 [ 160 (1); 96 (2)] |
| 11 | MDM2_Site68821571 CAMK1D_Site12378440 PRKCB_Site23920921_L | C18 | 824 | 0.754 | [ 0.691 ; 0.817] | 0.1197 | 73.1 | 74.4 | 70.8 | 62.4 | 81.0 | N= 256 [ 160 (1); 96 (2)] |

EP 4 055 191 B1

| 0 | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| | MDM2_Site68821576_V MDM2_Site68821616_X MDM2_SV IFNAR1.1_Site33355829_I MDM2_Site68821586_W MDM2_Site68821572_T CAMK1D_AS MDM2_SWX MDM2_Site68821602 MDM2_Site68821563 IFNAR1.1_Site33355838_P IFNAR1.1_Site33355806_M GAB2_Site78330929_J MDM2_AS IFNAR1.1_Site33355830_C | | | | | | | | | | | |
| 12 | MDM2_Site68821571  CAMK1D_Site12378440 PRKCB_Site23920921_L MDM2_Site68821576_V MDM2_Site68821616_X MDM2_SV IFNAR1.1_Site33355829_I MDM2_Site68821586_W MDM2_Site68821572_T CAMK1D_AS MDM2_SWX MDM2_Site68821602 IFNAR1.1_Site33355838_P IFNAR1.1_Site33355806_M MDM2_WX GAB2_Site78330929_J MDM2_AS | C18 | 838 | 0.752 | [ 0.689 ; 0.815] | 0.0749 | 72.3 | 71.3 | 74.0 | 60.7 | 82.0 | N= 256 [ 160 (1); 96 (2)] |

EP 4 055 191 B1

(continued)

| Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|
| PRKCB_Site23920844_B | | | | | | | | | | | |

The panel of biomarkers analyzed above thus gave excellent diagnostic performances to differentially diagnose BD and Unipolar in the whole population of depressed patients of the validation cohort (256 subjects), suggesting that our panel of biomarkers could indeed have clinical applications in the differential diagnosis of BD.

We next sought to investigate whether the diagnostic performances of our assay could be further increased by taking into account sex-specific differences. Indeed, the existence of sex-specific transcriptional signatures has been suggested in depression. It has previously been shown that the brain transcriptional profile of depressed patients differs greatly by sex, suggesting that the molecular mechanisms underlying depression could differ between women and men[52]. Modifications in RNA editing were thus analyzed separately in the male and the female population of the validation cohort for depressive Uni and BD patients. When sex-specificity was introduced in the analysis, a significant improvement of diagnostic performances was observed. Noteworthy, the female validation cohort (n=179) included more subjects than the male validation cohort (n=77), as this is reflecting the proportion of males and females suffering depression in the general population worldwide. Indeed, in the female population of the validation cohort (n=179, 110 Unipolar and 69 BD patients), when we combined 17 variants included in 7 biomarkers, the AUC was 0.834 (Sp 93.6%; Se 65.2%, Figure 7A). The equation used in this case is the following:

Z= - ( 0.238519750711572 )* PRKCB_M + ( 0.228320745055679 )* MDM2_Site68821571 + ( 0.226760422516651 )* LYN_AH - ( 0.273858599096893 )* CAMK1D_DE + ( 0.166296414954285 )* MDM2_Site68821625 + ( 0.0683620503515642 )* PDE8A_Site85096717_C - ( 0.194138597912751 )* GAB2_CD + ( 0.00210889117258426 )* MDM2_Site68821572_T - ( 0.178806599979693 )* IFNAR1.1_E - ( 0.0810270919845117 )* GAB2_BCD + ( 0.118255695159956 )* PRKCB_Site23920921_L + ( -0.197975165223792 )* MDM2_Site68821576_V - ( 0.213318581871191 )* GAB2_Site78331080 + ( 0.192660926804618 )* GAB2_CDEJ + ( 0.124216008873012 )* LYN_AM + ( 0.102544252586713 )* MDM2_ASVX + ( 0.107503746972775 )* LYN_Site56012555

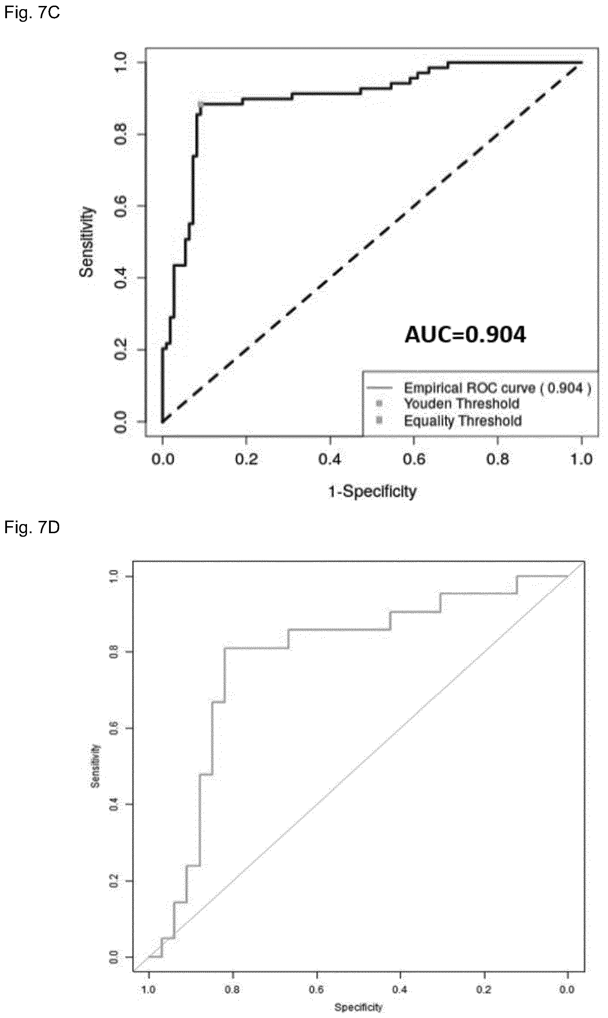

When the number of RNA editing variants was increased to 23, in 7 different targets, the AUC rose to 0.851 with both specificity and sensitivity above 80% (Sp 82.7%; Se 81.2%, Figure 7B). Moreover, when we used the 8 biomarkers included in the multiplex and combined 44 differentially edited sites, the AUC reached to 0.904, (Sp 90.9%; Se 88.4%, Figure 7C).

Using machine-learning approach with both mROC and RandomForest (see material and methods), we randomly separated the sex-specific populations into a train- and a test set. When randomly splitting the female population into a train set (70% of the whole population) and a test set (30% of the whole population) and applying the above-mentioned methods, we obtained an AUC of 0.782 in the test set with the mROC method (Sp 81.8%; Se 81.0%, Figure 7D) and an AUC of 0.858 with the RandomForest method (Sp 80.0%; Se 81.0%, Figure 7E). Altogether, the data obtained by two different machine-learning methods on the female validation cohort confirmed the excellent diagnostic performances of our panel of biomarkers.

The list of all combination tested in the female validation cohort, is presented in Table 7. All combinations were statistically significant between control and cases with a p value $<10^{-4}$.

**Table 7:** Diagnostic performances obtained with patients of the female population of the validation cohort n=179) analyzed by multiplex sequencing of 8 targets.

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 1 | PRKCB_M MDM2_Site68821571 LYN_AH CAMK1D_DE MDM2_Site68821625 MDM2_Site68821586_ W MDM2_Site68821612_ A PDE8A_ Site85096717_C GAB2_CD MDM2_Site68821572_ T IFNAR1.1_E GAB2_BCD PRKCB_ Site23920921_L MDM2_Site68821576_ V MDM2_T GAB2_Site78331080 GAB2_CDEJ LYN_AM MDM2_ASVX MDM2_Site68821616_ X IFNAR1.1_Site3335579 8 LYN_W MDM2_Site68821643 LYN_F GAB2_BDEH LYN_ Site56012491 GAB2_E MDM2_SVWGAB2_BD GAB2_AE CAMK1D_ DES GAB2_CDJ MDM2_Site68821567 | C44 | 2361 | 0.904 | [ 0.855 ; 0.952 ] | 0.3707 | 89.94 | 90.91 | 88.41 | 85.92 | 92.59 | N= 179 [ 110 (1); 69 (2)] |

(continued)

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
|  | MDM2_Site68821584 MDM2_SWX PRKCB_ADJK KCNJ15_AF LYN_Site56012553_A CAMK1 D_AG IFNAR1.1_Site3335582 0_O GAB2_BC GAB2_CE GAB2_BDJ MDM2_K |  |  |  |  |  |  |  |  |  |  |  |
| 2 | PRKCB_M MDM2_Site68821571 LYN_AH MDM2_Site68821586_W MDM2_Site68821612_A PDE8A_Site85096717_C GAB2_CD MDM2_Site68821572_T IFNAR1.1_E GAB2_BCD PRKCB_Site23920921_L MDM2_Site68821576_V MDM2_T GAB2_Site78331080 GAB2_CDEJ LYN_AM MDM2_ASVX MDM2_Site68821616_X IFNAR1.1_Site3335579 8 | C44 | 13211 | 0.916 | [ 0.871 ; 0.96 ] | 0.245 | 89.4 | 89.1 | 89.9 | 83.8 | 93.3 | N= 179 [ 110 (1); 69 (2)] |

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| | LYN_Site56012555 LYN_W | | | | | | | | | | | |
| | MDM2_Site68821643 LYN_F GAB2_BDEH LYN_ Site56012491 GAB2_E MDM2_SVWGAB2_BD GAB2_AE CAMK1D_ DES GAB2_CDJ MDM2_Site68821567 MDM2_Site68821584 GAB2_BCDJ MDM2_SWX PRKCB_ ADJK LYN_Site56012553_A CAMK1 D_AG MDM2_SW IFNAR1.1_Site3335582 0_O GAB2_BC GAB2_CE GAB2_BDJ MDM2_K | | | | | | | | | | | |
| 3 | PRKCB_M MDM2_Site68821571 LYN_AH CAMK1D_DE MDM2_Site68821625 MDM2_Site68821586_ W MDM2_Site68821612_ A PDE8A_ Site85096717_C GAB2_CD | C44 | 847 | 0.915 | [ 0.871 ; 0.959 ] | 0.274 | 89.4 | 89.1 | 89.9 | 83.8 | 93.3 | N= 179 [110 (1); 69 (2)] |

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| | MDM2_Site68821572_T IFNAR1.1_E GAB2_BCD PRKCB_Site23920921_L MDM2_Site68821576_V MDM2_T GAB2_Site78331080 GAB2_CDEJ LYN_AM MDM2_ASVX MDM2_Site68821616_X IFNAR1.1_Site33355798 LYN_Site56012555 LYN_W MDM2_Site68821643 LYN_F GAB2_BDEH LYN_Site56012491 GAB2_E GAB2_BD GAB2_AE CAMK1D_DES GAB2_CDJ MDM2_Site68821567 MDM2_Site68821584 GAB2_BCDJ MDM2_SWX PRKCB_ADJK LYN_Site56012553_A CAMK1D_AG MDM2_SW IFNAR1.1_Site33355820_O GAB2_BC | | | | | | | | | | | |

EP 4 055 191 B1

(continued)

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| | GAB2_BDJ MDM2_K | | | | | | | | | | | |
| 4 | LYN_AH CAMK1D_DE<br><br>MDM2_Site68821625<br>MDM2_Site68821586_W<br>MDM2_Site68821612_A<br>PDE8A_Site85096717_C<br>GAB2_CD<br>MDM2_Site68821572_T IFNAR1.1_E<br>GAB2_BCD PRKCB_Site23920921_L | C23 | 2584 | 0.842 | [ 0.78 ; 0.905 ] | 0.289 | 83.8 | 88.2 | 76.8 | 80.3 | 85.8 | N= 179 [110 (1); 69 (2)] |
| | MDM2_Site68821576_V MDM2_T<br>GAB2_Site78331080<br>GAB2_CDEJ<br>LYN_AM LYN_Site56012555<br>MDM2_Site68821616_X<br>MDM2_Site68821602<br>MDM2_SWX<br>IFNAR1.1_Site33355806_M<br>IFNAR1.1_ABCG<br>MDM2_Site68821584 | | | | | | | | | | | |
| 5 | PRKCB_M<br>MDM2_Site68821571<br>LYN_AH CAMK1D_DE | C23 | 1336 | 0.851 | [ 0.79; 0.911 ] | 0.228 | 82.1 | 82.7 | 81.2 | 74.7 | 87.5 | N= 179 [ 110 (1); 69 (2)] |

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| | MDM2_Site68821586_W MDM2_Site68821612_A PDE8A_Site85096717_C GAB2_CD MDM2_Site68821572_T IFNAR1.1_E GAB2_BCD PRKCB_Site23920921_L MDM2_Site68821576_V MDM2_T GAB2_Site78331080 GAB2_CDEJ LYN_AM MDM2_ASVX LYN_Site56012555 MDM2_Site68821616_X MDM2_Site68821602 IFNAR1.1_ABCG MDM2_Site68821584 | | | | | | | | | | | |
| 6 | PRKCB_M MDM2_Site68821571 LYN_AH CAMK1D_DE MDM2_Site68821625 MDM2_Site68821586_W MDM2_Site68821612_A PDE8A_Site85096717_C GAB2_CD | C23 | 348 | 0.843 | [ 0.782 ; 0.904 ] | 0.220 | 82.1 | 82.7 | 81.2 | 74.7 | 87.5 | N= 179 [ 110 (1); 69 (2)] |

EP 4 055 191 B1

53

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| | MDM2_Site68821572_T IFNAR1.1_E GAB2_BCD MDM2_Site68821576_V GAB2_Site78331080 GAB2_CDEJ LYN_AM MDM2_ASVX MDM2_Site68821616_X MDM2_Site68821602 MDM2_SWX IFNAR1.1_Site33355806_M IFNAR1.1_ABCG MDM2_Site68821584 | | | | | | | | | | | |
| 7 | MDM2_Site68821571 LYN_AH CAMK1D_DE MDM2_Site68821586_W PDE8A_Site85096717_C GAB2_CD MDM2_Site68821572_TIFNAR1.1_E | C17 | 1088 | 0.829 | [ 0.763 ; 0.895 ] | 0.398 | 82.7 | 90.9 | 69.6 | 82.8 | 82.6 | N= 179 [ 110 (1); 69 (2)] |
| | GAB2_BCD PRKCB_Site23920921_L MDM2_Site68821576_V MDM2_T GAB2_Site78331080 GAB2_CDEJ LYN_AM MDM2_ASVX | | | | | | | | | | | | |

EP 4 055 191 B1

54

(continued)

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| | LYN_Site56012555 | | | | | | | | | | | N= 179 [110 (1); 69 (2)] |
| 8 | PRKCB_M MDM2_Site68821571 LYN_AH CAMK1D_DE MDM2_Site68821625 PDE8A_Site85096717_C GAB2_CD MDM2_Site68821572_T IFNAR1.1_E GAB2_BCD PRKCB_Site23920921_L MDM2_Site68821576_V GAB2_Site78331080 GAB2_CDEJ LYN_AM MDM2_ASVX LYN_Site56012555 | C17 | 448 | 0.834 | [ 0.77 ; 0.897 ] | 0.437 | 82.7 | 93.6 | 65.2 | 86.5 | 81.1 | N= 179 [110 (1); 69 (2)] |
| 9 | PRKCB_M MDM2_Site68821571 LYN_AH CAMK1D_DE MDM2_Site68821586_W MDM2_Site68821612_A PDE8A_Site85096717_C MDM2_Site68821572_T IFNAR1.1_E GAB2_BCD PRKCB_Site23920921_L MDM2_T GAB2_Site78331080 | C17 | 517 | 0.824 | [ 0.758 ; 0.889 ] | 0.145 | 79.3 | 80.0 | 78.3 | 71.1 | 85.4 | N= 179 [ 110 (1); 69 (2)] |

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| | GAB2_CDEJ LYN_AM MDM2_ASVX LYN_Site56012555 | | | | | | | | | | | |
| 10 | PRKCB_M MDM2_Site68821571 LYN_AH CAMK1D_DE MDM2_Site68821612_ A PDE8A_ Site85096717_C MDM2_Site68821572_ T IFNAR1.1_E GAB2_BCD PRKCB_ Site23920921_L MDM2_Site68821576_ V MDM2_T GAB2_Site78331080 GAB2_CDEJ LYN_AM MDM2_ASVX LYN_Site56012555 | C17 | 558 | 0.828 | [ 0.762 ; 0.893 ] | 0.158 | 78.8 | 79.1 | 78.3 | 70.1 | 85.3 | N= 179 [ 110 (1); 69 (2)] |

The performances of the male population were even better. Indeed, when analyzing separately the male population of the validation cohort (n=77, 50 Unipolar and 27 BD patients) and included 18 editing variants in 6 BMKs, we obtained an AUC of 0.918 (Sp 86.0%; Se 88.9%, Figure 8A). In this case, the equation used is the following:

+ ( 0.1918844546789 )* CAMK1D_AS + ( 0.614946165065453 )* PRKCB_Site23920921_L + ( 0.55731200448775 )* CAMK1D_CE + ( 0.277761284589955 )* PRKCB_DJKM + ( -0.360618380284487 )* MDM2_SV + ( 0.306320519931475 )* IFNAR1.1_Site33355809 + ( 0.456841145698979 )* MDM2_ASVX + ( -0.0482494197161185 )* CAMK1D_ES + ( 0.0376282919358852 )* GAB2_DE - ( 0.710430719330234 )* LYN_F + ( 0.118310642804172 )* MDM2_Site68821643 + ( -0.135214425904151 )* IFNAR1.1_Site33355820_0 + ( 0.309202697888314 )* LYN_AF + ( -0.0028544497314405 )* LYN_Site56012521 + ( 0.266463664183522 )* MDM2_STVX + ( 0.315139778360142 )* CAMK1D_BD + ( - 0.0526783771932407 )* PRKCB_Site23920868 + ( 0.123898740861286 )* IFNAR1.1_ABCE.

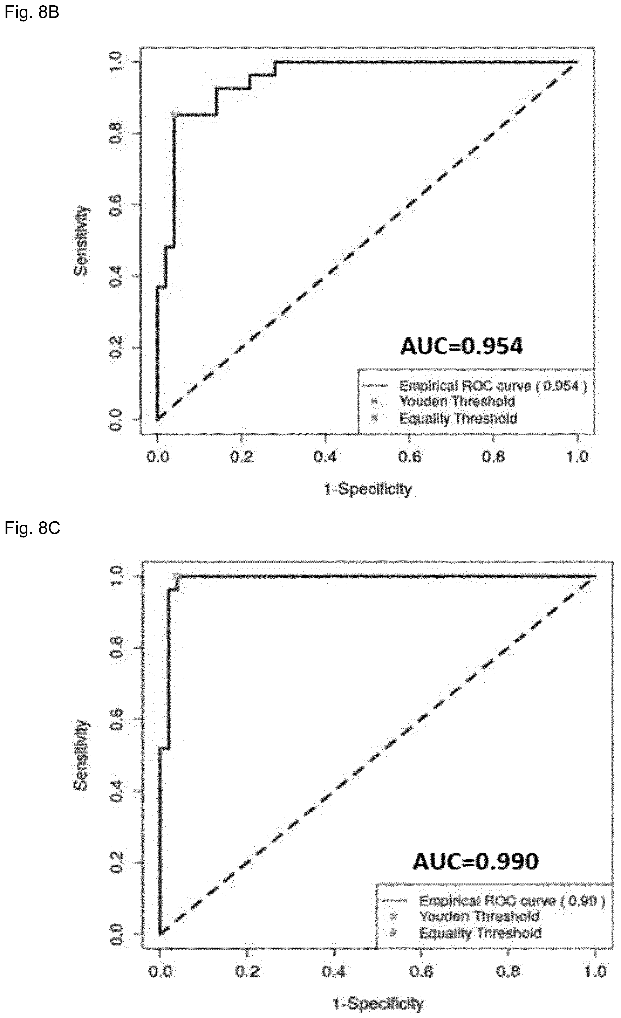

When 39 RNA editing variants were taken into account within those 6 BMKs, the AUC was above 0.9 (AUC 0.954, Spe 96.0%, Se 85.2%, Figure 8B). Furthermore, when the number of editing variants in those same 6 BMKs was increased to 47, we obtained an AUC of 0.99 (Sp 96.0%; Se 100%, Figure 8C).

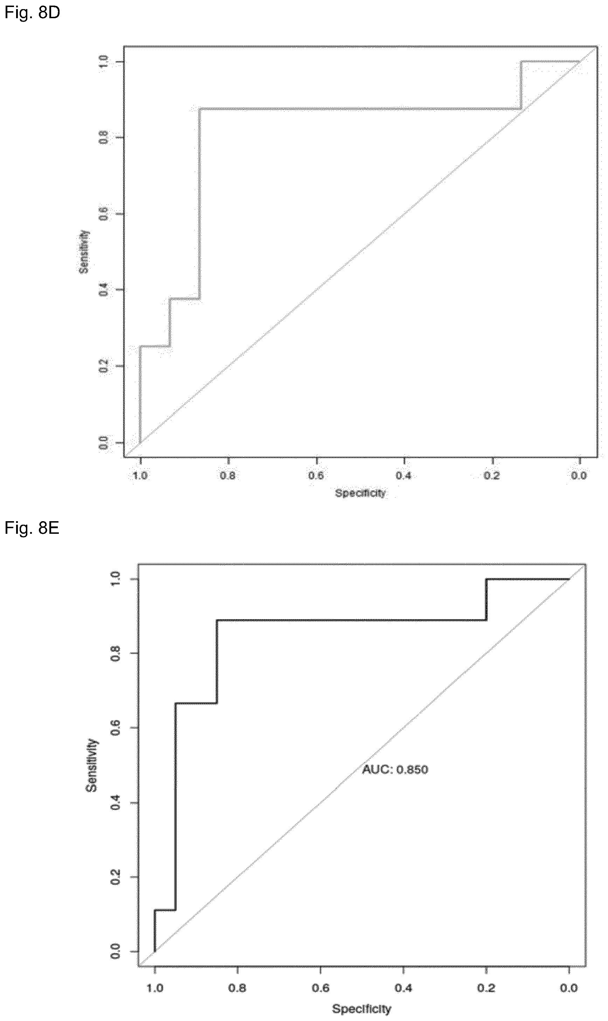

Using machine-learning, we randomly split the male population into a training (65% of the whole population) and a test set (35% of the whole population) and obtained an AUC with mROC was 0.817 (Sp 86.7%, Se 87.5%, Figure 8D), while the RandomForest method led to an AUC of 0.850 (Sp 89%, Se 80%, Figure 8E).

The list of all combination tested in the male validation cohort, is presented in Table 8. All combinations were statistically significant between control and cases with a p value $<10^{-4}$.

**Table 8:** Diagnostic performances obtained with patients of the male population of the validation cohort (n=77) analyzed by multiplex sequencing of 8 targets.

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 1 | CAMK1D_AS PRKCB_ Site23920921_L CAMK1D_CE PRKCB_ DJKM MDM2_BG MDM2_SV CAMK1D_EFS MDM2_ASVX CAMK1D_ES MDM2_AST GAB2_DE LYN_F MDM2_Site68821643 IFNAR1.1_Site33355820_ O LYN_AF LYN_Site56012521 MDM2_STVX CAMK1D_BD PRKCB_ Site23920868 IFNAR1.1_ABCE MDM2_SX PRKCB_Site23920844_B PRKCB_Site23920889_F CAMK1D_Site12378439 CAMK1 D_CS MDM2_STX MDM2_Site68821571 MDM2_VX MDM2_Site68821576_V MDM2_Site68821616_X CAMK1D_EF GAB2_DEJ MDM2_KSX PRKCB_Site23920927_M PRKCB_A CAMK1D_ABEGS GAB2_BH MDM2_KS | **C47** | 12517 | 0.990 | [0.97; 1.00] | 1.174 | 97.4 | **96.0** | **100.0** | 93.1 | 100.0 | N = 77 [50 (1); 27 (2)] |

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| | PRKCB_Site23920910_J GAB2_BD CAMK1D_DS CAMK1D_ Site12378351 PRKCB_Site23920919_K PDE8A_E CAMK1D_BES PDE8A_ Site85096684 GAB2_CEJ | | | | | | | | | | | |
| 2 | CAMK1D_AS PRKCB_ Site23920921_L CAMK1D_CE PRKCB_ DJKM MDM2_BG MDM2_SV CAMK1D_EFS MDM2_ASVX CAMK1D_ES MDM2_AST GAB2_DE LYN_F MDM2_Site68821643 LYN_AF LYN_Site56012521 MDM2_STVX CAMK1D_BD PRKCB_ Site23920868 IFNAR1.1_ABCE MDM2_SX PRKCB_Site23920844_B PRKCB_Site23920889_F CAMK1D_Site12378439 CAMK1D_CS MDM2_STX MDM2_Site68821571 | C47 | 12956 | 0.988 | [ 0.967 ; 1.00] | 0.717 | 97.4 | 96.0 | 100.0 | 93.1 | 100.0 | N=77 [50 (1); 27 (2)] |

EP 4 055 191 B1

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| | MDM2_VX MDM2_Site68821576_V MDM2_Site68821616_X CAMK1D_EF GAB2_DEJ MDM2_KSX PRKCB_Site23920927_M PRKCB_A CAMK1D_ABEGS GAB2_BH MDM2_KS PRKCB_Site23920910_J GAB2_BD CAMK1D_DS CAMK1D_ Site12378351 PRKCB_Site23920919_K MDM2_Site68821612_A PDE8A_E CAMK1D_BES PDE8A_ Site85096684 GAB2_CEJ | | | | | | | | | | | |
| 3 | CAMK1D_AS PRKCB_ Site23920921_L CAMK1D_CE PRKCB_ DJKM MDM2_BG MDM2_SV IFNAR1.1_Site33355809 CAMK1D_EFS MDM2_ASVX CAMK1D_ES MDM2_AST GAB2_DE LYN_F MDM2_Site68821643 IFNAR1.1_Site33355820_ O | **C47** | 6160 | 0.987 | [ 0.963 ; 1.00] | 1.220 | 97.4 | **96.0** | **100.0** | 93.1 | 100.0 | N= 77 [50 (1); 27 (2)] |

EP 4 055 191 B1

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| | LYN_Site56012521 MDM2_STVX CAMK1D_BD PRKCB_ Site23920868 IFNAR1.1_ABCE MDM2_SX PRKCB_Site23920844_B PRKCB_Site23920889_F CAMK1D_Site12378439 CAMK1D_CS MDM2_STX MDM2_Site68821571 MDM2_VX MDM2_Site68821576_V MDM2_Site68821616_X CAMK1D_EF GAB2_DEJ MDM2_KSX PRKCB_Site23920927_M PRKCB_A CAMK1D_ABEGS GAB2_BH MDM2_KS PRKCB_Site23920910_J GAB2_BD CAMK1D_DS CAMK1D Site12378351 PRKCB_Site23920919_K PDE8A_E CAMK1D_BES PDE8A_ Site85096684 GAB2_CEJ | | | | | | | | | | | |

EP 4 055 191 B1

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 4 | PRKCB_Site23920921_L CAMK1D_CE PRKCB_DJKM MDM2_BG MDM2_SV IFNAR1.1_Site33355809 CAMK1D_EFS MDM2_ASVX CAMK1D_ ES MDM2_AST GAB2_DE LYN_F MDM2_Site68821643 IFNAR1.1_Site33355820_ O LYN_AF LYN_Site56012521 MDM2_STVX PRKCB_Site23920868 IFNAR1.1_ABCE MDM2_Site68821576_V MDM2_Site68821612_A CAMK1D_Site12378439 PRKCB_Site23920889_F PRKCB_Site23920872_C MDM2_Site68821616_X MDM2_Site68821602 MDM2_Site68821571 | C27 | 3715 | 0.954 | [0.912; 0.996 ] | 0.618 | 92.2 | 96.0 | 85.2 | 92.0 | 92.3 | N=77 [50 (1); 27 (2)] |
| 5 | CAMK1D_AS PRKCB_ Site23920921_L CAMK1D_CE PRKCB_ DJKM MDM2_BG MDM2_SV IFNAR1.1_Site33355809 CAMK1D_EFS MDM2_ASVX | C27 | 1260 | 0.942 | [ 0.895 ; 0.99 ] | 0.099 | 90.9 | 86.0 | 100.0 | 79.4 | 100.0 | N=77 [50 (1); 27 (2)] |

(continued)

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| | MDM2_AST GAB2_DE LYN_F<br>MDM2_Site68821643<br>LYN_Site56012521<br>MDM2_STVX<br>CAMK1D_BD PRKCB_Site23920868<br>IFNAR1.1_ABCE<br>MDM2_Site68821576_V<br>MDM2_Site68821612_A<br>CAMK1D_Site12378439<br>PRKCB_Site23920844_B<br>PRKCB_Site23920889_F<br>PRKCB_Site23920872_C<br>MDM2_Site68821616_X<br>MDM2_Site68821602<br>MDM2_Site68821571 | | | | | | | | | | | |
| 6 | PRKCB_Site23920921_L<br>CAMK1D_CE<br>PRKCB_DJKM MDM2_BG MDM2_SV<br>IFNAR1.1_Site33355809<br>CAMK1D_EFS<br>MDM2_ASVX CAMK1D_ES MDM2_AST<br>GAB2_DE LYN_F<br>MDM2_Site68821643<br>IFNAR1.1_Site33355820_O<br>LYN_Site56012521<br>MDM2_STVX<br>CAMK1D_BD PRKCB_Site23920868 | C27 | 3755 | 0.939 | [0.889 ; 0.989] | 0.220 | 90.9 | **86.0** | **100.0** | 79.4 | 100.0 | N=77 [50(1); 27(2)] |

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| | MDM2_Site68821576_V MDM2_Site68821612_A CAMK1D_Site12378439 PRKCB_Site23920844_B PRKCB_Site23920889_F PRKCB_Site23920872_C MDM2_Site68821616_X MDM2_Site68821602 MDM2_Site68821571 | | | | | | | | | | | |
| 7 | CAMK1D_AS PRKCB_ Site23920921_L CAMK1D_CE PRKCB_ DJKM MDM2_SV IFNAR1.1_Site33355809 MDM2_ASVX CAMK1D_ES GAB2_DE LYN_F MDM2_Site68821643 IFNAR1.1_Site33355820_ O LYN_AF LYN_Site56012521 MDM2_STVX CAMK1D_BD PRKCB_ Site23920868 IFNAR1.1_ABCE | **C18** | 649 | 0.918 | [ 0.858 ; 0.977 1 | 0.138 | 87.0 | **86.0** | **88.9** | 77.4 | 93.5 | N=77 [50 (1); 27 (2)] |
| 8 | CAMK1D_AS PRKCB_ Site23920921_L CAMK1D_CE PRKCB_ DJKM MDM2_SV IFNAR1.1_Site33355809 MDM2_ASVX | **C18** | 646 | 0.917 | [ 0.857 ; 0.977 1 | 0.169 | 87.0 | **86.0** | **88.9** | 77.4 | 93.5 | N=77 [50 (1); 27 (2)] |

EP 4 055 191 B1

| Num | Combinaison mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| | CAMK1D_ES MDM2_AST GAB2_DE LYN_F IFNAR1.1_Site33355820_O LYN_AF LYN_Site56012521 MDM2_STVX CAMK1 D_BD PRKCB_Site23920868 IFNAR1.1_ABCE | | | | | | | | | | | |
| 9 | PRKCB_Site23920921_L CAMK1D _CE PRKCB_DJKM MDM2_SV IFNAR1.1_Site33355809 CAMK1D_EFS MDM2_ASVX CAMK1D_ES MDM2_AST GAB2_DE LYN_F MDM2_Site68821643 IFNAR1.1_Site33355820_O LYN_AF MDM2_STVX CAMK1 D_BD PRKCB_Site23920868 IFNAR1.1_ABCE | C18 | 1265 | 0.916 | [ 0.855 ; 0.976 ] | 0.145 | 87.0 | **86.0** | **88.9** | 77.4 | 93.5 | N = 77 [50 (1); 27 (2)] |

The data obtained with both methods thus gave excellent diagnostic performances for sex-specific diagnosis of DEP, with AUCs above 0.800, specificities and sensitivities above 80% for both females and males, indicative of a diagnostic assay for differential diagnosis of bipolar disorder that could be used into the clinic.

As we have identified RNA editing biomarkers for the differential diagnosis of BD, we sought to investigate whether the non-edited isoforms of the validated targets could be useful to discriminate the unipolar and bipolar patients populations and might be used in combination with RNA editing biomarkers. The AUC ROC for each non-edited target were then calculated, along with the significance for each non-edited isoform. Analysis of the non-edited isoforms of MDM2 on the validation cohort (n=256) gave an AUC of 0.609, and was significantly different between Unipolar and BD (p<0.05, Table 9).

Table 9: Diagnostic performances obtained with non-edited isoform of MDM2 on the validation cohort analyzed with multiplex sequencing

| Target | p-value | AUC ROC | % Non-edited | Population |
|---|---|---|---|---|
| MDM2 non-edited | 0.0039 | 0.609 | 34.2 | N= 256 [ 160 (0) ; 96 (1)] |

In addition, when the non-edited isoforms of the validated targets were combined with one isoform of the same targets, we observed significant differences between Unipolar and BD patients (Table 10), suggesting that the non-edited isoforms could also be used in the diagnostic assay.

Table 10: Diagnostic performances obtained with non-edited isoforms combined with edited targets on the validation cohort analyzed with multiplex sequencing

| Target | Ratio | p-value | AUC ROC | Population |
|---|---|---|---|---|
| CAMK1D | ABIM / non-edited | 0.0012 | 0.617 | N= 256 [ 160 (0); 96 (1)] |
| CAMK1D | BCDEP / non-edited | 0.0017 | 0.611 | N= 256 [ 160 (0); 96 (1)] |
| CAMK1D | AFGNS / non-edited | 0.0035 | 0.607 | N= 256 [ 160 (0); 96 (1)] |
| CAMK1D | COS / non-edited | 0.0047 | 0.605 | N= 256 [ 160 (0); 96 (1)] |
| GAB2 | ABCEFIJ / non-edited | 0.0003 | 0.636 | N= 256 [ 160 (0); 96 (1)] |
| GAB2 | FHI / non-edited | 0.0008 | 0.622 | N= 256 [ 160 (0); 96 (1)] |
| GAB2 | GHIJK / non-edited | 0.0036 | 0.604 | N= 256 [ 160 (0); 96 (1)] |
| GAB2 | ABGHIJK / non-edited | 0.0042 | 0.602 | N= 256 [ 160 (0) ; 96 (1)] |
| IFNAR1 | BCIJ / non-edited | 0.0021 | 0.607 | N= 256 [ 160 (0); 96 (1)] |
| IFNAR1 | ABCMP / non-edited | 0.0034 | 0.604 | N= 256 [ 160 (0); 96 (1)] |
| IFNAR1 | CR / non-edited | 0.0046 | 0.602 | N= 256 [ 160 (0); 96 (1)] |
| LYN | CDW / non-edited | 0.0012 | 0.625 | N= 256 [ 160 (0); 96 (1)] |
| LYN | CP / non-edited | 0.0045 | 0.605 | N= 256 [ 160 (0); 96 (1)] |
| PRKCB | ADGHJL / non-edited | 0.001 | 0.618 | N= 256 [ 160 (0); 96 (1)] |
| PRKCB | AEJK / non-edited | 0.002 | 0.610 | N= 256 [ 160 (0) ; 96 (1)] |
| PRKCB | DN / non-edited | 0.0025 | 0.611 | N= 256 [ 160 (0) ; 96 (1)] |
| PRKCB | BDFHJK / non-edited | 0.0026 | 0.612 | N= 256 [ 160 (0) ; 96 (1)] |
| PRKCB | DHIJa / non-edited | 0.003 | 0.609 | N= 256 [ 160 (0); 96 (1)] |
| PRKCB | M / non-edited | 0.0041 | 0.610 | N= 256 [ 160 (0); 96 (1)] |
| PRKCB | ADKLM / non-edited | 0.0042 | 0.606 | N= 256 [ 160 (0); 96 (1)] |

Bipolar depression is comprised of fluctuating episodes of both depression and mania. However, patients responding to treatment often experience a euthymic state, which is typically in the middle of a range from depression to mania. Despite the DSM-5 did not define any diagnostic criteria for euthymia[53], it usually refers to a status of clinical remission, often temporary, for mood syndromes such as bipolar disorder. As the panel of biomarkers we identified allow differential diagnosis of BD, we sought to investigate whether those biomarkers could differentiate between depressive bipolar patients and euthymic patients, in order to be able to follow-up patients during treatment and evaluate treatment response. We thus analyzed the diagnostic performances of the identified biomarkers in a population of depressive and euthymic bipolar patients (Figure 22).

Examples of combinations tested on the whole population of depressive bipolar patients and euthymic patients (n=137, see Figure 22) are presented in Table 11. All combinations were statistically significant between control and cases with a p value $<10^{-4}$.

**Table 11:** Diagnostic performances obtained with patients of the whole population of the of depressive bipolar patients and euthymic patients (n=137) analyzed by multiplex sequencing of 8 targets.

| Num | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 1 | GAB2_CE MDM2_T PDE8A_Site85096717_C MDM2_GM GAB2_DHJ PRKCB_ADHIJKM MDM2_ASX MDM2_BG GAB2_BDEJ GAB2_BE IFNAR1.1_Site33355820_O CAMK1D_P GAB2_Site78330955_G MDM2_AX MDM2_OS CAMK1D_BI MDM2_Site68821573 MDM2_Site68821612_A MDM2 _Site68821656 PRKCB_Site23920863 MDM2_Site68821629 MDM2_SX PRKCBDJK MDM2_SVW PRKCB_M GAB2_BDJ IFNAR1.1_Site33355756_K CAMK1D_ABEGS MDM2_SWX PDE8A_Site85096729_F MDM2_Site68821616_X MDM2_SW CAMK1D_Site12378438 PRKCB_Site23920921_L PRKCB_ADGHIJKM IFNAR1.1_Site33355798 LYN_Site56012567 GAB2_BDE | **C38** | **9953** | 0.977 | [ 0.956 ; 0.999 ] | -1.0351 | 95.6 | **95.0** | **95.8** | 97.9 | 90.5 | N= 136 [40 (1); 96 (2)] |

68

| Num | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 2 | CAMK1D_Site12378440 GAB2_CE MDM2_T PDE8A_Site85096717_C MDM2_GM GAB2_DHJ PRKCB_ADHIJKM MDM2_ASX MDM2_BG GAB2_BE IFNAR1.1_Site33355820_O CAMK1D_P GAB2_Site78330955_G MDM2_AX CAMK1D_BI MDM2_Site68821573 MDM2_Site68821612_A MDM2_Site68821656 PRKCB_Site23920863 MDM2_Site68821629 MDM2_SX PRKCB_DJK MDM2_SVW PRKCB_M GAB2_BDJ IFNAR1.1_Site33355756_K CAMK1D_Site12378311 CAMK1D_ABEGS MDM2_SWX PDE8A_Site85096729_F MDM2_Site68821616_X MDM2_SW GAB2_EH CAMK1D_Site12378438 PRKCB_Site23920921_L IFNAR1.1_Site33355798 LYN_Site56012567 GAB2_BDE | C38 | 4799 | 0.980 | [ 0.962 ; 0.997 ] | -0.6748 | 94.1 | 100.0 | 91.7 | 100.0 | 83.3 | N= 136 [40 (1); 96 (2)] |

EP 4 055 191 B1

| Num | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 3 | CAMK1D_Site12378440 GAB2_CE MDM2_T PDE8A_Site85096717_C MDM2_GM GAB2_DHJ PRKCB_ADHIJKM MDM2_ASX MDM2_BG GAB2_BDEJ GAB2_BE IFNAR1.1_Site33355820_O CAMK1D_P GAB2_Site78330955_G MDM2_AX MDM2_OS CAMK1D_BI MDM2_Site68821612_A MDM2_Site68821656 PRKCB_Site23920863 MDM2_Site68821629 PRKCB_DJK | C22 | 6 | 0.961 | [ 0.93 ; 0.992 ] | -0.8052 | 91.9 | 87.5 | 93.8 | 94.7 | 85.4 | N= 136 [40 (1); 96 (2)] |

| Num | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 4 | CAMK1D_Site12378440 GAB2_CE MDM2_T PDE8A_Site85096717_C MDM2_GM GAB2_DHJ PRKCB_ADHIJKM MDM2_ASX MDM2_BG GAB2_BE IFNAR1.1_Site33355820_O CAMK1D_P GAB2_Site78330955_G MDM2_AX MDM2_OS CAMK1D_BI MDM2_Site68821573 MDM2_Site68821612_A MDM2_Site68821656 PRKCB_Site23920863 MDM2_Site68821629 PRKCB_DJK | C22 | 14 | 0.957 | [ 0.924 ; 0.99 ] | -0.8048 | 91.9 | 87.5 | 93.8 | 94.7 | 85.4 | N= 136 [40 (1); 96 (2)] |

EP 4 055 191 B1

71

| Num | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 5 | CAMK1D_Site12378440 GAB2_CE MDM2_T PDE8A_Site85096717_C MDM2_GM GAB2_DHJ PRKCB_ADHIJKM MDM2_ASX MDM2_BG GAB2_BE IFNAR1.1_Site33355820_O CAMK1D_P GAB2_Site78330955_G MDM2_AX CAMK1D_BI MDM2_Site68821612_A MDM2_Site68821656 PRKCB_Site23920863 MDM2_Site68821629 PRKCB_DJK | C20 | 313 | 0.958 | [ 0.926 ; 0.991 ] | -0.7034 | 92.7 | 90.0 | 93.8 | 95.7 | 85.7 | N= 136 [40 (1); 96 (2)] |
| 6 | CAMK1D_Site12378440 GAB2_CE MDM2_T PDE8A_Site85096717_C MDM2_GM GAB2_DHJ PRKCB_ADHIJKM MDM2_BG GAB2_BDEJ GAB2_BE IFNAR1.1_Site33355820_O GAB2_Site78330955_G MDM2_AX CAMK1D_BI MDM2_Site68821573 MDM2_Site68821612_A MDM2_Site68821656 PRKCB_Site23920863 MDM2_Site68821629 PRKCB_DJK | C20 | 508 | 0.960 | [ 0.93 ; 0.991 ] | -0.9456 | 92.7 | 87.5 | 91.7 | 94.6 | 81.4 | N= 136 [40 (1); 96 (2)] |

EP 4 055 191 B1

72

In the female validation cohort (n=90), when only 9 variants included, present in 4 biomarkers, was introduced in the analysis, the AUC was 0.847 (Sp 76.2%; Se 82.6%, Figure 9A). The equation used in this case is the following:

Z= - ( 0.0895727555887869 )* MDM2_SWX - ( 0.284497312947692 )* CAMK1D_Site12378438 - ( 0.0124820173731589 )* MDM2_Site68821612_A - ( 0.420995574249043 )* GAB2_E - ( 0.329608502036281 )* MDM2_ASX + ( 0.362901517502019 )* MDM2_Site68821629 - ( 0.113622915523371 )* MDM2_Site68821573 - ( 0.0962399148413304 )* MDM2_SW- ( 0.148613926406161 )* PRKCB_Site23920927_M

When the number of editing variants was increased to reach 18 variants, present in 5 biomarkers, the performance increased and the AUC was 0.965 (Sp 90.5%; Se 97.7%, Figure 9B). Furthermore, when up to 24 differentially edited sites, all contained in 5 different biomarkers, were combined and analyzed, we obtained an outstanding performance, with an AUC of 0.984, with excellent specificity and sensitivity (90.5% and 98.6%, respectively, Figure 9C)

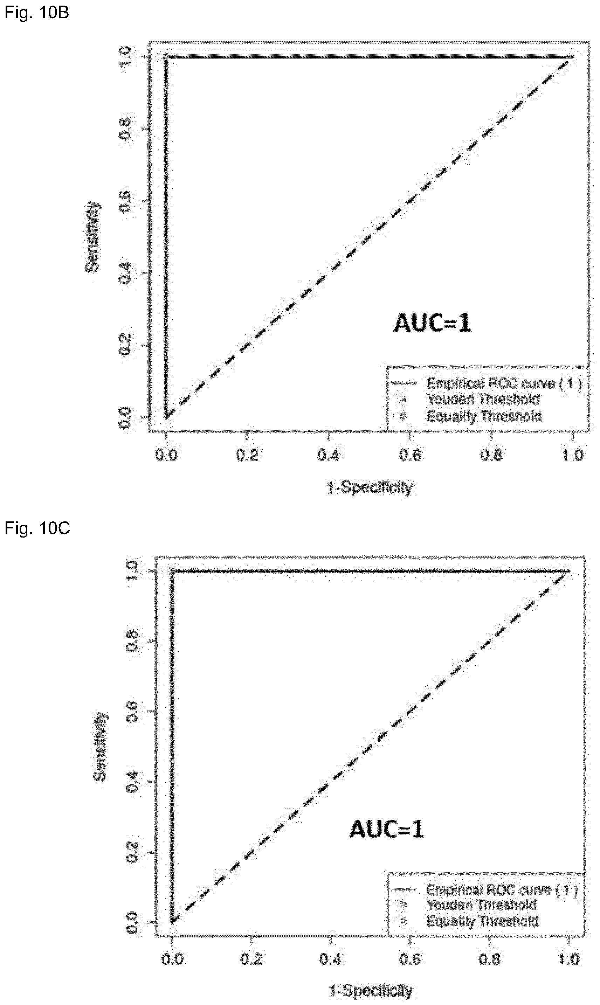

The performances of the male population were even better. Indeed, In the male validation cohort of depressed BD and euthymics, (n=47), when only 12 variants included, present in 6 different biomarkers, we obtained an AUC of 0.994 (Sp 95%, Se 100%, Figure 10A). When the number of editing variants was slightly increased to 23, included in 6 different biomarkers, the AUC rose to 1.00, with both specificity and sensitivity of 100% (Figure 10B). Including 28 editing variants in those 6 biomarkers gave the same performance: the AUC was 1.00, with 100% specificity and 100% Sensitivity (Figure 10C). In this case, the equation used was the following:

Z= - ( 1.52336682837312 )* IFNAR1.1_ABCF - ( 3.26057883489785 )* CAMK1D_R - ( 0.763880452664734 )* LYN_Site56012450 - ( -0.561253833023493 )* CAMK1D_RS - ( -3.33767767845627 )* GAB2_BDEHJK + ( 1.55430607429165 )* GAB2_Site78330955_G - ( 4.52292549457016 )* CAMK1D_ABEG - ( - 1.07661353764004 )* GAB2_CE - ( -3.45753212466969 )* CAMK1D_Site12378438 + ( 1.32699847321958 )* PRKCB_IJK - ( - 1.2518372990998 )* GAB2_BE - ( 1.79465029376655 )* CAMK1D_BF - ( 1.38897817068785 )* IFNAR1.1_AB - ( 2.00128623265317 )* PRKCB_M - ( -0.237584778376622 )* MDM2_A - ( 0.989889267419364 )* CAMK1D_GS - ( 0.328234691570068 )* IFNAR1.1_AC + ( 1.82350692467403 )* MDM2_T + ( 0.69901718503897 )* IFNAR1.1_Site33355820_O + ( 0.145098039959207 )* PRKCB_Site23920874 + ( 2.27039659579243 )* PRKCB_Site23920910_J - ( -0.815680192672586 )* GAB2_Site78331030 + ( - 0.287785099217686 )* PRKCB_Site23920919_K - ( 0.576710638047866 )* MDM2_SVW + ( 1.14884163937465 )* PRKCB_ADGHIJKM - ( 0.251734426707645 )* GAB2_EH + ( -2.18488230071751 )* PRKCB_Site23920921_L + ( 1.19656964307143 )* PRKCB_Z.

Altogether, these data suggest that the different biomarkers in the panel of 8 genes selected in the validation cohorts could also be used for differentiate depressive Bipolar Disorder patients from euthymic patients, suggesting that those biomarkers could also be used for treatment follow-up.

As the panel of biomarkers analyzed above thus gave excellent diagnostic performances to differentially diagnose BD and Uni in depressed patients, we sought to investigate whether those biomarkers could allow discrimination of BD from healthy controls (Table 12). We then investigated whether the RNA editing biomarkers validated for differential diagnosis of BD were differentially edited in a healthy control population as well. Age-, race- and sex-matched control subjects (n=143) were recruited from a list of volunteers from the Clinical Investigation Center (CHRU of Montpellier). RNA editing biomarkers were measured as described above and compared to the population of Bipolar Disorder depressed patients (n=96). In the whole population, when 60 editing biomarkers were combined from 7 different biomarkers, all of which were sequenced in multiplex in a single experiment, the AUC ROC was 0.899 (Sp 88.1%, Se 88.5%, Figure 11A). Examples of combinations tested on the whole population of depressive bipolar patients and healthy controls (n=239, see Figure 22) are presented in Table 12. All combinations were statistically significant between control and cases with a p value $<10^{-4}$.

**Table 12:** Diagnostic performances obtained with patients of the whole population of the of depressive bipolar patients and healthy controls (n=137) analyzed by multiplex sequencing of 8 targets.

| Num | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 1 | PRKCB_Site23920910_J PRKCB_Site23920921_L IFNAR1.1_Site33355809 PRKCB_DIJK IFNAR1.1_Site33355798 PRKCB_Site23920911 PRKCB_Site23920919_K MDM2_W IFNAR1.1_Site33355838_P IFNAR1.1_Site33355817 PRKCB_B PRKCB_Site23920844_B MDM2_SWX PRKCB_JK GAB2_CE PRKCB_M MDM2_STX PRKCB_Site23920893_G IFNAR1.1_Site33355840_E PRKCB_Site23920889_F IFNAR1.1_Site33355829_I CAMK1D_E CAMK1D_AE IFNAR1.1_Site33355839_L IFNAR1.1_Site33355793_G IFNAR1.1_Site33355789_A IFNAR1.1_Site33355801_N PRKCB_J KCNJ15_C IFNAR1.1_Site33355807_B PRKCB_DHIJK IFNAR1.1_Site33355830_C MDM2_Site68821586_W GAB2_ABDE GAB2_Site78331030 GAB2_DE LYN_Site56012490 PRKCB_Site23920884_D | C60 | 3755 | 0.899 | [ 0.858 ; 0.941 ] | 0.2352 | 88.3 | 88.1 | 88.5 | 83.3 | 92.0 | N= 239 [ 143 (1); 96 (2)] |

| Num | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
|  | IFNAR1.1_Site33355762_F GAB2_DEJ PRKCB_AD CAMK1D_Site12378346_E CAMK1D_BS CAMK1D_G PRKCB_Site23920902_I CAMK1D_A IFNAR1.1_Site33355763_H GAB2_BC KCNJ15_CD LYN_A GAB2_EJ LYN_ Site56012450 PRKCB_DIJ PRKCB_Site23920915 IFNAR1.1_ABCG CAMK1D_ES PRKCB_Site23920897 CAMK1D _Site12378351 MDM2_Site68821563 GAB2_BH |  |  |  |  |  |  |  |  |  |  |  |
| 2 | PRKCB_Site23920910_J PRKCB_Site23920921_L IFNAR1.1_Site33355809 PRKCB_DIJK IFNAR1.1_Site33355798 PRKCB_Site23920911 PRKCB_Site23920919_K MDM2_W IFNAR1.1_Site33355838_P IFNAR1.1_Site33355817 PRKCB_B PRKCB_ Site23920844_B MDM2_SWX PRKCB _JK GAB2_CE PRKCB_ M MDM2_STX PRKCB_Site23920893_G IFNAR1.1_Site33355840_E PRKCB_Site23920889_F IFNAR1.1_Site33355829_I |  |  |  |  |  |  |  |  |  |  |  |

EP 4 055 191 B1

| Num | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| | CAMK1D_E CAMK1D_AE IFNAR1.1_Site33355839_L IFNAR1.1_Site33355793_G IFNAR1.1_Site33355789_A IFNAR1.1_Site33355801_N PRKCB_J KCNJ15_C IFNAR1.1_Site33355807_B PRKCB_DHIJK IFNAR1.1_Site33355830_C MDM2_Site68821586_W GAB2_ABDE GAB2_Site78331030 GAB2_DE PRKCB_Site23920884_D IFNAR1.1_Site33355762_F GAB2_DEJ PRKCB_AD CAMK1D_Site12378346_E CAMK1D_BS CAMK1D_G PRKCB_Site23920902_I CAMK1D_A IFNAR1.1_Site33355763_H LYN_AG GAB2_BC KCNJ15_CD LYN_A GAB2_EJ GAB2_Site78331054 LYN_ Site56012450 PRKCB_DIJ PRKCB_Site23920915 IFNAR1.1_ABCG CAMK1D_ES PRKCB_Site23920897 MDM2_Site68821563 GAB2_BH | **C60** | 3957 | 0.906 | [ 0.867 ; 0.946 ] | 0.1692 | 87.9 | **86.7** | **89.6** | 81.9 | 92.5 | N= 239 [ 143 (1); 96 (2)] |

EP 4 055 191 B1

| Num | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 3 | PRKCB_Site23920910_J PRKCB_Site23920921_L IFNAR1.1_Site33355809 PRKCB_DIJK IFNAR1.1_Site33355798 PRKCB_Site23920911 PRKCB_Site23920919_K MDM2_W IFNAR1.1_Site33355838_P IFNAR1.1_Site33355817 PRKCB_Site23920844_B MDM2_SWX PRKCB_JK GAB2_CE MDM2_STX PRKCB_Site23920893_G IFNAR1.1_Site33355840_E PRKCB_Site23920889_F IFNAR1.1_Site33355829_I CAMK1D_E CAMK1D_AE IFNAR1.1_Site33355839_L IFNAR1.1_Site33355793_G IFNAR1.1_Site33355801_N PRKCB_J KCNJ15_C IFNAR1.1_Site33355807_B PRKCB_DHIJK IFNAR1.1_Site33355830_C MDM2_Site68821586_W PDE8A_Site85096729_F GAB2_ABDE GAB2_Site78331030 PRKCB_Site23920884_D IFNAR1.1_Site33355762_F IFNAR1.1_Site33355763_H | C38 | 3543 | 0.825 | [ 0.77 ; 0.881 ] | 0.1371 | 80.8 | **81.1** | **80.2** | 74.0 | 85.9 | N= 239 [ 143 (1); 96 (2)] |

EP 4 055 191 B1

(continued)

| Num | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| **4** | PRKCB_Site23920910_J<br>PRKCB_Site23920921_L<br>PRKCB_DIJK<br>IFNAR1.1_Site33355798<br>PRKCB_Site23920911 PRKCB_Site23920919_K<br>MDM2_W<br>IFNAR1.1_Site33355838_P<br>IFNAR1.1_Site33355817<br>PRKCB_Site23920844_B<br>MDM2_SWX PRKCB_JK<br>GAB2_CE PRKCB_M<br>MDM2_STX PRKCB_Site23920893_G<br>IFNAR1,1_Site33355840_E<br>PRKCB_Site23920889_F<br>IFNAR1.1_Site33355829_I CAMK1D_E<br>IFNAR1.1_Site33355839_L<br>IFNAR1.1_Site33355793_G<br>IFNAR1.1_Site33355789_A<br>IFNAR1.1_Site33355801_N<br>PRKCB_J KCNJ15_C<br>IFNAR1.1_Site33355807_B<br>PRKCB_DHIJK<br>IFNAR1.1_Site33355830_C<br>MDM2_Site68821586_W<br>PDE8A_Site85096729_F<br>GAB2_ABDE<br>GAB2_Site78331030<br>PRKCB_Site23920884_D<br>IFNAR1.1_Site33355762_F<br>IFNAR1.1 Site33355763 H | **C38** | **7535** | 0.825 | [0.77; 0.88] | 0.1556 | 80.8 | **81.8** | **79.2** | 74.5 | 85.4 | N= 239 [ 143 (1); 96 (2)] |

(continued)

| Num | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 5 | PRKCB_Site23920910_J PRKCB_Site23920921_L PRKCB_Site23920911 PRKCB_ Site23920919_K MDM2_W IFNAR1.1_Site33355838_P PRKCB_Site23920844_B MDM2_SWX PRKCB_JK PRKCB_Site23920893_G IFNAR1.1_Site33355840_E PRKCB_Site23920889_F IFNAR1.1_Site33355829_I CAMK1D_AE IFNAR1.1Site33355839L __ IFNAR1.1_Site33355793_G IFNAR1.1_Site33355789_A IFNAR1.1_Site33355801_N IFNAR1.1_Site33355807_B IFNAR1.1_Site33355830_C MDM2_Site68821586_W PRKCB_Site23920884_D IFNAR1.1_Site33355762_F IFNAR1.1 Site33355763 H | C24 | 314 | 0.778 | [ 0.718 ; 0.838 ] | 0.0533 | 74.9 | 74.1 | 76.0 | 66.4 | 82.2 | N= 239 [ 143 (1); 96 (2)] |

| Num | Combination mROC | Combi | ID | AUC ROC | CI 95% | Threshold | Acc (%) | Sp (%) | Se (%) | VPP (%) | VPN (%) | Population |
|---|---|---|---|---|---|---|---|---|---|---|---|---|
| 6 | PRKCB_Site23920910_J<br>PRKCB_Site23920921_L<br>PRKCB_Site23920911 PRKCB_Site23920919_K<br>MDM2_W<br>IFNAR1.1_Site33355838_P<br>MDM2_SWX<br>PRKCB_JK PRKCB_Site23920893_G<br>IFNAR1,1_Site33355840_E<br>PRKCB_Site23920889_F<br>IFNAR1.1_Site33355829_I<br>CAMK1D_AE<br>IFNAR1.1_Site33355839_L<br>IFNAR1.1_Site33355793_G<br>IFNAR1.1_Site33355789_A<br>IFNAR1.1_Site33355801_N<br>KCNJ15_C<br>IFNAR1.1_Site33355807_B<br>IFNAR1,1_Site33355830_C<br>MDM2_Site68821586_W<br>PRKCB_Site23920884_D<br>IFNAR1.1_Site33355762_F<br>IFNAR1.1_Site33355763_H | C24 | 1226 | 0.777 | [ 0.717 ; 0.837] | 0.0631 | 74.9 | 74.8 | 75.0 | 66.7 | 81.7 | N= 239 [ 143 (1); 96 (2)] |

EP 4 055 191 B1

80

When we introduced sex specificity in the analysis, and combined 32 editing biomarkers from the same 7 biomarkers in the women population (n=159, of which 90 were healthy controls and 69 BD patients), the AUC rose to 0.908 (Sp 84.4%, Se 92.8%, Figure 11B). Similarly, when 50 editing biomarkers from the same 7 biomarkers were combined and analyzed in the male population (n=80, of which 53 were healthy controls and 27 BD patients), we perfectly separated healthy controls and BD, with an AUC of 1 (Sp 100%, Se 100%, Figure 11C).

When the same analysis was run between healthy controls (n=143) and bipolar euthymic patients (n=41), with 53 editing biomarkers combined from 6 different biomarkers, the AUC ROC was 0.972 (Sp 95.8%, Se 95.0%, Figure 12A)

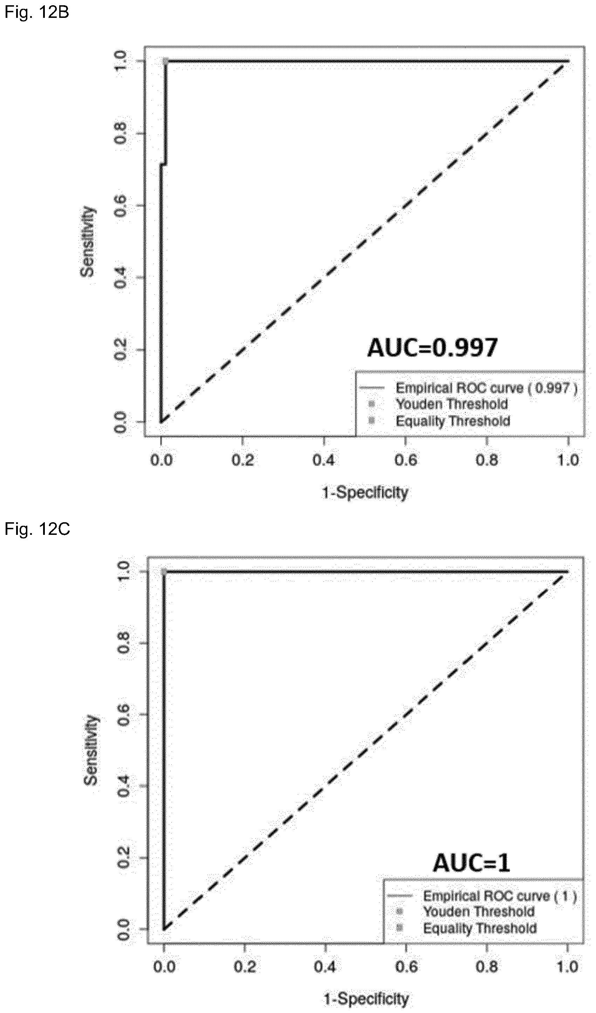

When we introduced sex specificity in the analysis, and combined 41 editing biomarkers from the 8 biomarkers in the women population (n=121, of which 90 were healthy controls and 21 Bipolar euthymic), the AUC rose to 0.997 (Sp 98.9%, Se 100%, Figure 12B). Similarly, when 35 editing biomarkers from 7 biomarkers were combined and analyzed in the male population (n=73, of which 53 were healthy controls and 20 Bipolar euthymic patients), we also perfectly separated healthy controls and BD, with an AUC of 1 (Sp 100%, Se 100%, Figure 12C).

Altogether, these data suggest that the different biomarkers in the panel of 8 genes selected in the validation cohorts could be used for differentiate depressive Bipolar Disorder patients or Bipolar euthymic patients from healthy controls with excellent sensitivity and specificity.

Bipolar depression is comprised of fluctuating episodes of both depression and mania. There are two main types of bipolar disorders: bipolar I and bipolar II, which according to the Diagnostic and Statistical Manual of Mental Disorders, 5th edition (DSM5) differ mostly by the severity of maniac episodes and experience of a major depressive episode[53]. Therefore, we sought to investigate whether the selected biomarkers described above could be used to discriminate between the two subtype of Bipolar Depression. We thus analyzed the diagnostic performances of the identified biomarkers in the population of bipolar patients, which have been classified by trained psychiatrists as either type I or type II BD (Figure 24). When we analyzed the editing sites of the whole population (n=140, of which 58 were type I BD and 82 type II BD) and combined 28 editing variants in 6 BMKs, we obtained an AUC of 0.898 (Sp 86.2%; Se 85.4%, Figure 13A).

Examples of combinations tested on the whole population of type I and type II bipolar patients (n=140) is presented in Table 13. All combinations were statistically significant between control and cases with a p value $<10^{-4}$.

**Table 13:** Diagnostic performances obtained with patients of the whole population type I and type II bipolar patients (n=140) analyzed by multiplex sequencing of 8 targets.

| Nu m | Combinaison mROC | Comb i | ID | AUC ROC | CI 95% | Threshol d | Acc (%) | Sp (%) | Se (%) | VPP (%) | VP N (%) | Population |
|---|---|---|---|---|---|---|---|---|---|---|---|---|
| 1 | CAMK1D_AGS CAMK1D_ AG CAMK1D _ABEG CAMK1D_Site12378375_G LYN_S KCNJ15_AF LYN_M GAB2_BE GAB2_Site78331030 PRKCB_DHIJKM CAMK1D_BF CAMK1D_AE LYN_C CAMK1D_Site12378316_ B PRKCB_K CAMK1D_EG MDM2_Site68821643 MDM2_SVWX CAMK1D_Site12378436 GAB2_DEJ PRKCB_A KCNJ15_AE LYN_ Site56012555 CAMK1D_ ABEGS CAMK1D_Site12378385_A MDM2_Site68821616_X GAB2_DHJ GAB2_DE | C29 | 8 | 0.898 | [ 0.844 ; 0.952 ] | -0.067 | 85.7 | 87.9 | 84.2 | 90.8 | 79.7 | N= 140 [58 (1); 82 (2)] |

EP 4 055 191 B1

(continued)

| Nu m | Combinaison mROC | Comb i | ID | AUC ROC | CI 95% | Threshol d | Acc (%) | Sp (%) | Se (%) | VPP (%) | VP N (%) | Population |
|---|---|---|---|---|---|---|---|---|---|---|---|---|
| 2 | CAMK1D_AGS CAMK1D_AG CAMK1D_ABEG CAMK1D_Site12378375_GLYN_S KCNJ15_AF LYN_M GAB2_BE GAB2_Site78331030 PRKCB_DHIJKM CAMK1D_BF CAMK1D_AE LYN_C CAMK1D_Site12378316_B PRKCB_K CAMK1D_EG MDM2_Site68821643 MDM2_SVWX CAMK1D_Site12378436 PRKCB_A KCNJ15_AE LYN_Site56012555 CAMK1D_ABEGS CAMK1D_Site12378385_A LYN_Site56012468_C MDM2_Site68821616_X GAB2_DHJ GAB2_DE | C29 | 47 | 0.898 | [ 0.845 ; 0.952 ] | -0.058 | 85.7 | 86.2 | 85.4 | 89.7 | 80.7 | N= 140 [58 (1); 82 (2)] |

(continued)

| Num | Combinaison mROC | Comb i | ID | AUC ROC | CI 95% | Threshold d | Acc (%) | Sp (%) | Se (%) | VPP (%) | VP N (%) | Population |
|---|---|---|---|---|---|---|---|---|---|---|---|---|
| 3 | CAMK1D_AG CAMK1D_ABEG CAMK1D_Site12378375_G LYN_S KCNJ15_AF LYN_M GAB2_BE GAB2_Site78331030 PRKCB_DHIJKM CAMK1D_BF CAMK1D_AE LYN_C CAMK1D_Site12378316_B PRKCB_KCAMK1D_EG MDM2_Site68821643 MDM2_SVWX CAMK1D_Site12378436 GAB2_DEJ PRKCB_A KCNJ15_AE LYN_ Site56012555 CAMK1D_ ABEGS CAMK1D_Site12378385_A LYN_Site56012468_C MDM2_Site6882161_X GAB2_DHJ GAB2_DE | C29 | 40 8 | 0.897 | [ 0.843 ; 0.951 ] | -0.038 | 85.7 | 87.9 | 84.2 | 90.8 | 79.7 | N= 140 [58 (1); 82 (2)] |

When we introduced sex specificity in the analysis, and combined 49 editing biomarkers from the 8 biomarkers of the panel in the women population (n=88, of which 36 were type I BD and 52 type II BD), the AUC rose to 0.980 (Sp 97.2%, Se 94.2%, Figure 13B). Similarly, when 48 editing biomarkers from the same 8 biomarkers were combined and analyzed in the male population (n=52, of which 22 were type I BD and 30 type II BD), we perfectly separated healthy controls and BD, with an AUC of 1 (Sp 100%, Se 100%, Figure 13C).

Altogether, these data suggest that the different biomarkers in the panel of 8 genes selected in the validation cohorts could be used for differentiate the two subtypes of Bipolar Disorder with excellent sensitivity and specificity.

**References**

1. Grande I, Berk M, Birmaher B, Vieta E. Bipolar disorder. Lancet. Apr 9 2016;387(10027):1561-1572.

2. Sharma V, Khan M, Smith A. A closer look at treatment resistant depression: is it due to a bipolar diathesis? Journal of affective disorders. Feb 2005;84(2-3):251-257.

3. Grotegerd D, Suslow T, Bauer J, et al. Discriminating unipolar and bipolar depression by means of fMRI and pattern classification: a pilot study. European archives of psychiatry and clinical neuroscience. Mar 2013;263(2):119-131.

4. Sagar R, Pattanayak RD. Potential biomarkers for bipolar disorder: Where do we stand? The Indian journal of medical research. Jan 2017;145(1):7-16.

5. Tasic L, Larcerda ALT, Pontes JGM, et al. Peripheral biomarkers allow differential diagnosis between schizophrenia and bipolar disorder. Journal of psychiatric research. Sep 23 2019;119:67-75.

6. Gatsiou A, Vlachogiannis N, Lunella FF, Sachse M, Stellos K. Adenosine-to-Inosine RNA Editing in Health and Disease. Antioxidants & redox signaling. Sep 20 2018;29(9):846-863.

7. Dobin A, Gingeras TR. Optimizing RNA-Seq Mapping with STAR. Methods in molecular biology. 2016;1415:245-262.

8. DePristo MA, Banks E, Poplin R, et al. A framework for variation discovery and genotyping using next-generation DNA sequencing data. Nature genetics. May 2011;43(5):491-498.

9. John D, Weirick T, Dimmeler S, Uchida S. RNAEditor: easy detection of RNA editing events and the introduction of editing islands. Briefings in bioinformatics. Nov 1 2017;18(6):993-1001.

10. Yang H, Wang K. Genomic variant annotation and prioritization with ANNOVAR and wANNOVAR. Nature protocols. Oct 2015;10(10):1556-1566.

11. Tempel S. Using and understanding RepeatMasker. Methods in molecular biology. 2012;859:29-51.

12. Kasprzyk A, Keefe D, Smedley D, et al. EnsMart: a generic system for fast and flexible access to biological data. Genome research. Jan 2004;14(1):160-169.

13. Langmead B, Salzberg SL. Fast gapped-read alignment with Bowtie 2. Nature methods. Mar 4 2012;9(4):357-359.

14. Schneider VA, Graves-Lindsay T, Howe K, et al. Evaluation of GRCh38 and de novo haploid genome assemblies demonstrates the enduring quality of the reference assembly. Genome research. May 2017;27(5):849-864.

15. Li H, Handsaker B, Wysoker A, et al. The Sequence Alignment/Map format and SAMtools. Bioinformatics. Aug 15 2009;25(16):2078-2079.

16. Li H. A statistical framework for SNP calling, mutation discovery, association mapping and population genetical parameter estimation from sequencing data. Bioinformatics. Nov 1 2011;27(21):2987-2993.

17. Gentleman RC, Carey VJ, Bates DM, et al. Bioconductor: open software development for computational biology and bioinformatics. Genome biology. 2004;5(10):R80.

18. Linden A. Measuring diagnostic and predictive accuracy in disease management: an introduction to receiver operating characteristic (ROC) analysis. Journal of evaluation in clinical practice. Apr 2006;12(2):132-139.

19. Kramar A, Faraggi D, Fortune A, Reiser B. mROC: a computer program for combining tumour markers in predicting disease states. Computer methods and programs in biomedicine. Sep 2001;66(2-3):199-207.

20. Breiman L. Random Forests. Machine Learning. Kluwer Academic Publishers. 2001;45:5-32.

21. Su JQ, Liu JS. Linear Combinations of Multiple Diagnostic Markers. Journal of the American Statistical Association. 1993;88(424):1350-1355.

22. Wang H. A note on iterative marginal optimization: a simple algorithm for maximum rank correlation estimation. Computational Statistics and Data Analysis. 2007;51:2803-2812.

23. Staack A, Badendieck S, Schnorr D, Loening SA, Jung K. Combined determination of plasma MMP2, MMP9, and TIMP1 improves the non-invasive detection of transitional cell carcinoma of the bladder. BMC urology. Aug 10 2006;6:19.

24. Blagus R, Lusa L. Class prediction for high-dimensional class-imbalanced data. BMC bioinformatics. Oct 20 2010;11:523.

25. Picardi E, Manzari C, Mastropasqua F, Aiello I, D'Erchia AM, Pesole G. Profiling RNA editing in human tissues: towards the inosinome Atlas. Scientific reports. Oct 9 2016;5:14941.

26. Bazak L, Levanon EY, Eisenberg E. Genome-wide analysis of Alu editability. Nucleic acids research. Jun 2014;42(11):6876-6884.

27. Consortium GO. Gene Ontology Consortium: going forward. Nucleic acids research. Jan 2015;43(Database issue):D1049-1056.

28. Raudvere U, Kolberg L, Kuzmin I, et al. g:Profiler: a web server for functional enrichment analysis and conversions of gene lists (2019 update). Nucleic acids research. Jul 2 2019;47(W1):W191-W198.

29. Pomaznoy M, Ha B, Peters B. GOnet: a tool for interactive Gene Ontology analysis. BMC bioinformatics. Dec 7 2018;19(1):470.

30. Chimienti F, Cavarec L, Vincent L, et al. Brain region-specific alterations of RNA editing in PDE8A mRNA in suicide decedents. Translational psychiatry. Feb 15 2019;9(1):91.

31. Salvetat N, Van der Laan S, Vire B, et al. RNA editing blood biomarkers for predicting mood alterations in HCV patients. Journal of neurovirology. Jul 22 2019.

32. Szklarczyk D, Gable AL, Lyon D, et al. STRING v11: protein-protein association networks with increased coverage, supporting functional discovery in genome-wide experimental datasets. Nucleic acids research. Jan 8 2019;47(D1):D607-D613.

33. Khandelwal N, Dey SK, Chakravarty S, Kumar A. miR-30 Family miRNAs Mediate the Effect of Chronic Social Defeat Stress on Hippocampal Neurogenesis in Mouse Depression Model. Frontiers in molecular neuroscience. 2019;12:188.

34. Shelton RC. The molecular neurobiology of depression. The Psychiatric clinics of North America. Mar 2007;30(1):1-11.

35. Costas J, Gratacos M, Escaramis G, et al. Association study of 44 candidate genes with depressive and anxiety symptoms in post-partum women. Journal of psychiatric research. Aug 2010;44(11):717-724.

36. Colledge M, Snyder EM, Crozier RA, et al. Ubiquitination regulates PSD-95 degradation and AMPA receptor surface expression. Neuron. Oct 30 2003;40(3):595-607.

37. Liu B, Mink S, Wong KA, et al. PIAS1 selectively inhibits interferon-inducible genes and is important in innate immunity. Nature immunology. Sep 2004;5(9):891-898.

38. Murakami Y, Ishibashi T, Tomita E, et al. Depressive symptoms as a side effect of Interferon-alpha therapy induced by induction of indoleamine 2,3-dioxygenase 1. Scientific reports. Jul 20 2016;6:29920.

39. Brodie EJ, Infantino S, Low MSY, Tarlinton DM. Lyn, Lupus, and (B) Lymphocytes, a Lesson on the Critical Balance of Kinase Signaling in Immunity. Frontiers in immunology. 2018;9:401.

40. Hayashi T, Umemori H, Mishina M, Yamamoto T. The AMPA receptor interacts with and signals through the protein tyrosine kinase Lyn. Nature. Jan 7 1999;397(6714):72-76.

41. Moura-Alves P, Fae K, Houthuys E, et al. AhR sensing of bacterial pigments regulates antibacterial defence. Nature. Aug 28 2014;512(7515):387-392.

42. Song MS, Song SJ, Kim SY, Oh HJ, Lim DS. The tumour suppressor RASSF1A promotes MDM2 self-ubiquitination by disrupting the MDM2-DAXX-HAUSP complex. The EMBO journal. Jul 9 2008;27(13):1863-1874.

43. Aleksandrova LR, Phillips AG, Wang YT. Antidepressant effects of ketamine and the roles of AMPA glutamate receptors and other mechanisms beyond NMDA receptor antagonism. Journal of psychiatry & neuroscience : JPN. Jun 2017;42(4):222-229.

44. Verploegen S, Lammers JW, Koenderman L, Coffer PJ. Identification and characterization of CKLiK, a novel granulocyte Ca(++)/calmodulin-dependent kinase. Blood. Nov 1 2000;96(9):3215-3223.

45. Lin E, Kuo PH, Liu YL, Yu YW, Yang AC, Tsai SJ. A Deep Learning Approach for Predicting Antidepressant Response in Major Depression Using Clinical and Genetic Biomarkers. Frontiers in psychiatry. 2018;9:290.

46. Zhou X, Chen Y, Mok KY, et al. Identification of genetic risk factors in the Chinese population implicates a role of immune system in Alzheimer's disease pathogenesis. Proceedings of the National Academy of Sciences of the United States of America. Feb 20 2018;115(8):1697-1706.

47. Beurel E, Lowell JA. Th17 cells in depression. Brain, behavior, and immunity. Mar 2018;69:28-34.

48. Fischer EH, Charbonneau H, Tonks NK. Protein tyrosine phosphatases: a diverse family of intracellular and transmembrane enzymes. Science. Jul 26 1991;253(5018):401-406.

49. Irie-Sasaki J, Sasaki T, Matsumoto W, et al. CD45 is a JAK phosphatase and negatively regulates cytokine receptor signalling. Nature. Jan 18 2001;409(6818):349-354.

50. Basterzi AD, Yazici K, Buturak V, et al. Effects of venlafaxine and fluoxetine on lymphocyte subsets in patients with major depressive disorder: a flow cytometric analysis. Progress in neuro-psychopharmacology & biological psychiatry. Feb 1 2010;34(1):70-75.

51. Aston C, Jiang L, Sokolov BP. Transcriptional profiling reveals evidence for signaling and oligodendroglial abnormalities in the temporal cortex from patients with major depressive disorder. Molecular psychiatry. Mar 2005;10(3):309-322.

52. Seney ML, Huo Z, Cahill K, et al. Opposite Molecular Signatures of Depression in Men and Women. Biological psychiatry. Jul 1 2018;84(1):18-27.

53. American Psychiatric Association. Diagnostic and Statistical Manual of Mental Disorders. 5th ed. Washington, DC. 2013.

**Claims**

1. An in vitro method for differential diagnosing bipolar disorder and unipolar depression in a human patient from a biological sample of said patient, said method comprising:

    a) determining for a combination of at least two A to I editing RNA:

       - for each selected biomarker, the relative proportion of RNA editing at a given editing site for at least one or a combination of sites which can be edited on the RNA transcript of said at least two biomarkers, and/or
       - the relative percentage of an isoform or of a combination of isoforms of the RNA transcript of each of said two biomarkers;
       wherein said at least two A to I editing RNA biomarkers are selected from the group consisting of MDM2, PRKCB, PIAS1, IFNAR1, LYN, AHR, RASSF1, GAB2, CAMK1D, IL17RA, PTPRC, KCNJ15, IFNAR2 and PDE8A biomarkers;

    b) determining a result value obtained for each of said at least two biomarkers and a final result value based on an algorithm or equation that includes the result value obtained for each selected biomarker;
    c) determining whether said result value obtained in step b) is greater or not greater than a control value obtained for control unipolar subject, wherein the control value was determined in a manner comparable to that of the result value; and
    d) if said result value obtained for the patient is greater than a threshold, classifying said patient as having bipolar disorder or, if said result value is not greater than said threshold, classifying said patient having unipolar depression.

2. The in vitro method for differential diagnosing bipolar disorder and unipolar depression in a human patient according to claim 1, wherein in step b), said at least two A to I editing RNA biomarkers are selected from the group consisting of MDM2, PRKCB, IFNAR1, LYN, GAB2, CAMK1D, KCNJ15 and PDE8A.

3. The in vitro method for differential diagnosing bipolar disorder and unipolar depression in a human patient according to one of claims 1 and 2 wherein in step b),

    the result value or final result value, is calculated by an algorithm implementing a multivariate method including:

       - mROC program, particularly to identify the linear combination, which maximizes the AUC (Area Under the Curve) ROC and wherein the equation for the respective combination is provided and can be used as a new virtual marker Z, as follows:

$$Z = a.(\text{Biomarker 1}) + b.(\text{Biomarker 2}) + \ldots i.(\text{Biomarker i}) + \ldots n.(\text{Biomarker n})$$

    where i are calculated coefficients and (Biomarker i) are the level of the considered biomarker (i.e. level of RNA editing site or of isoforms for a given target/biomarker); and/or

       - a Random Forest (RF) approach applied to assess the RNA editing site(s) and/or isoforms combinations, particularly to rank the importance of the RNA editing site(s) and/or isoform(s), and to combine the best RNA editing site(s) and/or isoform(s), and/or optionally
       - a multivariate analysis applied to assess the RNA editing site(s) and/or isoforms combinations for the diagnostic, said multivariate analysis being selecting for example from the group consisting of:
       - Logistic regression model applied for univariate and multivariate analysis to estimate the relative risk of patient at different level of RNA editing site or isoforms values;
       - CART (Classification And Regression Trees) approach applied to assess RNA editing site(s) and/or isoforms combinations; and/or
       - Support Vector Machine (SVM) approach;
       - Artificial Neural Network (ANN) approach;
       - Bayesian network approach;
       - WKNN (weighted k-nearest neighbours) approach;
       - Partial Least Square - Discriminant Analysis (PLS-DA);
       - Linear and Quadratic Discriminant Analysis (LDA / QDA), and

- Any other mathematical method that combines biomarkers

4. The method of one of claims 1 to 3, wherein said unipolar depression disorder is mainly major depressive episode (DEP).

5. The method of one of claims 1 to 4, wherein said biological sample is whole blood, serum, plasma, urine, cerebrospinal fluid or saliva, preferred is the whole blood sample.

6. The method of one of claims 1 to 5, wherein for each selected biomarker, said result value is statistically/specifically different from said control result value at $p < 0.05$.

7. The method of one of claims 1 to 6, wherein the following criteria has to be satisfied for the biomarker or the combination of biomarkers selected tin step a):

   - the coverage >30;
   - AUC>0.8;
   - 0.95 >FoldChange >1.05, and
   - $p < 0.05$.

8. The method of one of claims 1 to 7, wherein said selected A to I editing RNA biomarker(s) comprised in step a) is selected from the group consisting of:
   a combination comprising at least 3, 4, 5, 6 or 7 of the following biomarkers: MDM2, PRKCB, IFNAR1, LYN, GAB2, CAMK1D, KCNJ15 and PDE8A, preferably the combination of the cited 8 biomarkers.

9. The method of one of claims 1 to 8, wherein said selected A to I editing RNA biomarker(s) comprised in step a) the combination of the following 5 biomarkers: GAB2, IFNAR1, KCNJ15, MDM2 and PRKCB.

10. The method of one of claims 1 to 9, wherein said selected A to I editing RNA biomarker(s) comprised in step a) the combination of the following 5 biomarkers: GAB2, IFNAR1, KCNJ15, MDM2 and PRKCB and at least another biomarker selected from the group consisting the following biomarkers: CAMK1D and LYN, preferably the combination of the following 6 biomarkers: GAB2, IFNAR1, KCNJ15, MDM2, PRKCB and LYN when the model is adjusted by age, sex, psychiatric treatment and addiction, or preferably the following 6 biomarkers: GAB2, IFNAR1, KCNJ15, MDM2, PRKCB and CAMK1D when the model is adjusted by age and sex.

11. The method of one of claims 1 to 10, wherein said one or combination of at least 2, 3, 4, 5, 6 or 7 selected biomarkers comprises the calculation of the relative percentage of at least one of the RNA edition site or isoform listed in the Table 2A (edition sites), 2B (isoforms) or 11 (edition sites) for each of the selected biomarker.

12. The method of one of claims 1 to 11, wherein said combination of at least 2, 3, 4, 5, 6, 7 or 8 selected biomarkers is selected from the biomarkers combinations given in Table 12, and 13, preferably the combinations comprising the following 8 biomarkers MDM2, PRKCB, IFNAR1, LYN, GAB2, CAMK1D, KCNJ15 and PDE8A.

13. The method of one of claims 1 to 12, wherein the algorithm or equation allowing the calculation of the result value or depression score Z are selected from the Z equations listed in the examples, preferably the equation implemented a combination of the following 8 biomarkers MDM2, PRKCB, IFNAR1, LYN, GAB2, CAMK1D, KCNJ15 and PDE8A.

14. An in vitro method for differential diagnosing bipolar disorder and unipolar depression in a human patient according to one of claims 1 to 11 wherein if the patient to be tested is a female, in step a) said at least one or combination of at least two A to I editing RNA biomarker(s) is selected from the group of biomarkers consisting of MDM2, PRKCB, PIAS1, IFNAR1, LYN, AHR, RASSF1, GAB2, CAMK1D, IL17RA, PTPRC, KCNJ15, IFNAR2 and PDE8A biomarker, preferably the biomarkers combinations or Z equation given in the Examples or figures with patients of the female population, more preferably the combinations comprising the 8 biomarkers MDM2, PRKCB, IFNAR1, LYN, GAB2, CAMK1D, KCNJ15 and PDE8A, more preferably the combinations listed in Table 6.

15. An in vitro method for differential diagnosing bipolar disorder and unipolar depression in a human patient according to one of claims 1 to 13, wherein if the patient to be tested is a male, in step a) said at least one or combination of at least two A to I editing RNA biomarker(s) is selected from the group of biomarkers consisting of MDM2, PRKCB, PIAS1, IFNAR1, LYN, AHR, RASSF1, GAB2, CAMK1D, IL17RA, PTPRC, KCNJ15, IFNAR2 and PDE8A biomarker, preferably the biomarkers combinations or Z equation given in the Examples or figures with patients of the male population, more preferably the combinations comprising the 8 biomarkers MDM2, PRKCB, IFNAR1, LYN, GAB2, CAMK1D, KCNJ15 and PDE8A, more preferably the combinations listed in Table 7.

16. An in vitro method for differential diagnosing bipolar disorder and unipolar depression in a human patient wherein said method comprises the method for diagnosing depression for a human patient according to one of claims 1 to 15, wherein the combination of biomarkers and/or the Z equation associated with said combination used for differential diagnosing bipolar disorder and unipolar depression in a human patient is different whether the patient to be tested is a female or a male.

17. The method of one of claims 1 to 16, wherein in step a) the relative proportion of RNA editing at a given editing and/or the percentage of an isoform are measuring by NGS in said biological sample.

18. The method of one of claims 1 to 17, wherein in step a), the amplicon/nucleic sequence used for the detection of the RNA editing sites and/or the isoforms of the RNA transcript of said biomarker is obtained or obtainable with:

 - the set of primers listed in table 1 for each of the selected biomarkers (SEQ ID NO. 1 to 36), or
 - a set or a combination of sets of primers allowing to obtain amplicon(s) including, or identical to, the amplicon(s) obtainable by the set of primers listed in Table 1 9 (SEQ ID No.1 to SEQ ID No.36).

19. A method for monitoring treatment for bipolar disorder or unipolar depression in a human patient, said method comprising:

 A) differential diagnosing bipolar disorder or unipolar depression in said human by the method according to one of claims 1 to 18 before the beginning of the treatment which is desired to be monitored.
 B) repeating steps (a) to c) of the method of one of claims 2 to 18, after a period of time during which said patient received treatment for said bipolar disorder or unipolar depression, to obtain a post-treatment result value;
 C) comparing the post-treatment result value from step (c) to:

 - the result value obtained before the period of time during which said patient receives treatment for said bipolar disorder or unipolar depression, and
 - to the result value (control final value) for control bipolar disorder or unipolar depression patients, and

classifying said treatment as being effective if the post-treatment result value obtained in step B) is closer than the result value obtained before the period of time during which said patient receives treatment for control bipolar disorder or unipolar depression patients.

20. A method for determining whether a patient will be a responder to a bipolar disorder treatment allowing the patient to be in a euthymic state (state of normal mood) from a blood sample of said patient, said method comprising the step of:

 A) differential diagnosing bipolar disorder for said human by the method according to one of claims 1 to 18 before the beginning of the treatment which is desired to be monitored.
 B) repeating steps (a) to c) of the method of one of claims 2 to 18, after a period of time during which said patient received treatment for said bipolar disorder, to obtain a post-treatment result value;
 C) comparing the post-treatment result value from step (c) to:

 - the result value obtained before the period of time during which said patient receives treatment for said bipolar disorder, and
 - to the result value (control final value) for control bipolar disorder patients being in an euthymic state, and

classifying said patient as being responder to the treatment if the post-treatment result value obtained in step B) is closer than the result value obtained before the period of time during which said patient receives treatment for control bipolar disorder patients being in an euthymic state.

21. Kit for differential diagnosing bipolar disorder and unipolar, in a human patient said kit comprising:

 1) - optionally, instructions to apply the method according to one of claims 1 to 18, in order to obtain the result value the analysis of which determining whether said patient presents a bipolar disorder or unipolar depression; and

2)

 i) a combination of at least two pairs of primers selected from the group of primers consisting of SEQ ID No.1 to SEQ ID No.36 and, the selection of which being dependent of the biomarker(s) used for differential diagnosing bipolar disorder and unipolar depression in human patient;
 or
 ii) a combination of at least two pairs of primers allowing to obtain the amplicons identical to the amplicons obtained by the at least two pairs of primers selected in i).

**Patentansprüche**

1. In-vitro-Verfahren zur Differentialdiagnose einer bipolaren Störung und einer unipolaren Depression bei einem menschlichen Patienten anhand einer biologischen Probe des Patienten, wobei das Verfahren umfasst:

 a) Bestimmen für eine Kombination aus mindestens zwei A- bis I-Editierungs-RNAs:

 - für jeden ausgewählten Biomarker des relativen Anteils der RNA-Editierung an einer bestimmten Editierungsstelle für mindestens eine oder eine Kombination von Stellen, die am RNA-Transkript der mindestens zwei Biomarker editiert werden können, und/oder
 - des relativen Prozentsatzes einer Isoform oder einer Kombination von Isoformen des RNA-Transkripts jedes der beiden Biomarker;
 wobei die mindestens zwei A- bis I-Editierungs-RNA-Biomarker aus der Gruppe ausgewählt sind, bestehend aus den Biomarkern MDM2, PRKCB, PIAS1, IFNAR1, LYN, AHR, RASSF1, GAB2, CAMK1D, IL17RA, PTPRC, KCNJ15, IFNAR2 und PDE8A;

 b) Bestimmen eines für jeden der mindestens zwei Biomarker erhaltenen Ergebniswerts und eines endgültigen Ergebniswerts basierend auf einem Algorithmus oder einer Gleichung, die den für jeden ausgewählten Biomarker erhaltenen Ergebniswert enthält;
 c) Bestimmen, ob der in Schritt b) erhaltene Ergebniswert größer oder nicht größer als ein für ein unipolares Kontrollobjekt erhaltener Kontrollwert ist, wobei der Kontrollwert auf eine mit dem Ergebniswert vergleichbare Weise bestimmt wurde; und
 d) wenn der für den Patienten erhaltene Ergebniswert größer als ein Schwellenwert ist, Klassifizieren des Patienten als Patient mit einer bipolaren Störung oder, wenn der Ergebniswert nicht größer als der Schwellenwert ist, Klassifizieren des Patienten als Patient mit einer unipolaren Depression.

2. In-vitro-Verfahren zur Differentialdiagnose einer bipolaren Störung und einer unipolaren Depression bei einem menschlichen Patienten nach Anspruch 1, wobei in Schritt b) die mindestens zwei A- bis I-Editierungs-RNA-Biomarker aus der Gruppe ausgewählt sind, bestehend aus MDM2, PRKCB, IFNAR1, LYN, GAB2, CAMK1D, KCNJ15 und PDE8A.

3. In-vitro-Verfahren zur Differentialdiagnose einer bipolaren Störung und einer unipolaren Depression bei einem menschlichen Patienten nach einem der Ansprüche 1 und 2, wobei in Schritt b) der Ergebniswert oder der Endergebniswert durch einen Algorithmus berechnet wird, der ein multivariates Verfahren implementiert, das einschließt:

 - mROC-Programm, insbesondere zur Ermittlung der linearen Kombination, die den AUC-ROC (Area Under the Curve-ROC) maximiert und wobei die Gleichung für die jeweilige Kombination bereitgestellt ist und als neuer virtueller Marker Z wie folgt verwendet werden kann:
 Z = a.(Biomarker 1) + b.(Biomarker 2) + ...i.(Biomarker i) +....n.(Biomarker n), wobei i berechnete Koeffizienten sind und (Biomarker i) die Ebene des betrachteten Biomarkers ist (d. h. die Ebene der RNA-Editierungsstelle oder der Isoformen für ein gegebenes Ziel/einen gegebenen Biomarker); und/oder
 - einen Random-Forest-Ansatz (RF-Ansatz), der angewendet wird, um die Kombinationen von RNA-Editierungsstelle(n) und/oder Isoformen zu bewerten, insbesondere um die Wichtigkeit der RNA-Editierstelle(n) und/oder Isoform(en) zu bewerten und die beste(n) RNA-Editierungsstelle(n) und/oder Isoform(en) zu kombinieren, und/oder optional
 - eine multivariate Analyse, die angewendet wird, um die Kombinationen von RNA-Editierungsstelle(n) und/oder Isoformen für die Diagnostik zu bewerten, wobei die multivariate Analyse beispielsweise aus der Gruppe ausgewählt wird, bestehend aus:

- logistischem Regressionsmodell, angewendet für univariate und multivariate Analysen, um das relative Risiko des Patienten auf verschiedenen Ebenen der RNA-Editierungsstelle oder der Isoformenwerte abzuschätzen;
- CART-Ansatz (Classification And Regression Trees-Ansatz), angewendet zur Bewertung von Kombinationen von RNA-Editierungsstelle(n) und/oder Isoformen; und/oder
- Support-Vector-Machine-Ansatz (SVM-Ansatz);
- Ansatz mit künstlichen neuronalen Netzen (KNN);
- Ansatz mit Bayesianischem Netzwerk;
- WKNN-Ansatz (gewichteter k-nächster Nachbar);
- Partielle-kleinste-Quadrate-Diskriminanzanalyse (PLS-DA);
- lineare und quadratische Diskriminanzanalyse (LDAIQDA) und
- jedes andere mathematische Verfahren, das Biomarker kombiniert

4. Verfahren nach einem der Ansprüche 1 bis 3, wobei es sich bei der unipolaren Depressionsstörung hauptsächlich um eine große depressive Phase (DEP) handelt.

5. Verfahren nach einem der Ansprüche 1 bis 4, wobei die biologische Probe Vollblut, Serum, Plasma, Urin, Zerebrospinalflüssigkeit oder Speichel ist, bevorzugt ist die Vollblutprobe.

6. Verfahren nach einem der Ansprüche 1 bis 5, wobei sich der Ergebniswert für jeden ausgewählten Biomarker statistisch/spezifisch um p<0,05 von dem Kontrollergebniswert unterscheidet.

7. Verfahren nach einem der Ansprüche 1 bis 6, wobei für den in Schritt a) ausgewählten Biomarker oder die ausgewählte Kombination von Biomarkern folgende Kriterien erfüllt sein müssen:

- Abdeckung >30;
- AUC>0,8;
- 0,95 >FoldChange >1,05 und
- p<0,05.

8. Verfahren nach einem der Ansprüche 1 bis 7, wobei der bzw. die in Schritt a) ausgewählte(n) A- bis I-Editierungs-RNA-Biomarker aus der Gruppe ausgewählt sind, bestehend aus:
einer Kombination, umfassend mindestens 3, 4, 5, 6 oder 7 der folgenden Biomarker: MDM2, PRKCB, IFNAR1, LYN, GAB2, CAMK1D, KCNJ15 und PDE8A, vorzugsweise die Kombination der genannten 8 Biomarker.

9. Verfahren nach einem der Ansprüche 1 bis 8, wobei der bzw. die in Schritt a) ausgewählte(n) A- bis I-Editierungs-RNA-Biomarker die Kombination der folgenden 5 Biomarker umfasst: GAB2, IFNAR1, KCNJ15, MDM2 und PRKCB.

10. Verfahren nach einem der Ansprüche 1 bis 9, wobei der bzw. die in Schritt a) ausgewählte(n) A- bis I-Editierungs-RNA-Biomarker die Kombination der folgenden 5 Biomarker umfasst: GAB2, IFNAR1, KCNJ15, MDM2 und PRKCB und mindestens ein weiterer Biomarker, ausgewählt aus der Gruppe bestehend aus den folgenden Biomarkern: CAMK1D und LYN, vorzugsweise die Kombination der folgenden 6 Biomarker: GAB2, IFNAR1, KCNJ15, MDM2, PRKCB und LYN, wenn das Modell nach Alter, Geschlecht, psychiatrischer Behandlung und Sucht angepasst wird, oder vorzugsweise der folgenden 6 Biomarker: GAB2, IFNAR1, KCNJ15, MDM2, PRKCB und CAMK1D, wenn das Modell nach Alter und Geschlecht angepasst wird.

11. Verfahren nach einem der Ansprüche 1 bis 10, wobei der eine oder die Kombination aus mindestens 2, 3, 4, 5, 6 oder 7 ausgewählten Biomarkern die Berechnung des relativen Prozentsatzes von mindestens einer der in Tabelle 2A (Editionsstellen), 2B (Isoformen) oder 11 (Editionsstellen) aufgeführten RNA-Editionsstellen oder Isoformen für jeden der ausgewählten Biomarker umfasst.

12. Verfahren nach einem der Ansprüche 1 bis 11, wobei die Kombination aus mindestens 2, 3, 4, 5, 6, 7 oder 8 ausgewählten Biomarkern aus den in Tabelle 12 und 13 angegebenen Biomarkerkombinationen ausgewählt wird, vorzugsweise den Kombinationen, die die folgenden 8 Biomarker umfassen: MDM2, PRKCB, IFNAR1, LYN, GAB2, CAMK1D, KCNJ15 und PDE8A.

13. Verfahren nach einem der Ansprüche 1 bis 12, wobei der Algorithmus oder die Gleichung, die die Berechnung des Ergebniswerts oder Depressionsscores Z ermöglichen, aus den in den Beispielen aufgeführten Z-Gleichungen ausgewählt werden, wobei die Gleichung vorzugsweise eine Kombination der folgenden 8 Biomarker implementiert: MDM2, PRKCB, IFNAR1, LYN, GAB2, CAMK1D, KCNJ15 und PDE8A.

14. In-vitro-Verfahren zur Differentialdiagnose einer bipolaren Störung und einer unipolaren Depression bei einem menschlichen Patienten nach einem der Ansprüche 1 bis 11, wobei, wenn der zu testende Patient weiblich ist, in Schritt a) mindestens ein oder eine Kombination aus mindestens zwei A- bis I-Editierungs-RNA-Biomarkern aus der Gruppe von Biomarkern ausgewählt wird, bestehend aus den Biomarkern MDM2, PRKCB, PIAS1, IFNAR1, LYN, AHR, RASSF1, GAB2, CAMK1D, IL17RA, PTPRC, KCNJ15, IFNAR2 und PDE8A, vorzugsweise die Biomarkerkombinationen oder die Z-Gleichung, die in den Beispielen oder Abbildungen mit Patienten der weiblichen Bevölkerung angegeben sind, bevorzugter die Kombinationen, die die 8 Biomarker MDM2, PRKCB, IFNAR1, LYN, GAB2, CAMK1D, KCNJ15 und PDE8A umfassen, noch bevorzugter die in Tabelle 6 aufgeführten Kombinationen.

15. In-vitro-Verfahren zur Differentialdiagnose einer bipolaren Störung und einer unipolaren Depression bei einem menschlichen Patienten nach einem der Ansprüche 1 bis 13, wobei, wenn der zu testende Patient männlich ist, in Schritt a) mindestens ein oder eine Kombination aus mindestens zwei A- bis I-Editierungs-RNA-Biomarkern aus der Gruppe von Biomarkern ausgewählt wird, bestehend aus den Biomarkern MDM2, PRKCB, PIAS1, IFNAR1, LYN, AHR, RASSF1, GAB2, CAMK1D, IL17RA, PTPRC, KCNJ15, IFNAR2 und PDE8A, vorzugsweise die Biomarkerkombinationen oder die Z-Gleichung, die in den Beispielen oder Abbildungen mit Patienten der männlichen Bevölkerung angegeben sind, bevorzugter die Kombinationen, die die 8 Biomarker MDM2, PRKCB, IFNAR1, LYN, GAB2, CAMK1D, KCNJ15 und PDE8A umfassen, noch bevorzugter die in Tabelle 7 aufgeführten Kombinationen.

16. In-vitro-Verfahren zur Differentialdiagnose einer bipolaren Störung und einer unipolaren Depression bei einem menschlichen Patienten, wobei das Verfahren das Verfahren zur Diagnose einer Depression bei einem menschlichen Patienten nach einem der Ansprüche 1 bis 15 umfasst, wobei die Kombination von Biomarkern und/oder die mit der Kombination verbundene Z-Gleichung, die zur Differentialdiagnose einer bipolaren Störung und einer unipolaren Depression bei einem menschlichen Patienten verwendet wird, unterschiedlich ist, je nachdem, ob es sich bei dem zu testenden Patienten um eine Frau oder einen Mann handelt.

17. Verfahren nach einem der Ansprüche 1 bis 16, wobei in Schritt a) der relative Anteil der RNA-Editierung bei einer gegebenen Editierung und/oder der Prozentsatz einer Isoform durch NGS in der biologischen Probe gemessen werden.

18. Verfahren nach einem der Ansprüche 1 bis 17, wobei in Schritt a) die Amplikon-/Nukleinsequenz, die für die Erkennung der RNA-Editierungsstellen und/oder der Isoformen des RNA-Transkripts des Biomarkers verwendet wird, erhalten wird oder erhältlich ist mit:

- dem in Tabelle 1 aufgeführte Primersatz

   für jeden der ausgewählten Biomarker (SEQ ID NO. 1 bis 36) oder

- einem Satz oder einer Kombination von Sätzen von Primern, die es ermöglichen, Amplikon(e) zu erhalten, einschließend die oder identisch mit dem bzw. den Amplikonen, die mit dem Satz von Primern erhältlich sind, die aufgeführt sind in Tabelle 1

   9 (SEQ ID No.1 bis SEQ ID No. 36).

19. Verfahren zur Überwachung der Behandlung einer bipolaren Störung oder unipolaren Depression bei einem menschlichen Patienten, wobei das Verfahren umfasst:

   A) Differentialdiagnose einer bipolaren Störung oder einer unipolaren Depression bei dem Menschen durch das Verfahren nach einem der Ansprüche 1 bis 18 vor Beginn der zu überwachenden Behandlung.
   B) Wiederholen der Schritte (a) bis c) des Verfahrens nach einem der Ansprüche 2 bis 18 nach einem Zeitraum, während dessen der Patient eine Behandlung gegen die bipolare Störung oder unipolare Depression erhielt, um einen Nachbehandlungsergebniswert zu erhalten;

C) Vergleichen des Nachbehandlungsergebniswerts von Schritt (c) mit:

- dem Ergebniswert, der vor dem Zeitraum erhalten wurde, während dessen der Patient wegen der bipolaren Störung oder unipolaren Depression behandelt wird, und
- dem Ergebniswert (Kontrollendwert) für Kontrollpatienten mit bipolarer Störung oder unipolarer Depression und

Klassifizieren der Behandlung als wirksam, wenn der in Schritt B) erhaltene Nachbehandlungsergebniswert für Kontrollpatienten mit bipolarer Störung oder unipolarer Depression näher als der Ergebniswert ist, der vor dem Zeitraum erhalten wurde, während dessen der Patient eine Behandlung erhält.

20. Verfahren zum Bestimmen, ob ein Patient auf eine Behandlung einer bipolaren Störung anspricht, indem man dem Patienten anhand einer Blutprobe ermöglicht in einem euthymen Zustand (Zustand normaler Stimmung) zu sein, das Verfahren umfassend den Schritt:

A) Differentialdiagnose einer bipolaren Störung bei dem Menschen durch das Verfahren nach einem der Ansprüche 1 bis 18 vor Beginn der zu überwachenden Behandlung.
B) Wiederholen der Schritte (a) bis c) des Verfahrens nach einem der Ansprüche 2 bis 18 nach einem Zeitraum, während dessen der Patient eine Behandlung gegen die bipolare Störung erhielt, um einen Nachbehandlungsergebniswert zu erhalten;
C) Vergleichen des Nachbehandlungsergebniswerts von Schritt (c) mit:

- dem Ergebniswert, der vor dem Zeitraum erhalten wurde, während dessen der Patient wegen der bipolaren Störung behandelt wird, und
- mit dem Ergebniswert (Kontrollendwert) für Kontrollpatienten mit bipolarer Störung in einem euthymen Zustand und

Klassifizieren des Patienten als Patient, der auf die Behandlung anspricht, wenn der in Schritt B) erhaltene Nachbehandlungsergebniswert für Kontrollpatienten mit bipolarer Störung erhält, die sich in einem euthymen Zustand befinden, näher als der Ergebniswert ist, der vor dem Zeitraum erhalten wurde, während dessen der Patient eine Behandlung erhält.

21. Kit zur Differentialdiagnose bipolarer und unipolarer Störung bei einem menschlichen Patienten, wobei das Kit umfasst:

1) - optional Anweisungen zum Anwenden des Verfahrens nach einem der Ansprüche 1 bis 18, um den Ergebniswert zu erhalten, dessen Analyse bestimmt, ob bei dem Patienten eine bipolare Störung oder eine unipolare Depression vorliegt; und
2)

i) eine Kombination aus mindestens zwei Primerpaaren, ausgewählt aus der Gruppe von Primern bestehend aus SEQ ID No. 1 bis SEQ ID No. 36, und wobei deren Auswahl von dem bzw. den Biomarker(n) abhängt, der bzw. die zur Differentialdiagnose einer bipolaren Störung und unipolaren Depression bei einem menschlichen Patienten verwendet werden;
oder
ii) eine Kombination von mindestens zwei Primerpaaren, die es ermöglicht, die Amplikone zu erhalten, die mit den Amplikonen identisch sind, die durch die mindestens zwei in i) ausgewählten Primerpaare erhalten wurden.

**Revendications**

1. Procédé in vitro de diagnostic différentiel du trouble bipolaire et de la dépression unipolaire chez un patient humain à partir d'un échantillon biologique dudit patient, ledit procédé comprenant :

a) la détermination d'une combinaison d'au moins deux ARN d'édition A à I :

- pour chaque biomarqueur sélectionné, la proportion relative de l'édition de l'ARN à un site d'édition donné pour au moins un ou une combinaison de sites qui peuvent être édités sur le produit de la transcription de l'ARN desdits au moins deux biomarqueurs, et/ou

- le pourcentage relatif d'un isoforme ou d'une combinaison d'isoformes du produit de la transcription de l'ARN de chacun desdits deux biomarqueurs ;

dans lequel lesdits au moins deux biomarqueurs d'ARN d'édition A à I sont sélectionnés dans le groupe constitué des biomarqueurs MDM2, PRKCB, PIAS1, IFNAR1, LYN, AHR, RASSF1, GAB2, CAMK1D, IL17RA, PTPRC, KCNJ15, IFNAR2 et PDE8A ;

b) la détermination d'une valeur de résultat obtenue pour chacun desdits au moins deux biomarqueurs et une valeur de résultat finale basée sur un algorithme ou une équation qui comporte la valeur de résultat obtenue pour chaque biomarqueur sélectionné ;

c) le fait de déterminer si ladite valeur de résultat obtenue à l'étape b) est supérieure ou non à une valeur de contrôle obtenue pour un sujet unipolaire de contrôle, dans lequel la valeur de contrôle a été déterminée d'une manière comparable à celle de la valeur de résultat ; et

d) si ladite valeur de résultat obtenue pour le patient est supérieure à un seuil, la classification dudit patient comme ayant un trouble bipolaire ou, si la valeur de résultat n'est pas supérieure audit seuil, la classification dudit patient comme ayant une dépression unipolaire.

2. Procédé in vitro de diagnostic différentiel du trouble bipolaire et de la dépression unipolaire chez un patient humain selon la revendication 1, dans lequel, à l'étape b), lesdits au moins deux biomarqueurs ARN d'édition A à I sont sélectionnés dans le groupe constitué de MDM2, PRKCB, IFNAR1, LYN, GAB2, CAMK1D, KCNJ15 et PDE8A.

3. Procédé in vitro de diagnostic différentiel du trouble bipolaire et de la dépression unipolaire chez un patient humain selon l'une des revendications 1 et 2 dans lequel, à l'étape b), la valeur de résultat ou la valeur de résultat final est calculée par un algorithme mettant en oeuvre un procédé multivarié comportant :

- le programme mROC, en particulier pour identifier la combinaison linéaire qui maximise la ROC ASC (aire sous la courbe) et dans lequel l'équation pour la combinaison respective est fournie et peut être utilisée comme un nouveau marqueur virtuel Z, comme suit :

$Z = a.(Biomarqueur\ 1) + b.(Biomarqueur\ 2) + ...i.(Biomarqueur\ i) + ....n.(Biomarqueur\ n)$ où i est le coefficient calculé et (Biomarqueur i) est le niveau du biomarqueur considéré (c'est-à-dire le niveau du site d'édition de l'ARN ou des isoformes pour une cible donnée/un biomarqueur donné) ; et/ou

- une approche par forêt aléatoire (RF) appliquée pour évaluer les combinaisons du ou des sites d'édition de l'ARN et/ou d'isoformes, en particulier pour classer l'importance du ou des sites d'édition de l'ARN et/ou du ou des isoformes, et pour combiner le ou les meilleurs sites d'édition de l'ARN et/ou isoformes, et/ou éventuellement

- une analyse multivariée appliquée pour évaluer les combinaisons du ou des sites d'édition de l'ARN et/ou d'isoformes pour le diagnostic, ladite analyse multivariée étant sélectionnée par exemple dans le groupe constitué :

- du modèle de régression logistique appliqué pour l'analyse univariée et multivariée afin d'estimer le risque relatif des patients à différents niveaux de valeurs du site d'édition de l'ARN ou des isoformes ;

- de l'approche CART (arbres de régression et de classification) appliquée pour évaluer les combinaisons du ou des sites d'édition de l'ARN et/ou d'isoformes ; et/ou

- de l'approche par machine à vecteurs de support (SVM) ;

- de l'approche par réseau de neurones artificiels (RNA) ;

- de l'approche par réseau bayésien ;

- de l'approche WKNN (k plus proches voisins pondérés) ;

- de l'analyse discriminante partielle par les moindres carrés (PLS-DA) ;

- de l'analyse discriminante linéaire et quadratique (LDAI QDA), et

- de tout autre procédé mathématique combinant des biomarqueurs.

4. Procédé selon l'une des revendications 1 à 3, dans lequel ledit trouble de dépression unipolaire est principalement un épisode dépressif caractérisé (EDC).

5. Procédé selon l'une des revendications 1 à 4, dans lequel ledit échantillon biologique est du sang total, du sérum, du plasma, de l'urine, du liquide céphalo-rachidien ou de la salive, l'échantillon de sang total étant préféré.

6. Procédé selon l'une des revendications 1 à 5, dans lequel, pour chaque biomarqueur sélectionné, ladite valeur de résultat est statistiquement/spécifiquement différente de ladite valeur de résultat de contrôle à $p < 0,05$.

**7.** Procédé selon l'une des revendications 1 à 6, dans lequel les critères suivants doivent être satisfaits pour le biomarqueur ou la combinaison de biomarqueurs sélectionnés à l'étape a) :

- la couverture > 30 ;
- AUC > 0,8 ;
- 0,95 > changement de pli > 1,05, et
- p < 0,05.

**8.** Procédé selon l'une des revendications 1 à 7, dans lequel ledit ou lesdits biomarqueurs d'ARN d'édition A à I sélectionnés compris à l'étape a) sont sélectionnés dans le groupe constitué par :
une combinaison comprenant au moins 3, 4, 5, 6 ou 7 des biomarqueurs suivants : MDM2, PRKCB, IFNAR1, LYN, GAB2, CAMK1D, KCNJ15 et PDE8A, de préférence la combinaison des 8 biomarqueurs cités.

**9.** Procédé selon l'une des revendications 1 à 8, dans lequel ledit ou lesdits biomarqueurs d'ARN d'édition A à I sélectionnés compris à l'étape a) la combinaison des 5 biomarqueurs suivants : GAB2, IFNAR1, KCNJ15, MDM2 et PRKCB.

**10.** Procédé selon l'une des revendications 1 à 9, dans lequel ledit ou lesdits biomarqueurs d'ARN d'édition A à I sélectionnés compris à l'étape a) la combinaison des 5 biomarqueurs suivants : GAB2, IFNAR1, KCNJ15, MDM2 et PRKCB et au moins un autre biomarqueur sélectionné dans le groupe constitué des biomarqueurs suivants : CAMK1D et LYN, de préférence la combinaison des 6 biomarqueurs suivants : GAB2, IFNAR1, KCNJ15, MDM2, PRKCB et LYN lorsque le modèle est ajusté en fonction de l'âge, du sexe, du traitement psychiatrique et de la dépendance, ou de préférence les 6 biomarqueurs suivants : GAB2, IFNAR1, KCNJ15, MDM2, PRKCB et CAMK1D lorsque le modèle est ajusté en fonction de l'âge et du sexe.

**11.** Procédé selon l'une des revendications 1 à 10, dans lequel ledit un ou la combinaison d'au moins 2, 3, 4, 5, 6 ou 7 biomarqueurs sélectionnés comprend le calcul du pourcentage relatif d'au moins l'un parmi un site d'édition de l'ARN ou un isoforme énuméré dans le tableau 2A (sites d'édition), 2B (isoformes) ou 11 (sites d'édition) pour chacun du biomarqueur sélectionné.

**12.** Procédé selon l'une des revendications 1 à 11, dans lequel ladite combinaison d'au moins 2, 3, 4, 5, 6, 7 ou 8 biomarqueurs sélectionnés est sélectionnée parmi les combinaisons de biomarqueurs données dans les tableaux 12 et 13, de préférence les combinaisons comprenant les 8 biomarqueurs suivants MDM2, PRKCB, IFNAR1, LYN, GAB2, CAMK1D, KCNJ15 et PDE8A.

**13.** Procédé selon l'une des revendications 1 à 12, dans lequel l'algorithme ou l'équation permettant le calcul de la valeur de résultat ou du score de dépression Z sont sélectionnés parmi les équations Z énumérées dans les exemples, de préférence l'équation a mis en oeuvre une combinaison des 8 biomarqueurs suivants MDM2, PRKCB, IFNAR1, LYN, GAB2, CAMK1D, KCNJ15 et PDE8A.

**14.** Procédé in vitro de diagnostic différentiel du trouble bipolaire et de la dépression unipolaire chez un patient humain selon l'une des revendications 1 à 11, dans lequel, si le patient à tester est une femme, à l'étape a), ledit au moins un ou une combinaison d'au moins deux biomarqueurs d'ARN d'édition A à I est sélectionné dans le groupe de biomarqueurs constitué du biomarqueur MDM2, PRKCB, PIAS1, IFNAR1, LYN, AHR, RASSF1, GAB2, CAMK1D, IL17RA, PTPRC, KCNJ15, IFNAR2 et PDE8A, de préférence les combinaisons de biomarqueurs ou l'équation Z données dans les exemples ou les figures avec des patients de la population féminine, plus préférablement les combinaisons comprenant les 8 biomarqueurs MDM2, PRKCB, IFNAR1, LYN, GAB2, CAMK1D, KCNJ15 et PDE8A, plus préférablement les combinaisons énumérées dans le tableau 6.

**15.** Procédé in vitro de diagnostic différentiel du trouble bipolaire et de la dépression unipolaire chez un patient humain selon l'une des revendications 1 à 13, dans lequel, si le patient à tester est un homme, à l'étape a), ledit au moins un ou une combinaison d'au moins deux biomarqueurs d'ARN d'édition A à I est sélectionné dans le groupe de biomarqueurs constitué du biomarquer MDM2, PRKCB, PIAS1, IFNAR1, LYN, AHR, RASSF1, GAB2, CAMK1D, IL17RA, PTPRC, KCNJ15, IFNAR2 et PDE8A, de préférence les combinaisons de biomarqueurs ou l'équation Z données dans les exemples ou les figures avec des patients de la population masculine, plus préférablement les combinaisons comprenant les 8 biomarqueurs MDM2, PRKCB, IFNAR1, LYN, GAB2, CAMK1D, KCNJ15 et PDE8A, plus préférablement les combinaisons énumérées dans le tableau 7.

**16.** Procédé in vitro de diagnostic différentiel du trouble bipolaire et de la dépression unipolaire chez un patient humain, dans lequel ledit procédé comprend le procédé de diagnostic de la dépression chez un patient humain selon l'une des revendications 1 à 15, dans lequel la combinaison de biomarqueurs et/ou l'équation Z associée à ladite combinaison utilisée pour le diagnostic différentiel du trouble bipolaire et de la dépression unipolaire chez un patient humain est différente selon que le patient à tester est une femme ou un homme.

**17.** Procédé selon l'une des revendications 1 à 16, dans lequel, à l'étape a), la proportion relative de l'édition de l'ARN à une édition donnée et/ou le pourcentage d'un isoforme sont mesurés par NGS dans ledit échantillon biologique.

**18.** Procédé selon l'une des revendications 1 à 17, dans lequel, à l'étape a), l'amplicon/séquence nucléique utilisé pour la détection des sites d'édition de l'ARN et/ou des isoformes du produit de la transcription de l'ARN dudit biomarqueur est obtenu ou peut être obtenu à l'aide :

- de la série d'amorces énumérées dans le tableau 1 pour chacun des biomarqueurs sélectionnés (SEQ ID NO. 1 à 36), ou
- d'un ensemble ou d'une combinaison d'ensembles d'amorces permettant d'obtenir un ou des amplicons comportant le ou les amplicons pouvant être obtenus par l'ensemble d'amorces énuméré dans le tableau 1 9 (SEQ ID N° 1 à SEQ ID N° 36).

**19.** Procédé de suivi du traitement d'un trouble bipolaire ou d'une dépression unipolaire chez un patient humain, ledit procédé comprenant :

A) le diagnostic différentiel du trouble bipolaire ou de la dépression unipolaire chez ledit humain par le procédé selon l'une des revendications 1 à 18 avant le début du traitement que l'on souhaite suivre.
B) la répétition des étapes (a) à (c) du procédé selon l'une des revendications 2 à 18, après une période de temps pendant laquelle ledit patient a reçu un traitement pour ledit trouble bipolaire ou ladite dépression unipolaire, afin d'obtenir une valeur de résultat post-traitement ;
C) la comparaison de la valeur de résultat post-traitement de l'étape (c) à :

- la valeur de résultat obtenue avant la période de temps pendant laquelle ledit patient reçoit un traitement pour ledit trouble bipolaire ou ladite dépression unipolaire, et
- à la valeur de résultat (valeur finale de contrôle) pour les patients contrôles atteints de troubles bipolaires ou de dépression unipolaire, et

la classification dudit traitement comme étant efficace si la valeur de résultat post-traitement obtenue à l'étape B) est plus proche de la valeur de résultat obtenue avant la période de temps pendant laquelle ledit patient reçoit un traitement pour des patients contrôles atteints de troubles bipolaires ou de dépression unipolaire.

**20.** Procédé destiné à déterminer si un patient répondra à un traitement du trouble bipolaire permettant au patient d'être dans un état euthymique (état d'humeur normale) à partir d'un échantillon de sang dudit patient, ledit procédé comprenant l'étape consistant à :

A) diagnostiquer de manière différentielle le trouble bipolaire chez ledit humain par le procédé selon l'une des revendications 1 à 18 avant le début du traitement que l'on souhaite suivre.
B) répéter les étapes (a) à (c) du procédé selon l'une des revendications 2 à 18, après une période de temps pendant laquelle ledit patient a reçu un traitement pour ledit trouble bipolaire, afin d'obtenir une valeur de résultat post-traitement ;
C) la comparaison de la valeur de résultat post-traitement de l'étape (c) à :

- la valeur de résultat obtenue avant la période de temps pendant laquelle ledit patient reçoit un traitement pour ledit trouble bipolaire, et
- à la valeur de résultat (valeur finale de contrôle) pour les patients contrôles atteints du trouble bipolaire se trouvant dans un état euthymique, et

classifier ledit patient comme répondant au traitement si la valeur de résultat post-traitement obtenue à l'étape B) est plus proche de la valeur de résultat obtenue avant la période de temps pendant laquelle ledit patient reçoit un traitement pour les patients contrôles atteints de troubles bipolaires se trouvant dans un état euthymique.

**21.** Kit pour le diagnostic différentiel du trouble bipolaire et du trouble unipolaire chez un patient humain, ledit kit comprenant :

1) - éventuellement, des instructions pour appliquer le procédé selon l'une des revendications 1 à 18, afin d'obtenir la valeur de résultat dont l'analyse permet de déterminer si ledit patient présente un trouble bipolaire ou une dépression unipolaire ; et
2)

i) une combinaison d'au moins deux paires d'amorces sélectionnées dans le groupe d'amorces constitué de SEQ ID N° 1 à SEQ ID N° 36 et dont la sélection dépend du ou des biomarqueurs utilisés pour le diagnostic différentiel du trouble bipolaire et de la dépression unipolaire chez le patient humain ;
ou
ii) une combinaison d'au moins deux paires d'amorces permettant d'obtenir des amplicons identiques aux amplicons obtenus par les au moins deux paires d'amorces sélectionnées en i).

Fig. 1A

Fig. 1B

Fig. 1C

Fig. 1D

Fig. 2

Fig. 3

Fig. 4A

Fig. 4B

Fig. 4C

Fig. 4D

Fig. 5A

Fig. 5B

Fig. 5C

Fig. 5D

Fig. 5E

Fig. 6A

Fig. 6B

Fig. 6C

Fig. 7A

Fig. 7B

Fig. 7C

Fig. 7D

Fig. 7E

Fig. 8A

Fig. 8B

Fig. 8C

Fig. 8D

Fig. 8E

Fig. 9A

Fig. 9B

Fig. 9C

Fig. 10A

Fig. 10B

Fig. 10C

Fig. 11A

Fig. 11B

Fig. 11C

Fig. 12A

Fig. 12B

AUC=0.997

Empirical ROC curve ( 0.997 )
Youden Threshold
Equality Threshold

Fig. 12C

AUC=1

Empirical ROC curve ( 1 )
Youden Threshold
Equality Threshold

Fig. 13A

Fig. 13B

Fig. 13C

Fig. 14

| | Total sample | CTRL | BD | Uni |
|---|---|---|---|---|
| **Demographics** | | | | |
| | n | n | n | n |
| Number | 58 | 31 | 14 | 13 |
| | | | | |
| Age (min-max) | 24-59 | 24-59 | 26-55 | 26-55 |
| Age (mean±SD) | 41.2 (±9.5) | 41.9 (±9.7) | 41.0 (±9.5) | 40.0 (±9.8) |
| p value (age, vs Ctrl) | | | 0.65 | 0.45 |
| p value (age, vs BD) | | | | 0.79 |
| | | | | |
| **Gender** | | | | |
| Male (n (%)) | 31 (52.5%) | 16 (51.6%) | 5 (35.7%) | 10 (81.25%) |
| Female (n (%)) | 28 (47.5%) | 15 (48.4%) | 9 (64.3%) | 3 (18.75%) |
| | | | | |
| **BMI (kg/m$^2$)** | | 25.3 | 23.9 | 23.7 |
| p value (BMI, vs Ctrl) | | | 0.35 | 0.3 |
| p value (BMI, vs BD) | | | | 0.89 |
| | | | | |
| **Clinical characteristics** | | | | |
| | | | | |
| **Average MADRS score** (mean±SEM) | | 0.80 (±0.2) | 21.7 (±2.9) | 25.6 (±4.7) |
| p value (MADRS, vs Ctrl) | | | 1.3E-13 | 3.60E-11 |
| p value (MADRS, vs BD) | | | | 0.47 |
| | | | | |
| **Average IDSC30 score** (mean±SEM) | | 2.65 (±0.5) | 29.4 (±3.9) | 29.2 (±5.3) |
| p value (IDSC30, vs Ctrl) | | | 1.10E-12 | 2.50E-10 |
| p value (IDSC30, vs BD) | | | | 0.98 |
| | | | | |
| **Average YMRS score** (mean±SEM) | | 0.06 (±0.06) | 1.5 (±0.6) | 1.25 (±0.5) |
| p value (YMRS, vs BD) | | | | 0.8 |

Fig. 15

| Category | GO ID | GO term | Ratio | p(FDR) |
|---|---|---|---|---|
| Biological Process | GO:0002682 | regulation of immune system process | 66/1391 | 2.37E-12 |
| Biological Process | GO:0050776 | regulation of immune response | 47/873 | 5.37E-10 |
| Biological Process | GO:0002376 | immune system process | 83/2370 | 1.09E-09 |
| Biological Process | GO:0050778 | positive regulation of immune response | 36/589 | 7.68E-09 |
| Biological Process | GO:0002768 | immune response-regulating cell surface receptor signaling pathway | 24/266 | 1.62E-08 |
| Biological Process | GO:0002757 | immune response-activating signal transduction | 26/332 | 3.53E-08 |
| Biological Process | GO:0002252 | immune effector process | 44/927 | 3.83E-08 |
| Biological Process | GO:0002253 | activation of immune response | 28/393 | 3.83E-08 |
| Biological Process | GO:0002429 | immune response-activating cell surface receptor signaling pathway | 22/234 | 3.83E-08 |
| Biological Process | GO:0002684 | positive regulation of immune system process | 43/882 | 3.83E-08 |
| Biological Process | GO:0002764 | immune response-regulating signaling pathway | 27/365 | 3.83E-08 |
| Biological Process | GO:0045321 | leukocyte activation | 42/894 | 1.13E-07 |
| Biological Process | GO:0001775 | cell activation | 45/1024 | 1.67E-07 |
| Biological Process | GO:0080090 | regulation of primary metabolic process | 144/5982 | 1.67E-07 |
| Biological Process | GO:0044093 | positive regulation of molecular function | 61/1713 | 2.90E-07 |
| Biological Process | GO:0006464 | cellular protein modification process | 88/2999 | 3.36E-07 |
| Biological Process | GO:0060255 | regulation of macromolecule metabolic process | 144/6072 | 3.63E-07 |
| Biological Process | GO:0048584 | positive regulation of response to stimulus | 68/2054 | 3.89E-07 |
| Biological Process | GO:0051171 | regulation of nitrogen compound metabolic process | 139/5827 | 5.79E-07 |
| Biological Process | GO:0006955 | immune response | 56/1560 | 8.88E-07 |

Fig. 16

| Pathway ID | Pathway name | Event hierarchy | Entities ratio | p(FDR) |
|---|---|---|---|---|
| R-HSA-202427 | Phosphorylation of CD3 and TCR zeta chains | Adaptative Immune System | 16/45 | 8.99E-10 |
| R-HSA-202433 | Generation of second messenger molecules | Adaptative Immune System | 16/58 | 1.79E-08 |
| R-HSA-202430 | Translocation of ZAP-70 to Immunological synapse | Adaptative Immune System | 14/42 | 1.79E-08 |
| R-HSA-389948 | PD-1 signaling | Adaptative Immune System | 14/45 | 3.27E-08 |
| R-HSA-202403 | TCR signaling | Adaptative Immune System | 21/146 | 8.91E-07 |
| R-HSA-388841 | Costimulation by the CD28 family | Adaptative Immune System | 19/97 | 1.30E-06 |
| R-HSA-202424 | Downstream TCR signaling | Adaptative Immune System | 17/124 | 3.53E-05 |
| R-HSA-877300 | Interferon gamma signaling | Cytokine Signaling in Immune System | 21/250 | 0.00273264 |
| R-HSA-2132295 | MHC class II antigen presentation | Adaptative Immune System | 15/148 | 0.00452889 |
| R-HSA-9616222 | Transcriptional regulation of granulopoiesis | Developmental biology | 9/71 | 0.02720614 |

Fig. 17

| Gene ID | GeneName | Region | Chr | Position (GRCh38) | Coverage | Fold Change | p value | AUC |
|---|---|---|---|---|---|---|---|---|
| ENSG00000177663 | IL17RA | 3UTR | 22 | 17110591 | 107 | 0.74 | 5.53E-03 | 0.929 |
| ENSG00000183049 | CAMK1D | intron | 10 | 12387673 | 34 | 0.66 | 6.32E-03 | 0.912 |
| ENSG00000142166 | IFNAR1 | 3UTR | 21 | 33355807 | 32 | 1.42 | 1.31E-02 | 0.910 |
| ENSG00000177663 | IL17RA | intron | 22 | 17089708 | 48 | 1.25 | 6.94E-03 | 0.906 |
| ENSG00000033327 | GAB2 | intron | 11 | 78330944 | 37 | 1.43 | 3.71E-03 | 0.896 |
| ENSG00000166501 | LYN | intron | 8 | 55890893 | 130 | 1.20 | 4.20E-02 | 0.880 |
| ENSG00000106546 | AHR | 3UTR | 7 | 17345159 | 61 | 1.36 | 1.47E-02 | 0.859 |
| ENSG00000033800 | PIAS1 | intron | 15 | 68138039 | 47 | 1.32 | 1.52E-02 | 0.857 |
| ENSG00000166501 | PRKCB | intron | 16 | 23920896 | 78 | 0.74 | 3.43E-03 | 0.853 |
| ENSG00000135679 | MDM2 | intron | 12 | 68821612 | 38 | 1.31 | 4.45E-02 | 0.850 |
| ENSG00000081237 | PTPRC | intron | 1 | 198680332 | 74 | 0.77 | 4.31E-03 | 0.846 |
| ENSG00000166501 | LYN | 3UTR | 8 | 56012553 | 44 | 1.48 | 2.11E-03 | 0.846 |
| ENSG00000159110 | IFNAR2 | intron | 21 | 33231766 | 37 | 0.73 | 6.82E-03 | 0.840 |
| ENSG00000068028 | RASSF1 | intron | 3 | 50332838 | 43 | 0.84 | 3.76E-02 | 0.825 |
| ENSG00000157551 | KCNJ15 | intron | 21 | 38288302 | 37 | 1.47 | 1.69E-02 | 0.819 |

## Fig. 18

| Category | Term ID | Term description | p(FDR) | matching proteins |
|---|---|---|---|---|
| Biological process | GO:0035556 | intracellular signal transduction | 7.64E-06 | AHR,CAMK1D,GAB2,IFNAR1,IFNAR2,LYN,MDM2,PIAS1,PRKCB,RASSF1 |
| Biological process | GO:0007259 | receptor signaling pathway via JAK-STAT | 1.41E-05 | IFNAR1,IFNAR2,LYN,PIAS1 |
| Biological process | GO:0007165 | signal transduction | 3.19E-05 | AHR,CAMK1D,GAB2,IFNAR1,IFNAR2,IL17RA,LYN,MDM2,PDE8A,PIAS1,PRKCB,PTPRC,RASSF1 |
| Biological process | GO:0050857 | positive regulation of antigen receptor-mediated signaling pathway | 9.82E-05 | LYN,PRKCB,PTPRC |
| Biological process | GO:0002682 | regulation of immune system process | 0.0002 | AHR,CAMK1D,GAB2,IFNAR2,LYN,PIAS1,PRKCB,PTPRC |
| Biological process | GO:0050853 | B cell receptor signaling pathway | 0.00029 | LYN,PRKCB,PTPRC |
| Biological process | GO:0071310 | cellular response to organic substance | 0.00039 | AHR,GAB2,IFNAR1,IFNAR2,IL17RA,LYN,MDM2,PDE8A,PIAS1 |
| Biological process | GO:0030888 | regulation of B cell proliferation | 0.0011 | AHR,LYN,PTPRC |
| Biological process | GO:0050776 | regulation of immune response | 0.0014 | GAB2,IFNAR2,LYN,PIAS1,PRKCB,PTPRC |
| Biological process | GO:0050861 | positive regulation of B cell receptor signaling pathway | 0.0014 | LYN,PRKCB |
| Biological process | GO:0002768 | immune response-regulating cell surface receptor signaling pathway | 0.0021 | GAB2,LYN,PRKCB,PTPRC |
| Biological process | GO:0007166 | cell surface receptor signaling pathway | 0.0025 | GAB2,IFNAR1,IFNAR2,IL17RA,LYN,PIAS1,PRKCB,PTPRC |
| Biological process | GO:0048522 | positive regulation of cellular process | 0.0025 | AHR,CAMK1D,GAB2,IL17RA,LYN,MDM2,PDE8A,PIAS1,PRKCB,PTPRC,RASSF1 |
| Biological process | GO:0048583 | regulation of response to stimulus | 0.0025 | CAMK1D,GAB2,IFNAR2,IL17RA,LYN,MDM2,PDE8A,PIAS1,PRKCB,PTPRC |
| Biological process | GO:0006950 | response to stress | 0.0041 | CAMK1D,IFNAR1,IFNAR2,IL17RA,LYN,MDM2,PRKCB,PTPRC,RASSF1 |
| Biological process | GO:0009893 | positive regulation of metabolic process | 0.0041 | AHR,CAMK1D,LYN,MDM2,PDE8A,PIAS1,PRKCB,PTPRC,RASSF1 |
| Biological process | GO:0051049 | regulation of transport | 0.0041 | CAMK1D,GAB2,IL17RA,KCNJ15,LYN,MDM2,PRKCB |
| Biological process | GO:0032879 | regulation of localization | 0.0046 | CAMK1D,GAB2,IL17RA,KCNJ15,LYN,MDM2,PRKCB,PTPRC |
| Biological process | GO:0006952 | defense response | 0.0047 | CAMK1D,IFNAR1,IFNAR2,IL17RA,LYN,PTPRC |
| Biological process | GO:0007049 | cell cycle | 0.0048 | AHR,MDM2,PIAS1,PRKCB,PTPRC,RASSF1 |

Fig. 19

| Pathway ID | Pathway name | p(FDR) | Matching entities |
|---|---|---|---|
| R-HSA-8939246 | RUNX1 regulates transcription of genes involved in differentiation of myeloid cells | 0.01235604 | PRKCB |
| R-HSA-3232118 | SUMOylation of transcription factors | 0.0203032 | MDM2;PIAS1 |
| R-HSA-912694 | Regulation of IFNA signaling | 0.02527928 | IFNAR2;IFNAR1 |
| R-HSA-399719 | Trafficking of AMPA receptors | 0.02527928 | PRKCB;MDM2 |
| R-HSA-399721 | Glutamate binding, activation of AMPA receptors and synaptic plasticity | 0.02527928 | PRKCB;MDM2 |
| R-HSA-6804757 | Regulation of TP53 Degradation | 0.02527928 | MDM2 |
| R-HSA-3232142 | SUMOylation of ubiquitinylation proteins | 0.02527928 | MDM2;PIAS1 |
| R-HSA-6806003 | Regulation of TP53 Expression and Degradation | 0.02527928 | MDM2 |
| R-HSA-512988 | Interleukin-3, Interleukin-5 and GM-CSF signaling | 0.02527928 | LYN;GAB2 |
| R-HSA-1433557 | Signaling by SCF-KIT | 0.02527928 | LYN;GAB2 |
| R-HSA-1280215 | Cytokine Signaling in Immune system | 0.02640054 | LYN;IFNAR2;GAB2;IL17RA;PIAS1;IFNAR1 |
| R-HSA-112314 | Neurotransmitter receptors and postsynaptic signal transmission | 0.02887316 | PRKCB;MDM2;KCNJ15 |

Fig. 20

| | Total sample | Unipolar | Bipolar |
|---|---|---|---|
| | | Depressive | Depressive |
| **Demographics** | | | |
| | n | n | n |
| **Number** | 35 | 16 | 19 |
| | | | |
| **Age** (min-max) | 20-60 | 19-59 | 27-60 |
| Age (mean±SD) | 42.5 (±10.6) | 40.1 (±12.4) | 44.6 (±8.7) |
| p value (*vs* Unipolar) | | | 0.22 |
| | | | |
| **Gender** | | | |
| Male (n (%)) | 18 (51.4 %) | 10 (62.5 %) | 8 (42.1 %) |
| Female (n (%)) | 17 (48.6 %) | 6 (37.5 %) | 11 (57.9 %) |
| | | | |
| **BMI (kg/m$^2$)** | | 22.7 | 23.8 |
| p value (*vs* Unipolar) | | | 0.50 |
| | | | |
| **Clinical characteristics** | | | |
| **Average MADRS score** (mean±SEM) | | 27.3 (±2.0) | 22.2 (±2.1) |
| p value (*vs* Unipolar) | | | 0.10 |
| | | | |
| **Average IDSC30 score** (mean±SEM) | | 32.2 (±2.1) | 29.5 (± 2.8) |
| p value (*vs* Unipolar) | | | 0.46 |
| | | | |
| **Average YMRS score** (mean±SEM) | | 0.55 (± 0.5) | 2.1 (± 0.7) |
| p value (*vs* Unipolar) | | | 0.11 |

EP 4 055 191 B1

Fig. 21

| | Total sample | Unipolar | Bipolar |
|---|---|---|---|
| | | Depressive | Depressive |
| **Demographics** | | | |
| | n | n | n |
| Number | 58 | 29 | 29 |
| **Age** (min-max) | 18-62 | 18-62 | 21-62 |
| Age (mean±SD) | 41.9 (±11.1) | 40.8 (±13.0) | 43.1 (±8.9) |
| p value (*vs* Unipolar) | | | 0.43 |
| **Gender** | | | |
| Male (n (%)) | 27 (46.6 %) | 14 (48.3 %) | 13 (44.8 %) |
| Female (n (%)) | 31 (53.4 %) | 15 (51.7 %) | 16 (55.2 %) |
| **BMI** ($kg/m^2$) | | 22.3 | 24.7 |
| p value (*vs* Unipolar) | | | 0.03 |
| **Clinical characteristics** | | | |
| **Average MADRS score** (mean±SEM) | | 34.6 (±1.2) | 31.7 (±1.2) |
| p value (*vs* Unipolar) | | | 0.09 |
| **Average IDSC30 score** (mean±SEM) | | 40.1 (±1.4) | 37.2 (± 1.9) |
| p value (*vs* Unipolar) | | | 0.23 |
| **Average YMRS score** (mean±SEM) | | 1.29 (± 0.4) | 3.1 (± 1.0) |
| p value (*vs* Unipolar) | | | 0.10 |

Fig. 22

| | Total sample | Unipolar | Bipolar | |
| | | Depressive | Depressive | Euthymic |
|---|---|---|---|---|
| **Demographics** | | | | |
| | **n** | **n** | **n** | **n** |
| **Number** | 297 | 160 | 96 | 41 |
| | | | | |
| **Age** (min-max) | 18-67 | 18-64 | 19-67 | 31-65 |
| Age (mean±SD) | 40.9 (±13.2) | 36.3 (±13.4) | 44.6 (±11.1) | 49.9 (±9.3) |
| p value (*vs* Unipolar) | | | <0.00001 | <0.00001 |
| | | | | |
| **Gender** | | | | |
| Male (n (%)) | 97 (32.7%) | 50 (31.2%) | 27 (28.1%) | 20 (48.8%) |
| Female (n (%)) | 200 (67.3%) | 110 (68.8%) | 69 (71.9%) | 21 (51.2%) |
| | | | | |
| **BMI (kg/m$^2$)** | 287 | 23.1 | 25.0 | 25.1 |
| p value (*vs* Unipolar) | | | 0.007 | 0.032 |
| | | | | |
| **Clinical characteristics** | | | | |
| **Average MADRS score** (mean±SEM) | 287 | 30.7 (±0.6) | 28.2 (±0.7) | 2.6 (±0.3) |
| p value (*vs* Unipolar) | | | 0.011 | <0.00001 |
| | | | | |
| **Average IDSC30 score** (mean±SEM) | 293 | 37.4 (±0.7) | 35.3 (±0.8) | 5.4 (±0.5) |
| p value (*vs* Unipolar) | | | 0.052 | <0.00001 |
| | | | | |
| **Average YMRS score** (mean±SEM) | 233 | 1.4 (±0.5) | 2.6 (±0.4) | 1.2 (±0.5) |
| p value (*vs* Unipolar) | | | 0.005 | 0.59 |

Fig. 23

| | Total sample | CTRL | Bipolar | |
|---|---|---|---|---|
| | | | Depressive | Euthymic |
| **Demographics** | | | | |
| | n | n | n | n |
| Number | 280 | 143 | 96 | 41 |
| | | | | |
| **Age** (min-max) | 18-67 | 18-65 | 19-67 | 31-65 |
| Age (mean±SD) | 39.4 (±13.4) | 39.4 (±14.1) | 44.6 (±11.1) | 49.9 (±9.3) |
| p value (*vs* Ctrl) | | | <0.00001 | <0.00001 |
| | | | | |
| **Gender** | | | | |
| Male (n (%)) | 100 (35.71 %) | 53 (37.1%) | 27 (28.1%) | 20 (48.8%) |
| Female (n (%)) | 180 (64.29 %) | 90 (62.9%) | 69 (71.9%) | 21 (51.2%) |
| | | | | |
| **BMI (kg/m$^2$)** | | 24.6 | 25.0 | 25.1 |
| p value (*vs* Ctrl) | | | 0.007 | 0.032 |
| | | | | |
| **Clinical characteristics** | | | | |
| **Average MADRS score** ((mean±SEM) | - | 0.8 (±0.1) | 28.2 (±0.7) | 2.6 (±0.3) |
| p value (*vs* Ctrl) | - | | 0.011 | <0.00001 |
| | | | | |
| **Average IDSC30 score** (mean±SEM) | - | 1.97 (±0.2) | 35.3 (± 0.8) | 5.4 (± 0.5) |
| p value (*vs* Ctrl) | - | | 0.052 | <0.00001 |

Fig. 24

| | Total sample | Bipolar Type 1 | Bipolar Type 2 |
|---|---|---|---|
| **Demographics** | | | |
| | n | n | n |
| Number | 140 | 58 | 82 |
| | | | |
| **Age** (min-max) | 19-67 | 21-67 | 19-66 |
| Age (mean±SD) | 45.4 (±10.8) | 45.4 (±10.4) | 45.5 (±11.1) |
| p value (*vs* Bipolar Type 1) | | | 0.933 |
| | | | |
| **Gender** | | | |
| Male (n (%)) | 52 (37.1 %) | 22 (37.9 %) | 30 (36.6 %) |
| Female (n (%)) | 88 (62.9 %) | 36 (62.1 %) | 52 (63.4 %) |
| | | | |
| **BMI (kg/m$^2$)** | 138 | 26.6 | 24.4 |
| p value (*vs* Bipolar Type 1) | | | 0.027 |
| | | | |
| **Clinical characteristics** | | | |
| **Average MADRS score** (mean±SEM) | 132 | 15.9 (±1.6) | 18.0 (±1.5) |
| p value (*vs* Bipolar Type 1) | | | 0.363 |
| | | | |
| **Average IDSC30 score** (mean±SEM) | 125 | 21.4 (±1.9) | 23.3 (± 1.8) |
| p value (*vs* Bipolar Type 1) | | | 0.453 |
| | | | |
| **Average YMRS score** (mean±SEM) | 127 | 1.93 (± 0.4) | 1.89 (± 0.4) |
| p value (vs Bipolar Type 1) | | | 0.94 |

Fig. 25

| Category | GO ID | GO term | FDR |
|---|---|---|---|
| Biological Process | GO:0002376 | immune system process | 1.77E-10 |
| Biological Process | GO:0002682 | regulation of immune system process | 7.45E-10 |
| Biological Process | GO:0002252 | immune effector process | 1.52E-08 |
| Biological Process | GO:0002253 | activation of immune response | 7.50E-08 |
| Biological Process | GO:0050776 | regulation of immune response | 1.19E-07 |
| Biological Process | GO:0006955 | immune response | 1.51E-07 |
| Biological Process | GO:0002757 | immune response-activating signal transduction | 1.72E-07 |
| Biological Process | GO:0002764 | immune response-regulating signaling pathway | 1.74E-07 |
| Biological Process | GO:0050778 | positive regulation of immune response | 2.22E-07 |
| Biological Process | GO:0002684 | positive regulation of immune system process | 4.15E-07 |
| Biological Process | GO:0048584 | positive regulation of response to stimulus | 6.90E-07 |
| Biological Process | GO:0002444 | myeloid leukocyte mediated immunity | 1.0526E-06 |
| Biological Process | GO:0002274 | myeloid leukocyte activation | 4.8148E-06 |
| Biological Process | GO:0002446 | neutrophil mediated immunity | 8.1445E-06 |
| Biological Process | GO:0002366 | leukocyte activation involved in immune response | 8.4827E-06 |
| Biological Process | GO:0035556 | intracellular signal transduction | 8.6505E-06 |
| Biological Process | GO:0043299 | leukocyte degranulation | 8.8488E-06 |
| Biological Process | GO:0045321 | leukocyte activation | 9.2368E-06 |
| Biological Process | GO:0002263 | cell activation involved in immune response | 9.7805E-06 |
| Biological Process | GO:0002768 | immune response-regulating cell surface receptor signaling pathway | 1.059E-05 |

EP 4 055 191 B1

Fig. 26

| Category | GO ID | GO term | FDR | N of Genes |
|---|---|---|---|---|
| Biological Process | GO:0002682 | regulation of immune system process | 1.00E-10 | 67 |
| Biological Process | GO:0002376 | immune system process | 1.51E-07 | 84 |
| Biological Process | GO:0050776 | regulation of immune response | 5.90E-09 | 50 |
| Biological Process | GO:0050778 | positive regulation of immune response | 2.68E-07 | 38 |
| Biological Process | GO:0045321 | leukocyte activation | 3.61E-07 | 42 |
| Biological Process | GO:0002684 | positive regulation of immune system process | 5.45E-07 | 45 |
| Biological Process | GO:0048584 | positive regulation of response to stimulus | 5.99E-07 | 73 |
| Biological Process | GO:0001775 | cell activation | 6.94E-07 | 45 |
| Biological Process | GO:0036211 | protein modification process | 7.43E-07 | 86 |
| Biological Process | GO:0006464 | cellular protein modification process | 7.43E-07 | 86 |
| Biological Process | GO:0060255 | regulation of macromolecule metabolic process | 9.07E-07 | 139 |
| Biological Process | GO:0002757 | immune response-activating signal transduction | 1.06E-06 | 28 |
| Biological Process | GO:0002253 | activation of immune response | 1.07E-06 | 30 |
| Biological Process | GO:0002252 | immune effector process | 1.07E-06 | 43 |
| Biological Process | GO:0002764 | immune response-regulating signaling pathway | 1.12E-06 | 29 |
| Biological Process | GO:0043412 | macromolecule modification | 1.73E-06 | 88 |
| Biological Process | GO:0019222 | regulation of metabolic process | 4.11E-06 | 145 |
| Biological Process | GO:0002366 | leukocyte activation involved in immune response | 4.11E-06 | 31 |
| Biological Process | GO:0002263 | cell activation involved in immune response | 4.55E-06 | 31 |
| Biological Process | GO:0002274 | myeloid leukocyte activation | 5.48E-06 | 30 |

Fig. 27A

Fig.27B

Fig. 28A

Fig. 28B

Fig. 29A

# RandomForest model1
## Adjusted by age, sex, treatments and addictions

| | Performance |
|---|---|
| AUC | 0.934 |
| CI 95% | [0.880 ; 0.987] |
| Se | 91.2% |
| Sp | 84.6% |
| PPV | 83.8% |
| NPV | 91.7% |
| Acc | 87.7% |
| Cutoff | 0.5 |

AUC=0.934

Fig. 29B

**RandomForest model2**
Adjusted by age, sex

| | Performance |
|---|---|
| AUC | 0.899 |
| CI 95% | [0.826 ; 0.971] |
| Se | 84.8% |
| Sp | 83.7% |
| PPV | 80.0% |
| NPV | 87.8% |
| Acc | 84.2% |
| Cutoff | 0.5 |

AUC=0.899

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2017211144 A

**Non-patent literature cited in the description**

- **GRANDE I ; BERK M ; BIRMAHER B ; VIETA E.** Bipolar disorder. *Lancet.,* 09 April 2016, vol. 387 (10027), 1561-1572
- **SHARMA V ; KHAN M ; SMITH A.** A closer look at treatment resistant depression: is it due to a bipolar diathesis?. *Journal of affective disorders,* February 2005, vol. 84 (2-3), 251-257
- **GROTEGERD D ; SUSLOW T ; BAUER J et al.** Discriminating unipolar and bipolar depression by means of fMRI and pattern classification: a pilot study. *European archives of psychiatry and clinical neuroscience.,* March 2013, vol. 263 (2), 119-131
- **SAGAR R ; PATTANAYAK RD.** Potential biomarkers for bipolar disorder: Where do we stand?. *The Indian journal of medical research.,* January 2017, vol. 145 (1), 7-16
- **TASIC L ; LARCERDA ALT ; PONTES JGM et al.** Peripheral biomarkers allow differential diagnosis between schizophrenia and bipolar disorder. *Journal of psychiatric research.,* 23 September 2019, vol. 119, 67-75
- **GATSIOU A ; VLACHOGIANNIS N ; LUNELLA FF ; SACHSE M ; STELLOS K.** Adenosine-to-Inosine RNA Editing in Health and Disease. *Antioxidants & redox signaling.,* 20 September 2018, vol. 29 (9), 846-863
- **DOBIN A ; GINGERAS TR.** Optimizing RNA-Seq Mapping with STAR. *Methods in molecular biology.,* 2016, vol. 1415, 245-262
- **DEPRISTO MA ; BANKS E ; POPLIN R et al.** A framework for variation discovery and genotyping using next-generation DNA sequencing data. *Nature genetics.,* May 2011, vol. 43 (5), 491-498
- **JOHN D ; WEIRICK T ; DIMMELER S ; UCHIDA S.** RNAEditor: easy detection of RNA editing events and the introduction of editing islands. *Briefings in bioinformatics.,* 01 November 2017, vol. 18 (6), 993-1001
- **YANG H ; WANG K.** Genomic variant annotation and prioritization with ANNOVAR and wANNOVAR. *Nature protocols.,* October 2015, vol. 10 (10), 1556-1566

- **TEMPEL S.** Using and understanding RepeatMasker. *Methods in molecular biology.,* 2012, vol. 859, 29-51
- **KASPRZYK A ; KEEFE D ; SMEDLEY D et al.** EnsMart: a generic system for fast and flexible access to biological data. *Genome research.,* January 2004, vol. 14 (1), 160-169
- **LANGMEAD B ; SALZBERG SL.** Fast gapped-read alignment with Bowtie 2. *Nature methods.,* 04 March 2012, vol. 9 (4), 357-359
- **SCHNEIDER VA ; GRAVES-LINDSAY T ; HOWE K et al.** Evaluation of GRCh38 and de novo haploid genome assemblies demonstrates the enduring quality of the reference assembly. *Genome research.,* May 2017, vol. 27 (5), 849-864
- **LI H ; HANDSAKER B ; WYSOKER A et al.** The Sequence Alignment/Map format and SAMtools. *Bioinformatics.,* 15 August 2009, vol. 25 (16), 2078-2079
- **LI H.** A statistical framework for SNP calling, mutation discovery, association mapping and population genetical parameter estimation from sequencing data. *Bioinformatics.,* 01 November 2011, vol. 27 (21), 2987-2993
- **GENTLEMAN RC ; CAREY VJ ; BATES DM et al.** Bioconductor: open software development for computational biology and bioinformatics. *Genome biology,* 2004, vol. 5 (10), R80
- **LINDEN A.** Measuring diagnostic and predictive accuracy in disease management: an introduction to receiver operating characteristic (ROC) analysis. *Journal of evaluation in clinical practice.,* April 2006, vol. 12 (2), 132-139
- **KRAMAR A ; FARAGGI D ; FORTUNE A ; REISER B.** mROC: a computer program for combining tumour markers in predicting disease states. *Computer methods and programs in biomedicine,* September 2001, vol. 66 (2-3), 199-207
- Random Forests. **BREIMAN L.** Machine Learning. Kluwer Academic Publishers, 2001, vol. 45, 5-32
- **SU JQ ; LIU JS.** Linear Combinations of Multiple Diagnostic Markers. *Journal of the American Statistical Association.,* 1993, vol. 88 (424), 1350-1355

- **WANG H.** A note on iterative marginal optimization: a simple algorithm for maximum rank correlation estimation. *Computational Statistics and Data Analysis.,* 2007, vol. 51, 2803-2812
- **STAACK A ; BADENDIECK S ; SCHNORR D ; LOENING SA ; JUNG K.** Combined determination of plasma MMP2, MMP9, and TIMP1 improves the non-invasive detection of transitional cell carcinoma of the bladder. *BMC urology.,* 10 August 2006, vol. 6, 19
- **BLAGUS R ; LUSA L.** Class prediction for high-dimensional class-imbalanced data. *BMC bioinformatics.,* 20 October 2010, vol. 11, 523
- **PICARDI E ; MANZARI C ; MASTROPASQUA F ; AIELLO I ; D'ERCHIA AM ; PESOLE G.** Profiling RNA editing in human tissues: towards the inosinome Atlas. *Scientific reports.,* 09 October 2016, vol. 5, 14941
- **BAZAK L ; LEVANON EY ; EISENBERG E.** Genome-wide analysis of Alu editability. *Nucleic acids research,* June 2014, vol. 42 (11), 6876-6884
- **CONSORTIUM GO.** Gene Ontology Consortium: going forward. *Nucleic acids research,* January 2015, vol. 43, D1049-1056
- **RAUDVERE U ; KOLBERG L ; KUZMIN I et al.** g:Profiler: a web server for functional enrichment analysis and conversions of gene lists (2019 update). *Nucleic acids research,* 02 July 2019, vol. 47 (W1), W191-W198
- **POMAZNOY M ; HA B ; PETERS B.** GOnet: a tool for interactive Gene Ontology analysis. *BMC bioinformatics.,* 07 December 2018, vol. 19 (1), 470
- **CHIMIENTI F ; CAVAREC L ; VINCENT L et al.** Brain region-specific alterations of RNA editing in PDE8A mRNA in suicide decedents. *Translational psychiatry.,* 15 February 2019, vol. 9 (1), 91
- **SALVETAT N ; VAN DER LAAN S ; VIRE B et al.** RNA editing blood biomarkers for predicting mood alterations in HCV patients. *Journal of neurovirology.,* 22 July 2019
- **SZKLARCZYK D ; GABLE AL ; LYON D et al.** STRING v11: protein-protein association networks with increased coverage, supporting functional discovery in genome-wide experimental datasets. *Nucleic acids research,* 08 January 2019, vol. 47 (D1), D607-D613
- **KHANDELWAL N ; DEY SK ; CHAKRAVARTY S ; KUMAR A.** miR-30 Family miRNAs Mediate the Effect of Chronic Social Defeat Stress on Hippocampal Neurogenesis in Mouse Depression Model. *Frontiers in molecular neuroscience,* 2019, vol. 12, 188
- **SHELTON RC.** The molecular neurobiology of depression. *The Psychiatric clinics of North America,* March 2007, vol. 30 (1), 1-11
- **COSTAS J ; GRATACOS M ; ESCARAMIS G et al.** Association study of 44 candidate genes with depressive and anxiety symptoms in post-partum women. *Journal of psychiatric research.,* August 2010, vol. 44 (11), 717-724
- **COLLEDGE M ; SNYDER EM ; CROZIER RA et al.** Ubiquitination regulates PSD-95 degradation and AMPA receptor surface expression. *Neuron.,* 30 October 2003, vol. 40 (3), 595-607
- **LIU B ; MINK S ; WONG KA et al.** PIAS1 selectively inhibits interferon-inducible genes and is important in innate immunity. *Nature immunology.,* September 2004, vol. 5 (9), 891-898
- **MURAKAMI Y ; ISHIBASHI T ; TOMITA E et al.** Depressive symptoms as a side effect of Interferon-alpha therapy induced by induction of indoleamine 2,3-dioxygenase 1. *Scientific reports.,* 20 July 2016, vol. 6, 29920
- **BRODIE EJ ; INFANTINO S ; LOW MSY ; TARLINTON DM.** Lyn, Lupus, and (B) Lymphocytes, a Lesson on the Critical Balance of Kinase Signaling in Immunity. *Frontiers in immunology.,* 2018, vol. 9, 401
- **HAYASHI T ; UMEMORI H ; MISHINA M ; YAMAMOTO T.** The AMPA receptor interacts with and signals through the protein tyrosine kinase Lyn. *Nature,* 07 January 1999, vol. 397 (6714), 72-76
- **MOURA-ALVES P ; FAE K ; HOUTHUYS E et al.** AhR sensing of bacterial pigments regulates antibacterial defence. *Nature,* 28 August 2014, vol. 512 (7515), 387-392
- **SONG MS ; SONG SJ ; KIM SY ; OH HJ ; LIM DS.** The tumour suppressor RASSF1A promotes MDM2 self-ubiquitination by disrupting the MDM2-DAXX-HAUSP complex. *The EMBO journal,* 09 July 2008, vol. 27 (13), 1863-1874
- **ALEKSANDROVA LR ; PHILLIPS AG ; WANG YT.** Antidepressant effects of ketamine and the roles of AMPA glutamate receptors and other mechanisms beyond NMDA receptor antagonism. *Journal of psychiatry & neuroscience : JPN.,* June 2017, vol. 42 (4), 222-229
- **VERPLOEGEN S ; LAMMERS JW ; KOENDERMAN L ; COFFER PJ.** Identification and characterization of CKLiK, a novel granulocyte Ca(++)/calmodulin-dependent kinase. *Blood.,* 01 November 2000, vol. 96 (9), 3215-3223
- **LIN E ; KUO PH ; LIU YL ; YU YW ; YANG AC ; TSAI SJ.** A Deep Learning Approach for Predicting Antidepressant Response in Major Depression Using Clinical and Genetic Biomarkers. *Frontiers in psychiatry,* 2018, vol. 9, 290

- **ZHOU X ; CHEN Y ; MOK KY et al.** Identification of genetic risk factors in the Chinese population implicates a role of immune system in Alzheimer's disease pathogenesis. *Proceedings of the National Academy of Sciences of the United States of America,* 20 February 2018, vol. 115 (8), 1697-1706
- **BEUREL E ; LOWELL JA.** Th17 cells in depression. *Brain, behavior, and immunity.,* March 2018, vol. 69, 28-34
- **FISCHER EH ; CHARBONNEAU H ; TONKS NK.** Protein tyrosine phosphatases: a diverse family of intracellular and transmembrane enzymes. *Science,* 26 July 1991, vol. 253 (5018), 401-406
- **IRIE-SASAKI J ; SASAKI T ; MATSUMOTO W et al.** CD45 is a JAK phosphatase and negatively regulates cytokine receptor signalling. *Nature,* 18 January 2001, vol. 409 (6818), 349-354
- **BASTERZI AD ; YAZICI K ; BUTURAK V et al.** Effects of venlafaxine and fluoxetine on lymphocyte subsets in patients with major depressive disorder: a flow cytometric analysis. *Progress in neuro-psychopharmacology & biological psychiatry.,* 01 February 2010, vol. 34 (1), 70-75
- **ASTON C ; JIANG L ; SOKOLOV BP.** Transcriptional profiling reveals evidence for signaling and oligodendroglial abnormalities in the temporal cortex from patients with major depressive disorder. *Molecular psychiatry.,* March 2005, vol. 10 (3), 309-322
- **SENEY ML ; HUO Z ; CAHILL K et al.** Opposite Molecular Signatures of Depression in Men and Women. *Biological psychiatry.,* 01 July 2018, vol. 84 (1), 18-27
- Diagnostic and Statistical Manual of Mental Disorders. American Psychiatric Association, 2013